# EUROPEAN PATENT APPLICATION

(11) **EP 4 470 565 A1**
(43) Date of publication of application: **04.12.2024**
(21) Application number: 23745881.5
(22) Date of filing: 09.01.2023
(51) Int. Cl.: A61K 45/06, A61K 31/21, A61P 17/00, A61P 17/02

(54) **METHOD FOR PREVENTING OR TREATING DISEASE OR DISORDER ASSOCIATED WITH ANTINEOPLASTIC AGENT**

(30) Priority: 28.01.2022 CN 202210108382
(71) Applicant: OnQuality Pharmaceuticals China Ltd., Shanghai 201112 (CN)
(72) Inventor: ZHANG, Shiyi, Shanghai 201112 (CN); LIU, Shilan, Shanghai 201112 (CN); WU, Chengguang, Shanghai 201112 (CN)
(74) Representative: Weickmann & Weickmann PartmbB
(86) International application number: PCT/CN2023/071255
(87) International publication number: WO 2023/142996

(57) **Abstract**

The present application relates to a method of preventing, ameliorating and/or treating a disease or disorder associated with administration of an anti-tumor agent, comprising administering a NO-NSAID compound or a pharmaceutically acceptable salt thereof to a subject in need thereof.

## Description

### TECHNICAL FILED

The present application relates to the field of biomedicine, and particularly to a method of preventing, ameliorating and/or treating diseases or disorders associated with the administration of anti-tumor agents in a subject.

### BACKGROUND

Anti-tumor agents (e.g., targeted anti-tumor drugs, chemotherapy drugs, and/or immunotherapies such as immune checkpoint inhibitors, etc.) frequently cause severe side effects, particularly on the skin, sensory features, and gastrointestinal tract. These severe side effects caused by these anti-tumor agents can significantly impair the patient's quality of life, reduce the patient's medication compliance and tolerance, lead to drug withdrawal or insufficient medication dosage, which adversely affecting the treatment effect, and even leading to accelerated disease progression, thereby shortening patient survival.

Currently, there are no effective treatment regimen to control the side effects associated with the administration of anti-tumor agents. Therefore, there is an urgent need for a therapeutic strategy that can successfully manage these side effects.

### SUMMARY OF THE INVENTION

The compounds, pharmaceutical compositions and methods provided in the present application effectively address the aforementioned issues by preventing, ameliorating, and/or treating the side effects associated with the administration of anti-tumor agents in a subject.

In one aspect, the present application provides a use of a NO-NSAID compound or a pharmaceutically acceptable salt thereof in the preparation of a medicament, which is used for preventing, ameliorating and/or treating a disease or disorder associated with the administration of an anti-tumor agent in a subject.

In some embodiments, the NO-NSAID compound comprises a nitric oxide (NO) releasing moiety and a nonsteroidal anti-inflammatory agent (NSAID) moiety, and the NO releasing moiety and the nonsteroidal anti-inflammatory agent moiety are covalently linked directly or via a spacer.

In some embodiments, the non-steroidal anti-inflammatory agent moiety comprises a cyclooxygenase (COX) inhibitory moiety.

In some embodiments, the COX inhibitory moiety comprises a moiety capable of reducing the expression of the COX, and/or reducing the activity of the COX.

In some embodiments, the COX inhibitory moiety acts directly on the COX protein and/or the nucleic acid encoding the COX protein.

In some embodiments, the COX inhibitory moiety is capable of inhibiting cyclooxygenase-1 (COX-1) and/or cyclooxygenase-2 (COX-2).

In some embodiments, the COX inhibitory moiety is capable of selectively inhibiting COX-2.

In some embodiments, the COX inhibitory moiety is capable of non-selectively inhibiting COX-1 and COX-2.

In some embodiments, the COX inhibitory moiety comprises one or more molecules, prodrugs thereof, active derivatives thereof, and/or active metabolites thereof, selected from the group consisting of acetaminophen, acemetacin, aceclofenac, alminoprofen, amfenac, bendazac, benoxaprofen, bromfenac, bucloxic acid , butibufen, carprofen, cinmetacin, clopirac, diclofenac, etodolac, felbinac, fenclozic acid, fenbufen, fenoprofen, fentiazac, flunoxaprofen, flurbiprofen, ibufenac, ibuprofen, indomethacin, isofezolac, isoxepac, indoprofen, ketoprofen, lonazolac, loxoprofen, metiazinic acid, mofezolac, miroprofen, naproxen, oxaprozin, pirozolac, pranoprofen, protizinic acid, salicylamide, sulindac, suprofen, suxibuzone, tiaprofenic acid, tolmetin, xenbucin, ximoprofen, zaltoprofen, zomepirac, aspirin, acemetcin, bumadizon, carprofenac, clidanac, diflunisal, enfenamic acid, fendosal, flufenamic acid, flunixin, gentisic acid, ketorolac, meclofenamic acid, mefenamic acid and mesalamine.

In some embodiments, the COX inhibitory moiety comprises one or more molecules, prodrugs thereof, active derivatives thereof, and/or active metabolites thereof, selected from the group consisting of naproxen, aspirin, diclofenac, ketoprofen, flurbiprofen and ibuprofen.

In some embodiments, the NO-releasing moiety is capable of generating at least one of NO⁺, NO⁻, N₂O, NO, N₂O₃, NO₂, NO₃⁻, and NO₂⁻.

In some embodiments, the NO-releasing moiety generates NO directly or indirectly.

In some embodiments, the NO-releasing moiety comprises organic molecules, inorganic molecules, and/or polymers or nanomaterials.

In some embodiments, the NO-releasing moiety comprises an component selected from the group consisting of: nitroglycerin, isosorbide mononitrate, butanediol mononitrate, pentaerythritol tetranitrate, isosorbide dinitrate, triethanolamine, nicorandil, propatylnitrate, 5-amino-3-(4-morpholinyl)-1,2,3-oxadiazole, isopentyl nitrite, 3,3-bis(aminoethyl)-1-hydroxy-2-carbonyl-1-triazene (NOC-18), Sulfo NONOate disodium salt, S- nitrosoglutathione, S-nitroso-N-acetylpenicillamine, 4-phenyl-3-furoxancarbonitrile, ((±)-(E)-4-Ethyl-2-[(E)-hydroxyimino]-5-nitro-3-hexenamide), Streptozocin, NG-Hydroxy-L-arginine acetate salt, O₂ -(2,4-Dinitrophenyl) 1-[(4- ethoxycarbonyl)piperazin-1-yl]diazen-1-ium-1,2-diolate), N-Nitrosodibutylamine, 3-morpholinosydnonimine (SIN-1) , Linsidomine, Molsidomine, 3-(4-acetylphenyl)sydnone, Diethylamine NONOate /AM) and Itramin.

In some embodiments, the NO-releasing moiety comprises a component selected from the group consisting of nitroxyl complex, metal nitrosyl complex, metal nitrosamino complex, nitrates and nitrites.

In some embodiments, the NO-releasing moiety comprises a component selected from the group consisting of S-nitrosothiol nanosilica spheres, S-nitrosethanedithiol chitosan and oligopropylenediamine-grafted NONOate.

In some embodiments, the NO-releasing moiety has a molecular weight that is less than or equal to 2000 Daltons, less than or equal to 1500 Daltons, less than or equal to 1200 Daltons, less than or equal to 1000 Daltons, or less than or equal to 900 Daltons, less than or equal to 800 Daltons, less than or equal to 700 Daltons, less than or equal to 600 Daltons, less than or equal to 500 Daltons, less than or equal to 400 Daltons, less than or equal to 300 Daltons, less than or equal to 200 Daltons and/or less than or equal to 100 Daltons.

In some embodiments, the NO-releasing moiety has one or more of the following groups: diazeniumdiolates, hydroxydiazenesulfonic acid, S-nitrosothiol, furoxans, oxime, N-nitrosamine, N-hydroxyguanidine, nitrate, nitrite, nitrate ester, nitrite ester, sydnonimines, sydnones, oxtriazole-5-imine, oxtriazol-5-one, hydroxylamine, dioxadiazetidine, N-hydroxynitrosamine, N- nitrosoimine, hydroxyurea and metal nitrosamino complexes.

In some embodiments, the NO-releasing moiety comprises -NO₂ or -ONO₂.

In some embodiments, in the NO-NSAID compound, the NO releasing moiety is linked to the NSAID moiety through a cleavable linker.

In some embodiments, in the NO-NSAID compound, the NO-releasing moiety is linked to the NSAID moiety through a linker comprising an ester bond and/or a disulfide bond.

In some embodiments, in the NO-NSAID compound, a spacer is included between the NO releasing moiety and the NSAID moiety.

In some embodiments, the spacer comprises: one or more optionally substituted -CH2- , optionally substituted phenyl, and/or a combination thereof.

In some embodiments, the NO-NSAID compound further comprises a H₂S releasing moiety.

In some embodiments, the NO-NSAID compound comprises a structure represented by Formula (1): 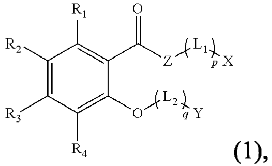 wherein:
Z is O or NH,
R₁, R₂, R₃ and R₄ are each independently H, halogen, NO₂, N₃, C₁-C₁₀ alkyl, OR, OC(O)R, N(R)₂, NH-C(O)R, S(O)R or N N-R, wherein each R is independently a C₁-C₁₀ alkyl or aryl group;
L₁ comprises a structure selected from the group consisting of: -C(O)-, -(CH₂)ₘ-, -(CH₂)ₘ-O-, - (CH₂)ₘ-C(O)-, -(CH₂)ₘ-C(O)O-, -(CH₂)ₘ-OC(O)O-, -C(O)-(CH₂)ₘ-O-, -C(O)-(CH₂)ₘ-C(O)-, - OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7;
L₂ comprises a structure selected from the group consisting of: -C(O)-, -(CH₂)ₘ-, -(CH₂)ₘ-O-, - (CH₂)ₘ-C(O)-, -(CH₂)ₘ-C(O)O-, -(CH₂)ₘ-OC(O)O-, -C(O)-(CH₂)ₘ-O-, -C(O)-(CH₂)ₘ-C(O)-, - OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7;
P and q are each independently 0 or 1;
X is the H₂S releasing moiety or the NO releasing moiety;
Y is the NO releasing moiety or the H₂S releasing moiety, and Y and X are not simultaneously the NO releasing moiety nor simultaneously the H₂S releasing moiety;
The NO releasing moiety is -C(O)-(CH₂)ₙ-ONO₂ or -(CH₂)ₙ-ONO₂, wherein n is 1, 2, 3, 4, 5, 6 or 7; and the H₂S releasing moiety is: 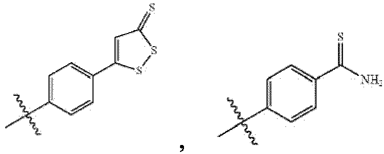 or

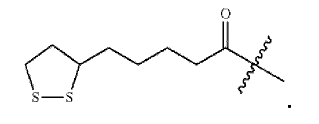

In some embodiments, the NO-NSAID compound comprises a structure represented by Formula (2): 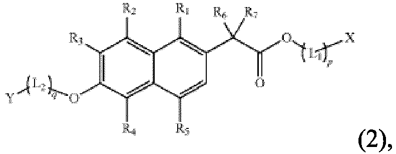 wherein:
p and q are each independently 0 or 1;
L₁ and L₂ are each independently comprise a structure selected from the group consisting of - C(O)-, -(CH₂)ₘ-, -(CH₂)ₘ-O-, -(CH₂)ₘ -C(O)-, -(CH₂)ₘ-C(O)O-, -(CH₂)ₘ -OC(O)O-, -C(O)-(CH₂)ₘ-O-, -C(O)-(CH₂)ₘ-C(O)-, -OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7;
X is the H₂S releasing moiety or the NO releasing moiety;
Y is the NO releasing moiety or the H₂S releasing moiety, and the Y and the X are not simultaneously the NO releasing moiety nor simultaneously not the H₂S releasing moiety;
The NO releasing moiety is selected from: -NO, -C(O)-(CH₂)ₙ-ONO₂, -O-(CH₂)ₙ-ONO₂, - (CH₂)ₙ-ONO₂, -C(O)-CH₂-C(CH₃)₂-SNO, -NH-CH₂-C(CH₃)₂-SNO, -CH₂-C(CH₃)₂-SNO, 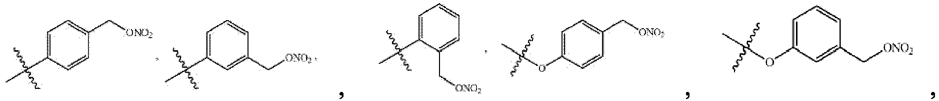 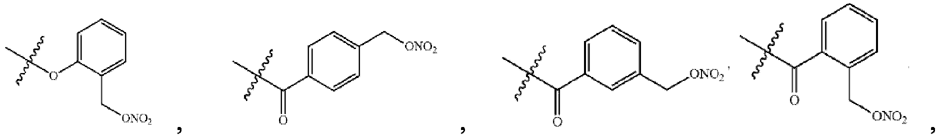 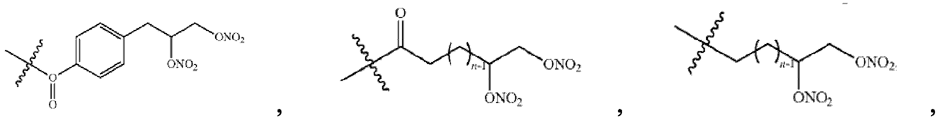 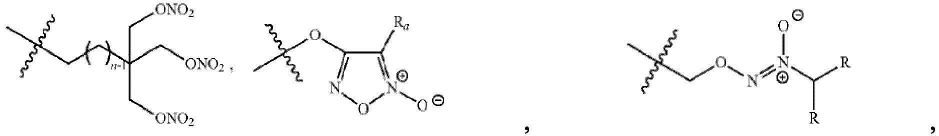 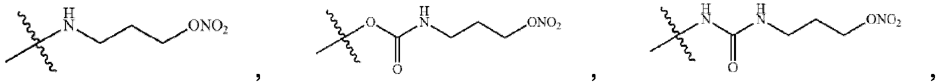 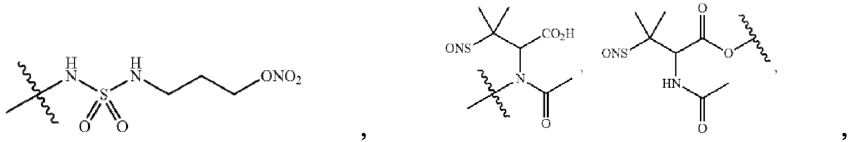 and 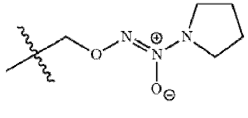 wherein n is 1, 2, 3, 4, 5, 6 or 7, Rₐ is H, C₁-C₁₀alkyl, aryl, S(O)₂-aryl, CN or CON(R_{b})₂, and each R_{b} is independently H or C₁-C₁₀ alkyl; and
the H₂S release moiety is selected from: 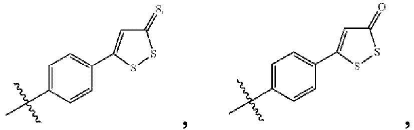 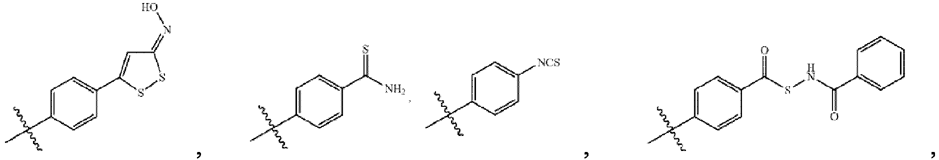 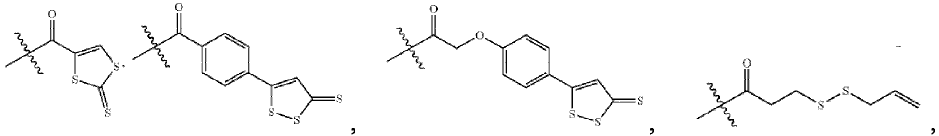 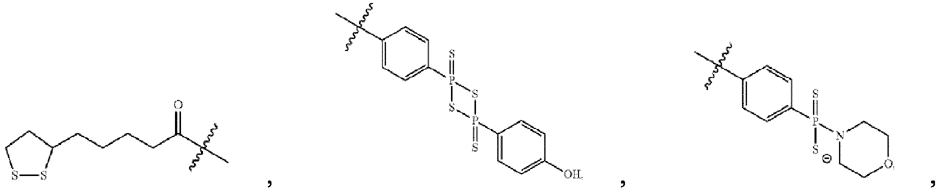 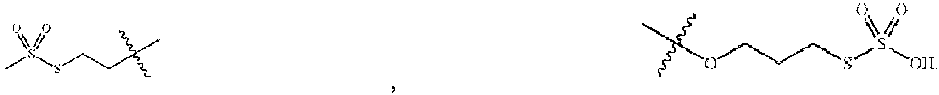 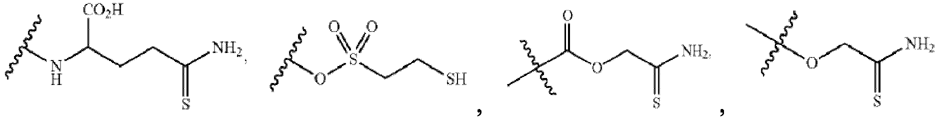 and

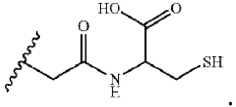

In some embodiments, the NO-NSAID compound comprises a structure selected from the group consisting of 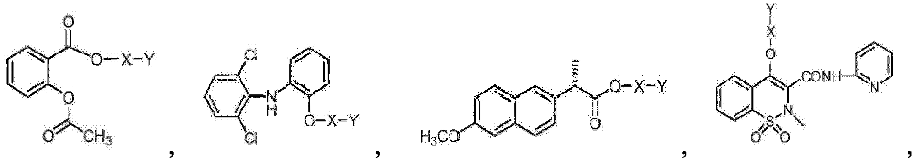 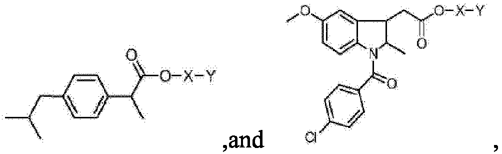
Wherein, X is -(CH₂)ₙ₁-Z-(CH₂)ₙ₂-, n1 and n2 are each independently 0, 1, 2, 3, 4 or 5;
Z is O, N, S, C, 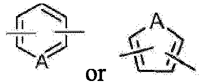 , A is O, N, S, or C; and
Yis

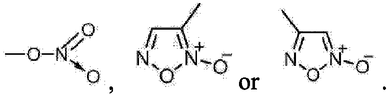

In some embodiments, the NO-NSAID compound comprises a structure represented by Formula (3):

M-X-Y-ONO₂ (3),

wherein M is a structure selected from the following group: 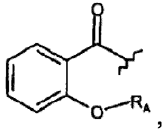 wherein R_{A} is a hydrogen atom or -C(O)CH₃; 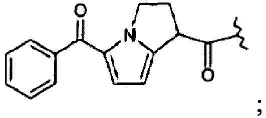 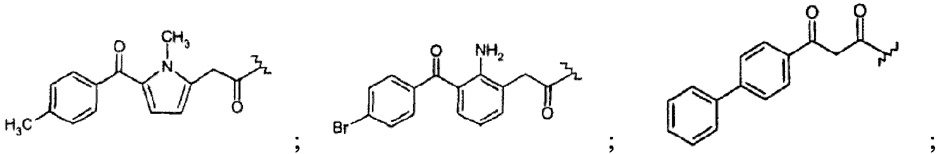 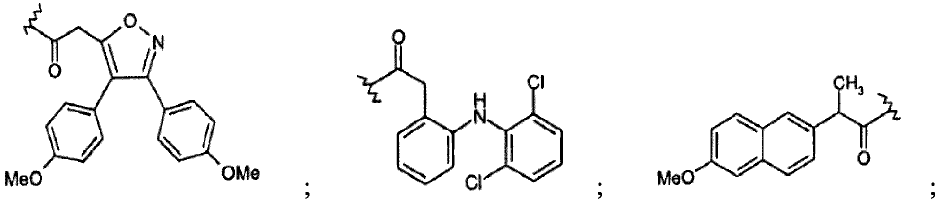 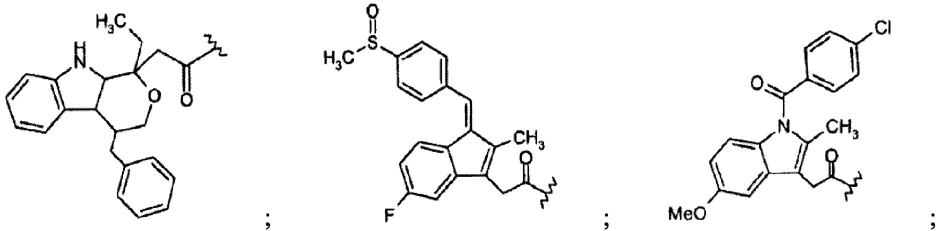 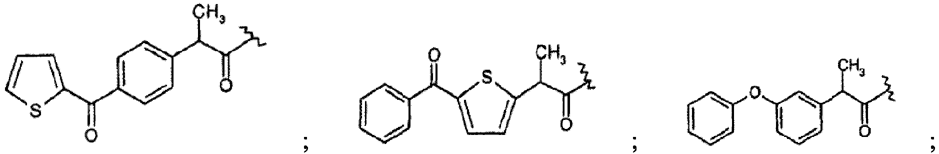 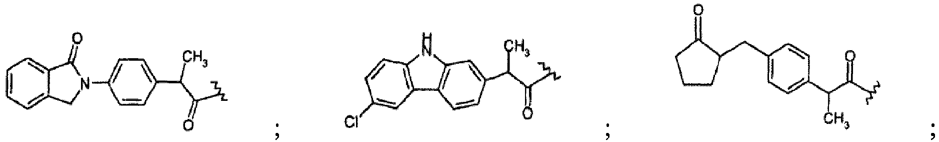 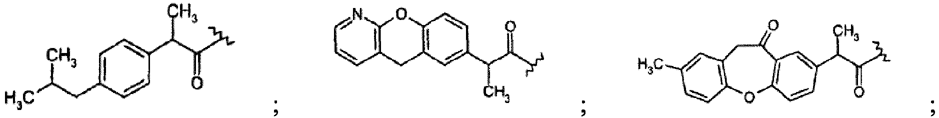 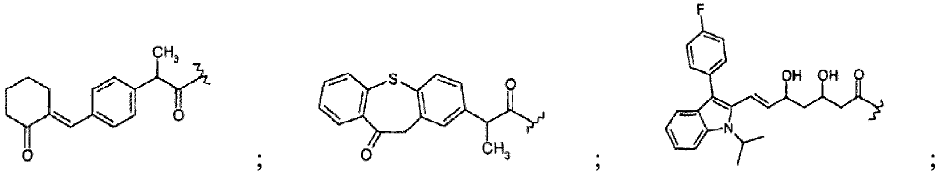 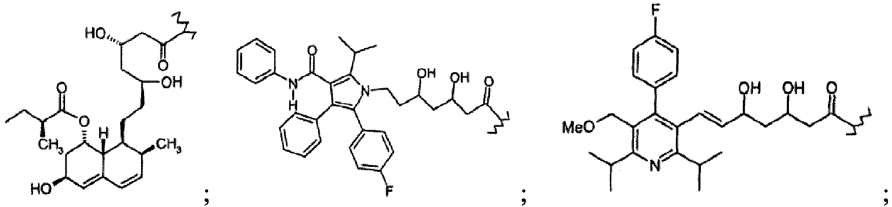 and 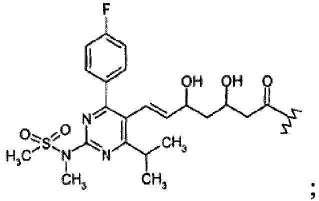
X is -O-, -S- or -NR¹-, wherein R¹ is H or a linear or branched C₁-C₆ alkyl group;
Y is a divalent group with the following definition:
   a)
      -Linear or branched C₁₋C₂₀ alkylene, optionally substituted by one or more substituents selected from the group consisting of: halogen, hydroxyl, -ONO₂, -OC(O)(C₁-C₁₀ alkyl)-ONO₂ and -O(C₁-C₁₀ alkyl)-ONO₂;
      -C₅-C₇ cycloalkylene, which is optionally substituted by a linear or branched C₁-C₁₀ alkyl group;
   b) 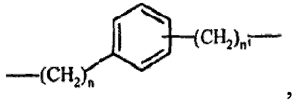 wherein n is an integer selected from 0-20, and n' is an integer 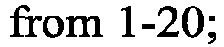
   c) 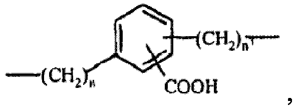 wherein n is an integer selected from 0-20, and n' is an integer from 1-20;
   d) 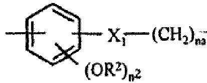 wherein X₁ is -OCO- or -COO-, and R² is H or CH₃, na is an integer from 1-20, and n² is an integer from 0-2;
   e) 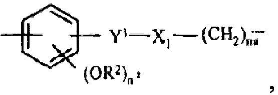 wherein Y¹ is -CH₂-CH₂-(CH₂)n²-, or -CH=CH-(CH₂)n²-, X₁, na, n² and R² are as defined above;
   f) 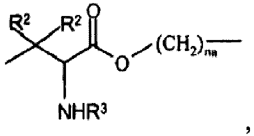 wherein na and R² are as defined above, and R³ is H or -COCH₃; or
   g) 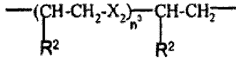 , or 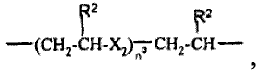 wherein X₂ is -O- or -S-, n³ is an integer from 1 to 6, and R² is defined as above.

In some embodiments, the NO-NSAID compound comprises a structure represented by Formula (3):

M-X-Y-ONO₂ (3),

wherein M is selected from the following structures: 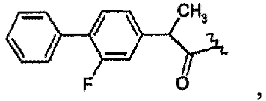 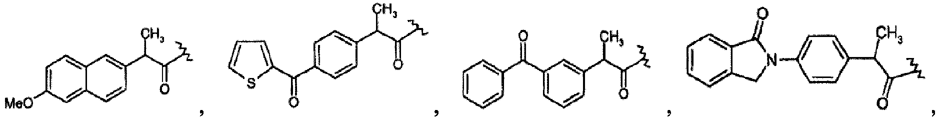 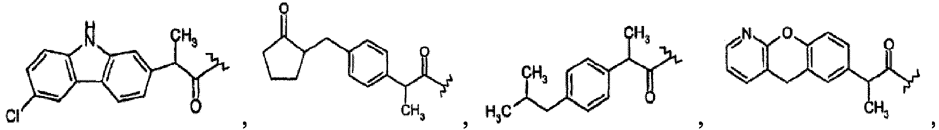 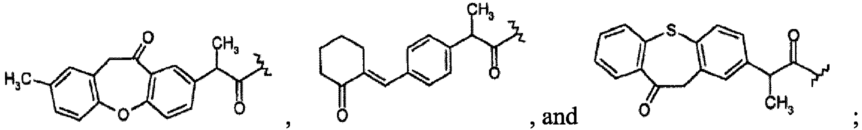
X is -O-; Y is a linear or branched C₁-C₁₀ alkylene group.

In some embodiments, the NO-NSAID compound comprises a structure represented by formula (4):

A-(B)*_{b0}*-(D)*_{c0}*-NO₂ (4)

wherein:
c0 is 0 or 1, b0 is 0 or 1, and at least one of c0 and b0 is not 1;
A is R-T₁-, wherein R-T₁- is a free radical of the formula R-T₁-OH or a prodrug of acetylsalicylic acid, wherein T₁ = (CO), and R is the remaining radical of acetylsalicylic acid;
B is T_{B}-X₂-T_{BI}-, TB is X, T_{BI} is (CO) or X, X is O, S, N or NR_{1C}, Ric is H or a linear or branched alkyl group having 1 to 5 carbon atoms; X₂ is a group of a compound of formula H-X₂-H selected from the group consisting of hydroxy acid, gallic acid, ferulic acid, gentisic acid, citric acid, caffeic acid, p-coumaric acid and vanillic acid;
D is a divalent group: -T_{c}-Y-, wherein when b0 is 1, T_{c} is (CO) when T_{BI} is X , or T_{c} is X when T_{BI} is (CO), and X is O, S, N or NR_{1C}, Ric is H or a linear or branched alkyl group with 1 to 5 carbon atoms; when b0 is 0, T_{c} =X, and X is O, S, N or NR_{1C}, Ric is H or a linear or branched alkyl group with 1 to 5 carbon atoms;
Y is Y*ₚ*, Y*_{O}* or Y*_{Ar}* ,
Y*ₚ* is 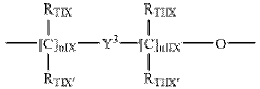 , wherein nIX is an integer from 0 to 3, nIIX is an integer from 1 to 3, R*_{TIX}, R_{TIX'},* R*_{TIIX}* and R*_{TIIX'}* are each independently H or linear or branched C₁-C₄ alkyl, Y ³ is a saturated, unsaturated or aromatic heterocyclic ring containing one or two nitrogen atoms and has 6 atoms;
Y*_{O}* is selected from the group consisting of: C₁₋C₂₀ linear or branched alkyleneoxy, 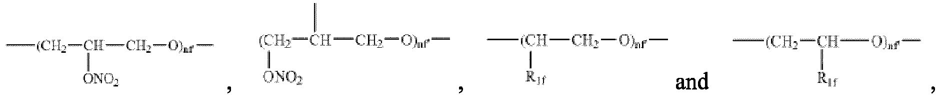 wherein nf is an integer from 1 to 6, and R_{1f} is H or CH₃,
*Y_{Ar}* is 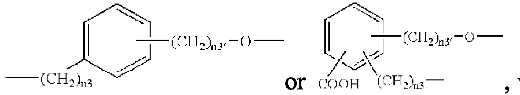 wherein n3 is an integer from 0 to 3, and n3' is an integer from 1 to 3; and
When b0 is 1, c0 is 1 and Y is Y*_{O}*, or when b0 is 0, c0 is 1 and Y is Y*ₚ* or Y*_{Ar}*.

In some embodiments, the NO-NSAID compound comprises a structure represented by Formula (5):

A-X₁-L-(W)*ₚ*-NO₂ (5)

wherein:
p is 0 or 1;
A is R-T₁, wherein R is the residue of the prodrug and has the following formula: 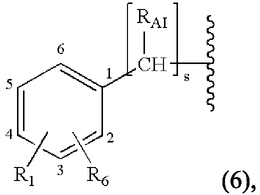 wherein s is 0 or 1; R_{AI} is H or CH₃;
R₁ is OCOR₃, NHCOR₃, OH, CH₂CH(CH₃)₂, phenyl, benzoyl or 4,6-dichlorophenylamino, R₃
is a C₁-C₅ linear or branched residue; R₆ is H or halogen;
Alternatively, when R₁ and R₆ are located at the adjacent 4- and 5-positions on the aromatic ring of formula (6), the structure of formula (7) is formed:

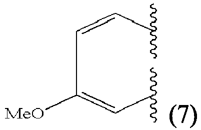

Alternatively, R has the structure of the following formula: 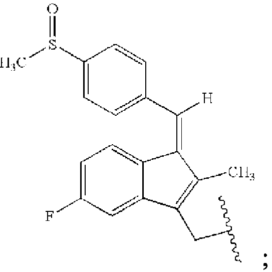
T₁ is (CO)t or (X)_{t'}, wherein X is O, S or NR_{1c}, R_{1c} is H or a linear or branched alkyl group having 1 to 5 carbon atoms, t and t' are 0 or 1, t is 1 when t' is 0, and t is 0 when t' is 1;
X₁ is -T_{B}-Y-T_{BI}, wherein T_{B} and T_{BI} can be the same or different. When t is 0, T_{B} is (CO), when t' is 0, T_{B} is X, and X is as defined above; T_{BI} is (CO)ₜₓ or (X)ₜₓₓ, wherein tx and txx are 0 or 1, when txx is 0, tx is 1, when txx is 1, tx is 0, and X is as defined above;
Y is a divalent linking group selected from the following: 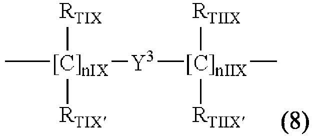
Wherein: nIX is an integer from 0 to 3, nIIX is an integer from 1 to 3, R_{TIX}, R_{TIX'}, R_{TIIX} and R_{TIIX'} are each independently selected from H and C₁-C₄ linear or branched alkyl groups; Y³ is a saturated, unsaturated or aromatic heterocyclic ring with 5 or 6 atoms and containing 1 or 2 nitrogen atoms;
C₁₋C₂₀ linear or branched alkylene, which is optionally substituted with a substituent selected from the group consisting of: NHCOR₃, -NH₂ and -OH, R₃ is a C₁-C₅ linear or branched residue;
A cycloalkylene group having 5 to 7 carbon atoms, optionally substituted with a C₁₋C₂₀ linear or branched alkylene side chain, which is optionally substituted with a substituent selected from the group consisting of: NHCOR₃, -NH₂ and -OH, R₃ is a C₁-C₅ linear or branched residue, wherein one or more carbons atoms in the cycloalkylene ring are optionally replaced by a heteroatom; 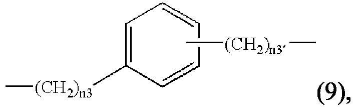 wherein n3 is an integer from 0 to 3, and n3' is an integer from 1 to 3; 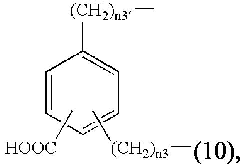 wherein n3 is an integer from 0 to 3, and n3' is an integer from 1 to 3; 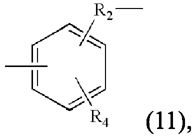 wherein R ₄ is hydroxyl, hydrogen or R₅O, R₅ is C₁-C₁₀ linear, branched or cyclic alkyl group, and R₂ is C₂-C₁₀ linear or branched alkenylene containing one or more double bonds; 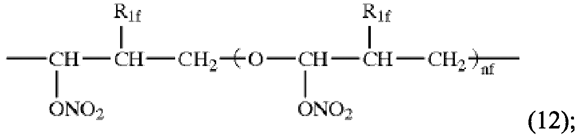 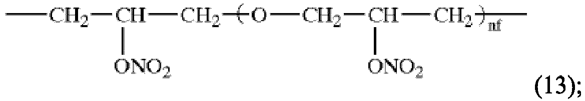 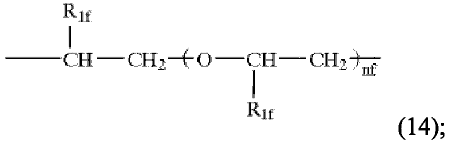 and 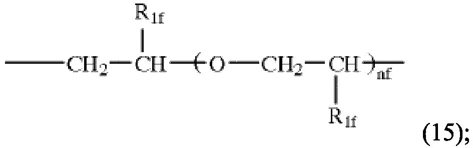
Wherein R_{1f} is H or CH₃, and nf is an integer from 0 to 6;
L is a covalent bond, CO or X, as defined above; and
W is Y_{T}O, Y_{T} has the same meaning as Y, and in the compound of formula (5), Y and Y_{T} can be the same or different.

In some embodiments, the NO-NSAID compound comprises a structure represented by formula (5):

A-X₁-L-(W)*ₚ*-NO₂ (5),

wherein:
The p is 0 or 1,
A is R-T₁-, wherein R has the structure of 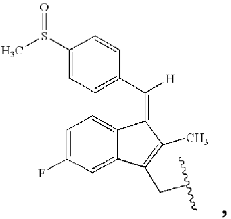 and
T₁ is (CO)ₜ or (X)_{t'}, t and t' are 0 or 1, t' is 0 when t is 1, and t' is 1 when t is 0;
X₁ is -T_{B}-Y-T_{B1}-, wherein T_{B} and T_{B1} are the same or different, when t is 0, T_{B} is (CO), and when t' is 0, T_{B} is X;
T_{B1} is (CO)tx or (X)txx, wherein tx and txx are each independently 0 or 1, tx is 1 when txx is 0, and tx is 0 when txx is 1;
X is O, S or NR_{1C}, R_{1C} is H or a linear or branched alkyl group having 1 to 5 carbon atoms;
Y is selected from 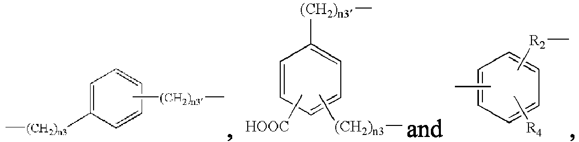 wherein n3 is an integer from 0 to 3, n3' is an integer from 0 to 3, R₄ is hydroxyl, hydrogen or RsO-alkoxy, wherein R₅ is a C₁-C₁₀ linear, branched or cyclic alkyl, R² is C₂-C₁₀ linear or linear alkenylene and optionally contains one or more double bonds;
L is a covalent bond, CO, or X, X is O, S, or NR_{1C}, and R_{1C} is H or a linear or branched alkyl group with 1-5 carbon atoms; and
W is Y_{T}O, Y_{T} is selected from 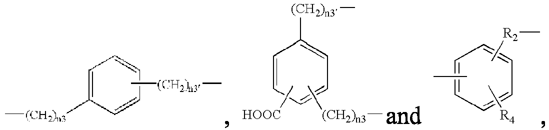 wherein n3 is an integer from 0 to 3, n3' is an integer from 0 to 3, R₄ is hydroxyl, hydrogen or RsO-alkoxy, wherein R₅ is C₁-C₁₀ linear, branched or cyclic alkyl, R² is C₂-C₁₀ linear or linear alkenylene and optionally contains one or more double bonds.

In some embodiments, the NO-NSAID compound comprises a structure represented by Formula (16): 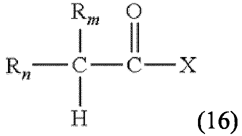
wherein Rₘ is H or lower alkyl,
Rₙ is a structure selected from the following group: 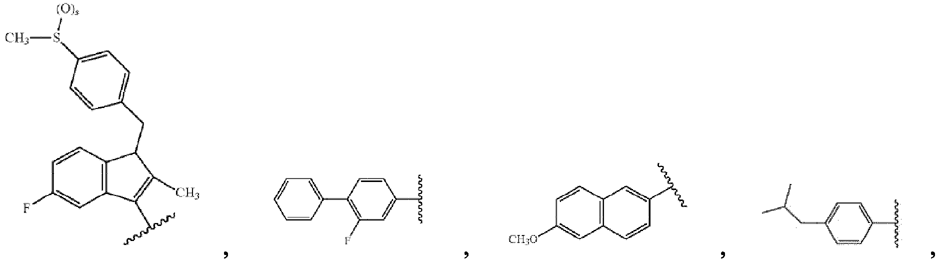 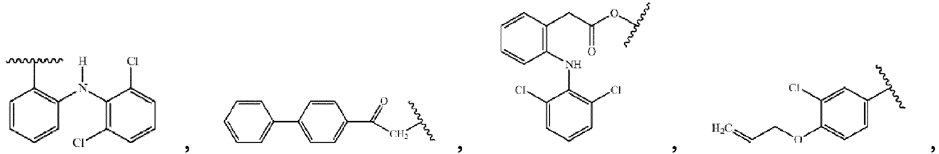 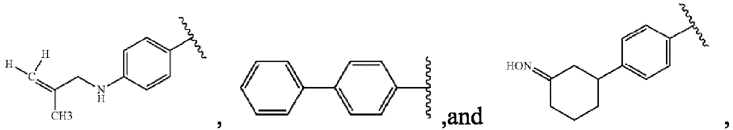
s is 0 or 1,
X is -Y-(CR₄R_{4'})*ₒ*-C(R₄)(ONO₂)-(CR₄R_{4'})*_{q}*-(T)*ₒ*-(W)*_{q}*(T)*ₒ*-(CR₄R_{4'})*ₒ*-R₅;,
Each R₄ and R₄' is independently hydrogen, lower alkyl, -OH, -CH₂OH, -ONO₂, -NO₂ or - CH₂ONO₂, or R₄ and R₄' together with the carbon atom to which they are connected to form a cycloalkyl or heterocycle;
W is a covalent bond or carbonyl group;
Each T is independently oxygen, (S(O)ₒ)ₒ or NRⱼ;
Rⱼ is hydrogen, alkyl, aryl, heterocycle, alkylcarbonyl, alkylaryl, alkylsulfinyl, alkylsulfonyl, arylsulfinyl, arylsulfonyl, sulfonamido, N-alkylsulfonamido, N, N-diarylsulfonamido, N-arylsulfonamido, N-alkyl-N-arylsulfonamido, formamido and/or hydroxyl;
Each q is independently 1, 2 or 3;
Each o is independently 0, 1 or 2;
Y is oxygen or sulfur; and
R₅ is hydrogen, hydroxyl, alkyl, aryl, alkylsulfonyl, arylsulfonyl, carboxylic ester, alkylcarbonyl, arylcarbonyl, formamido, alkoxyalkyl, alkoxyaryl, cycloalkyl and/or heterocycle.

In some embodiments, the NO-NSAID compound comprises a structure represented by Formula (17) or Formula (18):

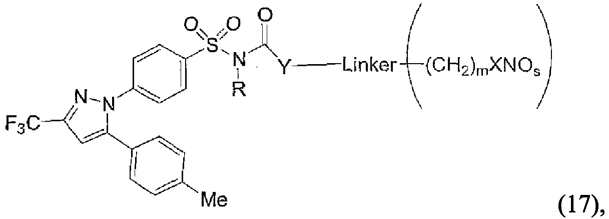

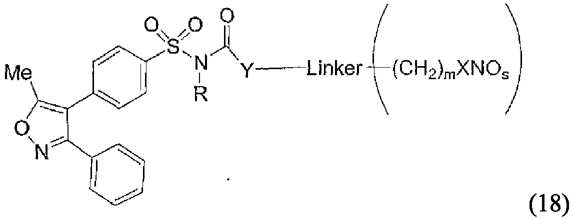

Each s is independently 1 or 2; k is 1, 2, 3 or 4; each m is independently 0, 1, 2, 3 or 4; each X is independently O or S;
Y is a bond, S, O or NRi, wherein R ₁ is hydrogen or C₁-C₆ alkyl;
R is hydrogen or C₁-C₆ alkyl;
The Linker is a linker, which is selected from the following group:
   1)-(CHz)n, n is 0, 1, 2, 3 or 4;
   2) C₃-C₆ cycloalkyl, optionally substituted with a group selected from the group consisting of halogen, C₁-C₃ alkyl, C₁-C₃ alkoxy, hydroxyl, NO, CO₂, CF₃, CN, CH₂COOH, CH₂COO-C₁₋₃ alkyl and C₁-C₃ thioalkyl;
   3) Aryl group, the aryl group is selected from phenyl and naphthyl, the aryl group is optionally substituted with a group selected from the following group: halogen, C₁-C₃ alkyl, C₁-C₃ alkoxy, hydroxyl, NO₂, CO₂, CF₃, CN, CH₂COOH, CH₂COO-C₁₋₃ alkyl and C₁-C₃ thioalkyl; and
   4) Heteroaryl, which is optionally substituted by a group selected from the group consisting of: halogen, C₁-C₃ alkyl, C₁-C₃ alkoxy, hydroxyl, NO₂, CO₂, CF₃, CN, CH₂COOH, CH₂COO-C₁₋₃ alkyl and C₁-C₃ thioalkyl.

In some embodiments, the NO-NSAID compound comprises a structure represented by Formula (19):

A-T-Y-ONO₂ (19),

wherein:
A is a medicament selected from the following group: a residue of (A-OH or AH), a non-steroidal anti-inflammatory drug, an analgesic, an antipyretic and a COX-2 inhibitor;
T is -O-, -NH-, -S-, -CO- or -(CH₂)ₙ₁OCO-, wherein n1 is an integer from 1 to 20;
A is selected from the group consisting of 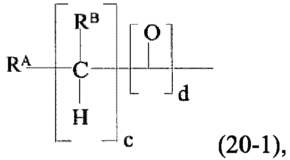
wherein c and d are each independently 0 or 1,
R^{B} is selected from: H, linear or branched C₁-C₁₂ alkyl and C₂-C₁₂ alkenyl;
When c is 0 and d is 1, R^{A} is a structure selected from the following group: 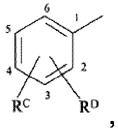 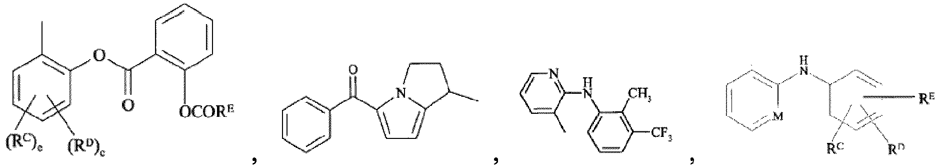 and 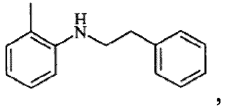
R^{c} is selected from the group consisting of H, halogen, amino, R^{E}CONH- and -OCOR^{E},
R^{D} is selected from the group consisting of: H, OH, halogen, linear or branched C₁-C₄ alkyl, linear or branched C₁-C₄ alkoxy, trifluoromethyl, amino and mono- or di-(C₁-C₄) alkylamino,
R^{E} is selected from H and linear or branched C₁-C₅ alkyl,
e is 0 or 1,
M is C or N;
When c is 1, d is 1, and R^{B} is hydrogen, R^{A} is a structure selected from the following group: 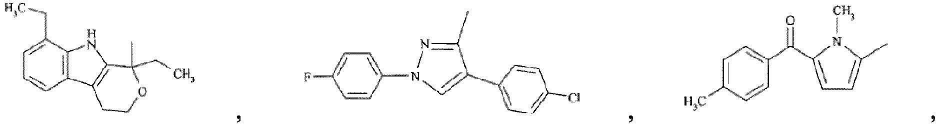 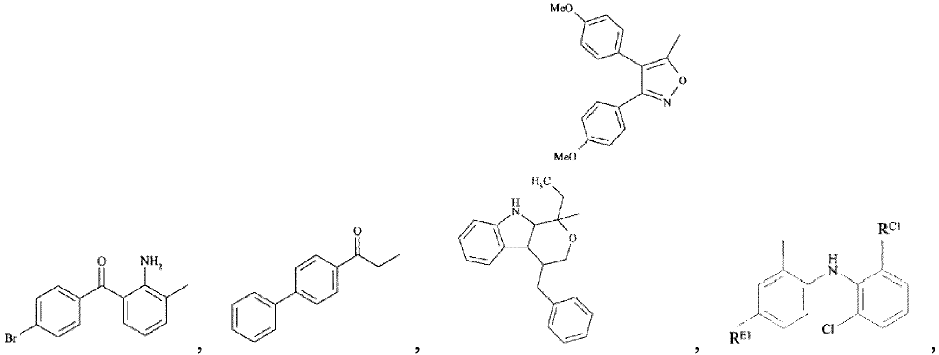 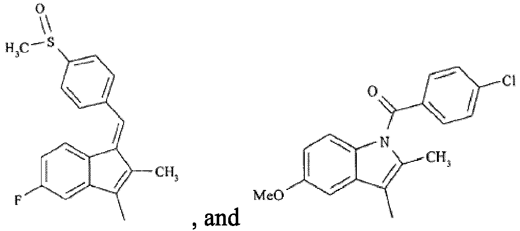
wherein R^{E1} is H or CH₃, and R^{C1} is Cl or F;
When c is 1, d is 1, and R^{B} is CH₃, R^{A} is a structure selected from the following group: 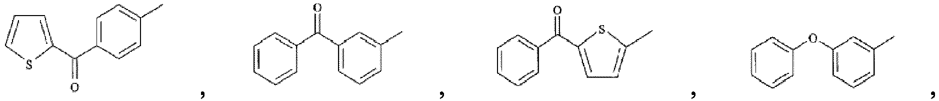 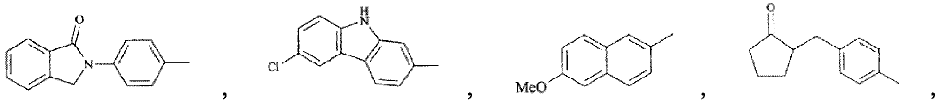 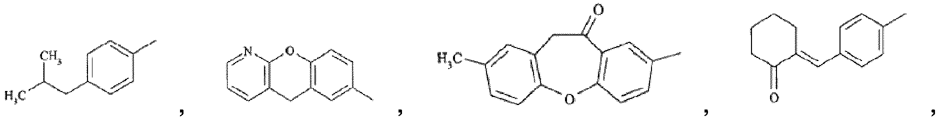 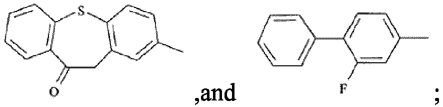
When c is 0 and d is 0, R^{A} is a structure selected from the following group: 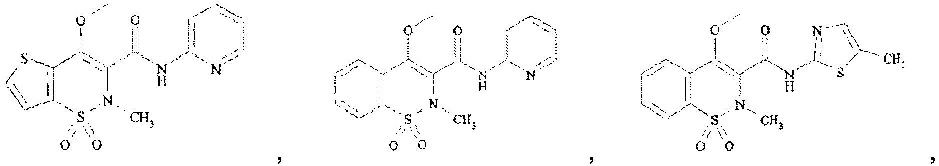 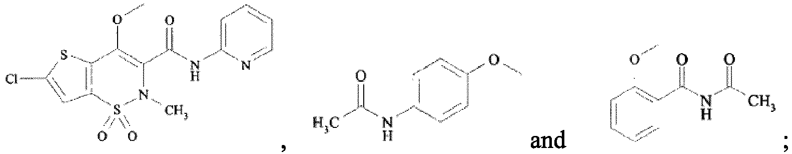 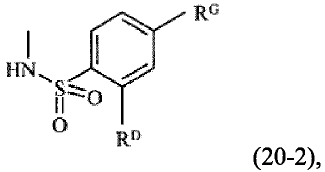
Wherein:
   R^{D} as descried above,
   R^{G} is a structure selected from the group consisting of: 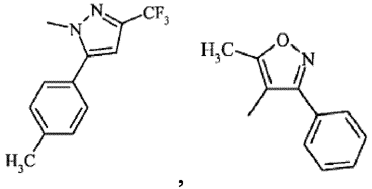 and 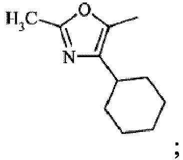 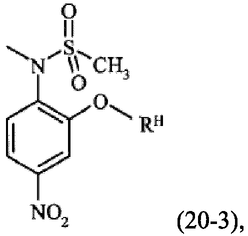
   Wherein R^{H} is phenyl or cyclohexyl; 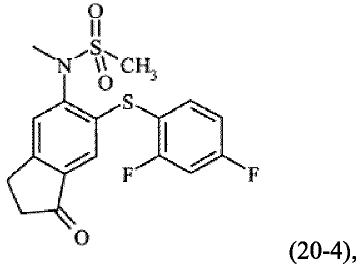
   Y is a divalent group with the following meaning:
      a)
         - Linear or branched C₁₋C₂₀ alkylene;
         - Cycloalkylene, the cycloalkylene has 5 to 7 carbon atoms in the ring, which is optionally substituted by the side chain R¹, wherein R¹ is a linear or branched alkyl having 1 to 10 carbon atoms;
      b) 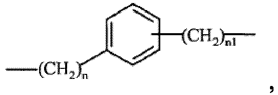 wherein n is an integer from 0 to 20, and n1 is an integer from 1 to 20;
      c) 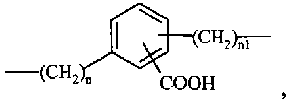 wherein n is an integer from 0 to 20, and n1 is an integer from 1 to 20;
      d) 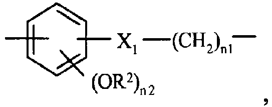 n1 is an integer from 1 to 20, and n2 is an integer from 0 to 2, X₁ is -OCO- or -COO-, and R² is H or CH₃;
      e) 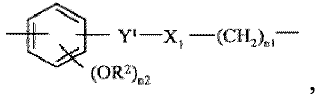 wherein n1, n2, R² and X₁ are as described above, Y¹ is - CH₂-CH₂- or -CH=CH-(CH₂)ₙ₂-;
      f) 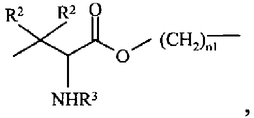 wherein n1 and R² are as described above, and R³ is H or COCH₃;
      g) 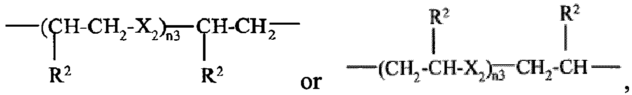 wherein X₂ is -O- or -S-, n3 is an integer from 1 to 6, and R² is as described above;
      h) 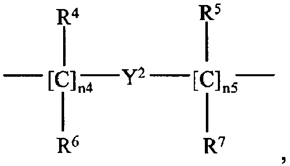 n4 is an integer from 0 to 10, n5 is an integer from 1 to 10, R⁴, R⁵, R⁶, R⁷ are each independently selected from H and linear or branched C₁-C₄ alkyl, and -ONO₂ is connected to 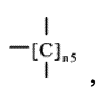 , n5 is as described above, Y² is a 5- or 6-membered saturated, unsaturated or aromatic ring, and contains one or more heteroatoms selected from the group consisting of nitrogen, oxygen and sulfur.

In some embodiments, the NO-NSAID compound comprises a structure represented by Formula (21): 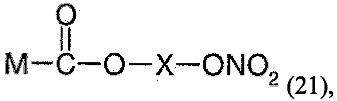 wherein
X is a linker and M is a structure selected from the following group: 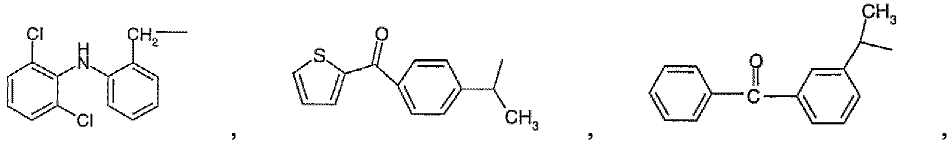 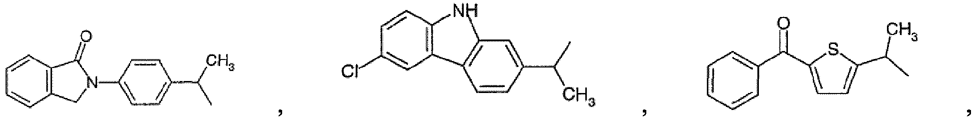 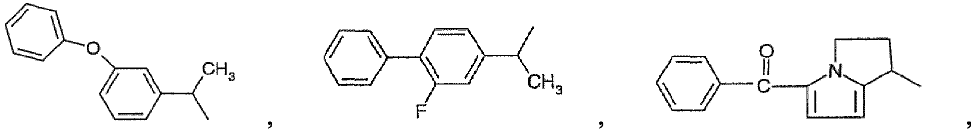 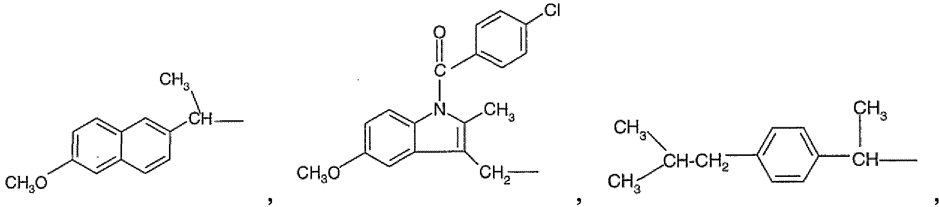 and

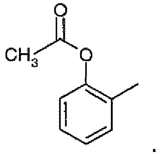

In some embodiments, the NO-NSAID compound comprises a compound selected from the group consisting of: 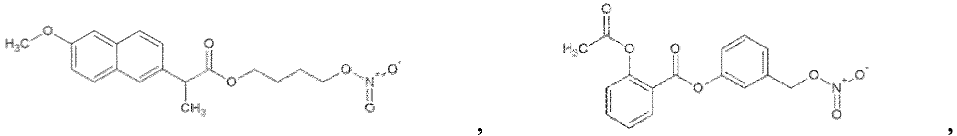 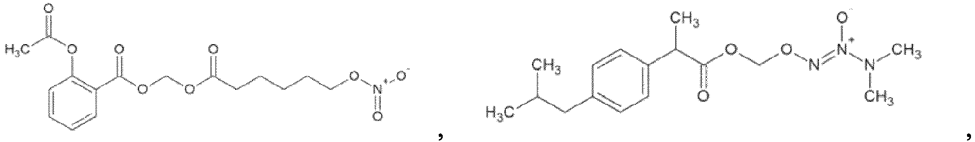 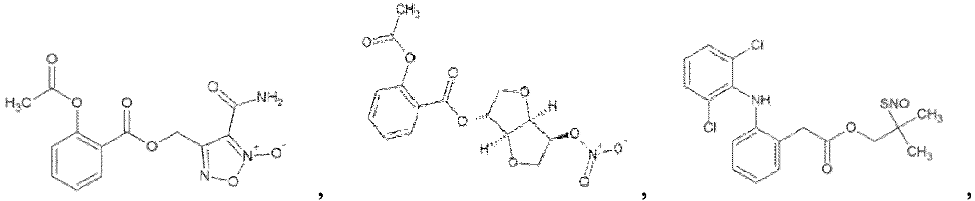 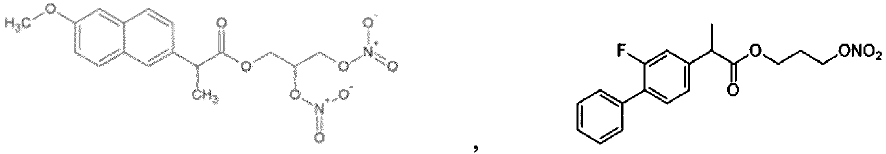 and

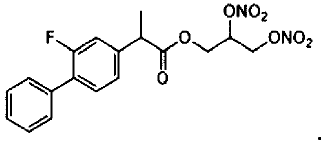

In some embodiments, in the NO-NSAID compound, the molar ratio of the NO-releasing moiety to the NSAID moiety is from about 2:1 to about 1:2.

In some embodiments, the anti-tumor agent comprises a small molecule compound, a small molecule conjugate, a protein and/or a polynucleotide.

In some embodiments, the anti-tumor agent comprises a chemotherapeutic agent, a targeted therapeutic agent, and/or an immunotherapeutic agent.

In some embodiments, the anti-tumor agent is a targeted therapeutic agent.

In some embodiments, the targeted therapeutic agent comprises a small molecule compound and/or an antibody or an antigen-binding fragment thereof.

In some embodiments, the antibody comprises a monoclonal antibody, a multispecific antibody, a chimeric antibody, a humanized antibody, a fully human antibody and/or an antibody-drug conjugate.

In some embodiments, the antigen-binding fragment comprises Fab, Fab', F(ab)₂, Fv fragment, F(ab')₂, scFv, di-scFv and/or dAb.

In some embodiments, the targeted therapeutic agent targets a molecule within a tumor cell, on the surface of a tumor cell, and/or in the tumor microenvironment.

In some embodiments, the targeted therapeutic agent targets a protein and/or a nucleic acid molecule.

In some embodiments, the targeted therapeutic agent targets a tumor-associated antigen.

In some embodiments, the targeted therapeutic agent targets one or more targets selected from the group consisting of: VEGF, EGFR, EGFR1, EGFR2, EGFR3, EGFR4, HER2, HER3, HER4, VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR- ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

In some embodiments, the targeted therapeutic agent inhibits the activity of one or more targets selected from the group consisting of: VEGF, EGFR, EGFR1, EGFR2, EGFR3, EGFR4, HER2, HER3, HER4, VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof..

In some embodiments, the targeted therapeutic agent reduces the expression of one or more targets selected from the group consisting of: VEGF, EGFR, EGFR1, EGFR2, EGFR3, EGFR4, HER2, HER3, HER4, VEGFR, VEGFR1, VEGFR2 , VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

In some embodiments, the targeted therapeutic agent comprises a hormone, a signal transduction inhibitor, a gene expression modulator, an apoptosis inducer, an angiogenesis inhibitor, and/or a toxin delivery molecule.

In some embodiments, the targeted therapeutic agent is a tyrosinase inhibitor.

In some embodiments, the targeted therapeutic agent is a VEGFR inhibitor and/or a VEGF inhibitor.

In some embodiments, the VEGFR inhibitor inhibits VEGFR1, VEGFR2, and/or VEGFR3.

In some embodiments, the targeted therapeutic agent is an EGFR inhibitor. In some embodiments, the targeted therapeutic agent is a BRAF inhibitor. In some embodiments, the targeted therapeutic agent is a PDGFR inhibitor. In some embodiments, the targeted therapeutic agent is an FGFR inhibitor. In some embodiments, the targeted therapeutic agent is an mTOR inhibitor. In some embodiments, the targeted therapeutic agent is a HER2 inhibitor. In some embodiments, the targeted therapeutic agent is selected from one or more of the group consisting of: afatinib, dacomitinib, osimertinib, EAI045, gefitinib, almonertinib, pyrotinib, brigatinib, neratinib, olmutinib, bosutinib, icotinib, vandetanib, lapatinib, aflutinib, BPI-7711, mobocertinib, dovitinib, zorifertinib, varlitinib, orelabrutinib, acalabrutinib, brutinib, ibrutinib, dasatinib, pirtobrutinib, tolebrutinib, rilzabrutinib, fenebrutinib, evobrutinib, selumetinib, tivozanib, dovitinib, surufatinib, binimetinib, cobimetinib, trametinib, regorafenib, GSK-1120212, alpelisib, duvelisib, copanlisib, idelalisib, nortriptyline, inavolisib, dactolisib, apitolisib, parsaclisib, buparlisib, rigosertib, enzastaurin, paxalisib, leniolisib, ipatasertib, zotarolimus, sirolimus, everolimus, temsirolimus, sorafenib, apatinib, lenvatinib, sunitinib, cabozantinib, axitinib, nintedanib, brivanib, vatalanib, fruquintinib, dabrafenib, vemurafenib, encorafenib , pazopanib, crizotinib, panobinostat, erlotinib, rituximab, panitumumab, cetuximab, erfonrilimab, efactinib, cadonilimab, ramucirumab, bevacizumab, anlotinib, ponatinib, famitinib, erdafitinib, AZD4547, infigratinib (also referred to as infigratinib in the present application), BCD -217, amivantamab, MCLA-129, EMB-01, LY3164530, JNJ-61186372, anti-EGFR and cMet bispecific antibody, GB263, their pharmaceutically acceptable salts and any combination thereof.

In some embodiments, the targeted therapeutic agent is administered in combination with one or more other therapies.

In some embodiments, the anti-tumor agent is a chemotherapeutic agent.

In some embodiments, the chemotherapeutic agent comprises a pyrimidine nucleoside analog and/or a prodrug thereof.

In some embodiments, the chemotherapeutic agent comprises one or more selected from the group consisting of: capecitabine, cytarabine, docetaxel, doxorubicin, fluorouracil (5-FU), floxuridine, tegafur, idarubicin, paclitaxel, epirubicin, acelarin (NUC-1031), doxorubicin, leucovorin, cisplatin, paclitaxel, cyclophosphamide, vincristine and 5-FU prodrug.

In some embodiments, the 5-FU prodrug comprises tegafur, 5'-deoxy-fluorouridine, floxuridine, 2'-deoxy-fluorouridine, prodrug derivative of floxuridine, prodrug derivative of 2'-deoxy-fluorouridine, trifluoro-methyl-2'-deoxyuridine, 6-azauridine and/or 3-deazauridine.

In some embodiments, the chemotherapeutic agent is administered in combination with one or more other therapies.

In some embodiments, the one or more additional therapies comprises one or more additional anti-tumor therapies.

In some embodiments, the disease or disorder is caused by administration of the anti-tumor agent.

In some embodiments, the disease or disorder onset or progression after administration of the anti-tumor agent.

In some embodiments, the subject did not suffer from the disease or disorder prior to administration of the anti-tumor agent.

In some embodiments, the disease or disorder comprises an epithelial tissue disease or disorder.

In some embodiments, the epithelial tissue disease or disorder comprises a disease or disorder associated with endothelial cell lesions, and/or a disease or disorder associated with epithelial cell lesions.

In some embodiments, the endothelial cells comprise vascular endothelial cells.

In some embodiments, the epithelial cells comprise skin epithelial cells, oral epithelial cells, nasal epithelial cells, gastric epithelial cells, and/or intestinal epithelial cells.

In some embodiments, the disease or disorder comprises a skin disease or disorder, a sensory disease or disorder, and/or a gastrointestinal disease or disorder.

In some embodiments, the skin disease or disorder comprises alopecia, body odor, bullous dermatitis, dry skin, eczema, erythema multiforme, erythroderma, lipoatrophy, hair color changes, abnormal hair texture, hirsutism, hyperhidrosis, hyperkeratosis, hypertrichosis, hypohidrosis, lipohypertrophy, nail changes, nail discoloration, nail loss, nail ridges, skin pain, hands-foot syndrome, photosensitivity, pruritus, purpura, acneiform rash, maculopapular rash, scalp pain, skin atrophy, skin hyperpigmentation, skin hypopigmentation, skin induration, skin ulcers, Stevens-Johnson syndrome, subcutaneous emphysema, telangiectasia, toxic epidermal necrosis, rash and/or urticaria.

In some embodiments, the skin disease or disorder is hand-foot syndrome.

In some embodiments, the severity of the skin disease or disorder is NCI-CTCAE grade 1 or above, grade 2 or above, grade 3 or above, grade 4 or above, or grade 5.

In some embodiments, the subject comprises a cancer patient.

In some embodiments, the disease or disorder affects a different site than the cancer.

In some embodiments, the medicament does not substantially affect the therapeutic effect of the anti-tumor agent.

In some embodiments, the medicament is prepared for topical administration.

In some embodiments, the medicament is prepared for transdermal administration.

In some embodiments, the medicament is prepared as a cream, lotion, gel, ointment, salve, spray, liposomal formulation, liniment, and/or aerosol.

In some embodiments, the administration site of the medicament is different from the administration site of the anti-tumor agent.

In some embodiments, the administration site of the medicament is not the site of cancer occurrence or potential metastasis.

In some embodiments, the administration method of the medicament is different from the administration method of the anti-tumor agent.

In some embodiments, the concentration of the NO-NSAID compound in the medicament is from about 0.005% w/w to about 40% w/w.

In some embodiments, the concentration of the NO-NSAID compound in the medicament is from about 0.5% w/w to about 10% w/w.

In another aspect, the present application provides a method of preventing, ameliorating and/or treating a disease or disorder associated with administration of an anti-tumor agent in a subject, the method comprising administering to the subject in need thereof an effective amount of NO-NSAID compound or a pharmaceutically acceptable salt thereof.

In another aspect, the present application provides a NO-NSAID compound, or pharmaceutically acceptable salt thereof, for use in preventing, ameliorating, and/or treating a disease or disorder associated with administration of an anti-tumor agent in a subject.

In another aspect, the present application provides a pharmaceutical combination comprising: 1) an anti-tumor agent; and 2) a NO-NSAID compound.

In some embodiments, in the pharmaceutical combination, the anti-tumor agent and the NO-NSAID compound are not mixed with each other.

In some embodiments, in the pharmaceutical combination, the anti-tumor agent and the NO-NSAID compound are each independently present in a separate container.

In some embodiments, in the pharmaceutical combination, the NO-NSAID compound is prepared for transdermal administration.

In some embodiments, in the pharmaceutical combination, the NO-NSAID compound is prepared as an ointment.

In some embodiments, in the pharmaceutical combination, the concentration of the NO-NSAID compound is from about 0.005% w/w to about 40% w/w.

In some embodiments, in the pharmaceutical combination, the concentration of the NO-NSAID compound is from about 0.5% w/w to about 10% w/w.

In some embodiments, in the pharmaceutical combination, the NO-NSAID compound in 2) is capable of preventing, ameliorating and/or treating a disease or disorder associated with the anti-tumor agent in 1).

In some embodiments, in the pharmaceutical combination, the NO-NSAID compound in 2) does not substantially affect the therapeutic effect of the anti-tumor agent in 1).

In some embodiments, in the pharmaceutical combination, the NO-NSAID compound of 2) is administered before, simultaneously with, or after the anti-tumor agent of 1).

In another aspect, the present application provides a use of a NO-releasing agent and an NSAID in the preparation a medicament, the medicament is used for preventing, ameliorating and/or treating a disease or disorder associated with the administration of an anti-tumor agent in a subject.

In another aspect, the present application provides a method of preventing, ameliorating and/or treating a disease or disorder associated with administration of an anti-tumor agent in a subject, the method comprising administering to a subject in need thereof an effective amount of a NO-releasing agent and an NSAID.

In another aspect, the present application provides a pharmaceutical combination comprising: 1) an anti-tumor agent; 2) a NO releasing agent; and 3) an NSAID.

Other aspects and advantages of the present application will be readily apparent to those skilled in the art from the following detailed description. Only the exemplary embodiments of the present application are shown and described in the following detailed description. As will be appreciated by those skilled in the art, the disclosure of the present application allows persons skilled in the art to modify the disclosed embodiments without departing from the spirit and scope of the invention involved in the present application. Accordingly, the drawings and the description in the specification of the present application are merely exemplary, and not restrictive.

### BRIEF DESCRIPTION OF THE DRAWINGS

Specific features of the invention involved in the present application are set forth in the appended claims. The features and advantages of the invention involved in the present application can be better understood by referring to the exemplary embodiments detailed hereinafter and the accompanying drawings. A brief description of the drawings is as follows:
Figure 1 shows the model of hand-foot syndrome in rats.
Figure 2 shows the condition of rat paws after treatment with the medicament or pharmaceutical combination of the present application.
Figure 3 shows the pathological examination results of rat paws in each group.
Figure 4 shows the pain measurement results after medicament administration in each group, with "Blank" indicating the control group.
Figure 5 shows the pain measurement results after medicament administration in each group, with "Blank" indicating the control group.
Figure 6 shows the pain measurement results after medicament administration in each group, with "Blank" indicating the control group.
Figure 7 shows the pain measurement results after medicament administration in each group, with "Blank" indicating the control group.
Figure 8 shows the pain measurement results after medicament administration in each group, with "Blank" indicating the control group.
Figure 9 shows the pain measurement results after medicament administration in each group, with "Blank" indicating the control group.
Figure 10 shows the pain measurement results after medicament administration in each group, with "Blank" indicating the control group.
Figure 11 shows the pain measurement results after medicament administration in each group, with "Blank" indicating the control group.
Figure 12 shows the pain measurement results after medicament administration in each group, with "Blank" indicating the control group.
Figure 13 shows the pain measurement results after medicament administration in each group, with "Blank" indicating the control group.
Figure 14 shows the cytotoxicity alleviation effects of the medicaments in each group.
Figure 15 shows the cytotoxicity alleviation effects of the medicaments in each group.
Figure 16 shows the cytotoxicity alleviation effects of the medicaments in each group.
Figure 17 shows the cytotoxicity alleviation effects of the medicaments in each group.
Figure 18 shows the cytotoxicity alleviation effects of the medicaments in each group.
Figure 19 shows the cytotoxicity alleviating effects of the medicaments in each group.
Figure 20 shows the cytotoxicity alleviating effects of the medicaments in each group.
Figure 21 shows the cytotoxicity alleviation effects of the medicaments in each group.
Figure 22 shows the cytotoxicity alleviation effects of the medicaments in each group.
Figure 23 shows the cytotoxicity alleviation effects of the medicaments in each group.
Figure 24 shows the cytotoxicity alleviation effects of the medicaments in each group.
Figure 25 shows the cytotoxicity alleviation effects of the medicaments in each group.

### DETAILED DESCRIPTION

The embodiments of the present invention are described below by way of specific examples, and those familiar with this technology can readily appreciate other advantages and effects of the present invention from the disclosure of the present specification.

### Term Definition

In the present application, the term "NSAID" generally refers to "non-steroidal anti-inflammatory drugs". The term "non-steroidal" is used to distinguish these drugs from steroidal drugs, which have many effects including eicosanoid-depressing, anti-inflammatory effects, etc. As analgesics, NSAIDs are unique because they are non-narcotic. NSAIDs comprise aspirin, ibuprofen, and naproxen, etc. Most NSAIDs act as non-selective inhibitors of the enzyme cyclooxygenase, inhibiting both cyclooxygenase-1 (COX-1) and cyclooxygenase-2 (COX-2) isoenzymes. Cyclooxygenase catalyzes the formation of prostaglandins and thromboxanes from arachidonic acid (itself derived from the cellular phospholipid bilayer by phospholipase A2).

In the present application, the term "NO-NSAID compound" generally refers to an NSAID derivative that is capable of releasing nitric oxide (NO). For example, the NO-NSAID compound may comprise a nitrooxy derivative of the NSAID.

In the present application, the term "spacer" generally refers to a group that separates one compound or part thereof from another compound or part thereof. For example, the spacer may be a chemical bond, group or linker that connects one compound or part thereof to another compound or part thereof.

In the present application, the term "cyclooxygenase (COX) inhibitory moiety" generally refers to a molecule or part thereof capable of reducing the activity and/or function of cyclooxygenase *in vivo* and/or *in vitro.*

In the present application, the term "cyclooxygenase (COX)", also known as prostaglandin-endoperoxide synthase (PTGS), generally refers to an enzyme (e.g. isoenzyme family, EC 1.14.99.1), which is capable of forming prostaglandins from arachidonic acid, including thromboxane and prostaglandins, such as prostacyclin acid. It is a member of the animal-type heme peroxidase family, also known as prostaglandin G/H synthase. The specific reaction catalyzed by COX is the conversion of arachidonic acid to prostaglandin H2 via the short-lived prostaglandin G2 intermediate. The COX may include COX-1 and COX-2. Among them, those that specifically inhibit the COX-2 isozyme are referred to as COX-2 selective inhibitors. COX-1 and COX-2 have similar molecular weights, approximately 70kDa and 72kDa, respectively, and share 65% amino acid sequence homology with almost the identical catalytic sites. Both proteins have three domains: an N-terminal EGF-like domain, a small 4-helix membrane anchor, and a core heme peroxidase catalytic domain. Both form dimers. Among them, isoleucine at position 523 in COX-1 is replaced by valine in COX-2. The smaller Val523 residue in COX-2 allows access to a hydrophobic side pocket in the enzyme (sterically hindered by Ile523). Molecules that bind to this alternative site can be considered as COX-2 selective inhibitors.

As used herein, the term "VEGFR" generally refers to Vascular Endothelial Growth Factor Receptor which belongs to the Receptor tyrosine kinases (RTKs) family. It comprises primarily three subtypes, including VEGFR1, VEGFR2, and VEGFR3 as reported. Of those, VEGFR1, and VEGFR2 are primarily distributed on the surface of tumor vascular endothelium, regulating the tumor angiogenesis; and VEGFR3s is primarily distributed on the surface of lymph endothelium, regulating the tumor lymphangiogenesis. It is reported that VEGFR2 is the primary VEGF signal transduction receptors during the angiogenesis and mitosis processes. The VEGF family comprises primarily VEGF-A, VEGF-B, VEGF-C, VEGF-D, VEGF-E and placental growth factor (PGF). VEGF-A in combination with VEGFR-1 and VEGFR-2 can regulate almost all the cell responses to VEGF as reported. The activation of VEGFR-2 in endothelial cells results in increased proliferation, migration, survival and improved vascular permeability (see, Waldner, Maximilian J. et al., the Journal of Experimental Medicine 207.13, 2010). The expression of VEGFR or the increased kinase activity thereof is associated with a series of human cancers.

As used herein, the term "VEGF" generally refers to Vascular Endothelial Growth Factor which is known as the specific growth factor of the key endothelial cells required by pathological angiogenesis signal pathway according to reports. The inhibition of VEGF receptor tyrosine kinase signal pathway blocks the angiogenesis during the growth of tumors, resulting in stagnation or regression of angiogenic tumors (see, Gerald McMahon, the Oncologist 2000, 5:3-10).

As used herein, the term "VEGFR inhibitor" generally refers to any substance or agent capable of causing the decreased expression, quantity or activity of VEGFR which is known in the art or may be discovered in the future, comprising any substance which may cause an inhibition of biological activity associated with VEGFR activity when the substance or agent is administered to a subject (including an inhibition of downstream biological effect produced by combination of any VEGFR with its natural ligand). In some embodiments, the VEGFR inhibitor may comprise any agent capable of blocking the VEGFR activity or any downstream biological effect thereof during the treatment of cancers. For instance, the VEGFR inhibitor may be used to treat tumors. For instance, the VEGFR inhibitor may inhibit one or more functions of VEGFR directly. For instance, the VEGFR inhibitor may combine with a nucleic acid sequence encoding VEGFR. For instance, the VEGFR inhibitor may reduce the transcription level of VEGFR protein.

As used herein, the term "VEGF inhibitor" generally refers to any substance or agent capable of causing the decreased expression, quantity or activity of VEGF which is known in the art or may be discovered in the future, comprising any substance which may cause an inhibition of biological activity associated with VEGF activity when the substance or agent is administered to a subject. In some embodiments, the VEGF inhibitor may comprise any agent capable of blocking the VEGF activity or any downstream biological effect thereof during the treatment of cancers. For instance, the VEGF inhibitor may be used to treat tumors. For instance, the VEGF inhibitor may inhibit one or more functions of VEGF directly. For instance, the VEGF inhibitor may combine with a nucleic acid sequence encoding VEGF. For instance, the VEGFR inhibitor may reduce the transcription level of VEGF protein.

As used herein, whatever *in vitro* or *in vivo*, the method of detecting and/or evaluating the inhibition level of the VEGF and/or VEGFR is normal in the art. And the method may be used in verify, standardize, screen and/or evaluate the VEGF inhibitor and/or VEGFR inhibitor of the present application.

As used herein, the term "tumor" generally refers to neoformation formed by local tissue cell proliferation under the action of various tumorigenic factors, which is also known as neoplasm. According to the cellular properties of the neoformation and the harm extent to an organism, the tumors are in turn classified to two types, i.e., benign tumors and malignant tumors, and cancer is a general term for malignant tumors. The tumors may be selected from the group consisting of malignant epithelial malignant tumors (epithelium-derived cancers), lung cancer (e.g., non-small cell lung cancer), breast cancer, skin cancer, bladder cancer, colon cancer, bowel cancer, prostate cancer, pancreas cancer, uterine cancer, cervical cancer, ovarian cancer, esophagus cancer, head and neck cancer, stomach cancer and laryngeal cancer. For instance, the tumor may be liver cancer, kidney cancer, colorectal cancer, stomach cancer, esophageal cancer or thyroid cancer.

For instance, the VEGFR inhibitor may comprise a small molecular VEGFR inhibitor, a protein macromolecule specifically binding to VEGFR, an RNAi inhibiting the expression of a VEGFR protein and/or an antisense oligonucleotide inhibiting the expression of VEGFR protein.

As used herein, the term "small molecular VEGFR inhibitor" may comprise a small molecular VEGFR inhibitor reversibly or irreversibly binding to VEGFR or a small molecular VEGFR inhibitor specifically binding to a mutant VEGFR. For instance, the small molecular VEGFR inhibitor may comprise Regorafenib, Ponatinib, Cabozantinib, Lenvatinib, Sorafenib, Apatinib, Axitinib, Nintedanib, Vandetanib, Sunitinib, Midostaurin, Tivozanib, Fruquintinib, Cediranib, Brivanib, Donafenib, Surufatinib, Anlotinib, Famitinib, Tesevatinib, Vorolanib, Motesanib, Linifanib, Semaxanib, Dovitinib, Orantinib, Vatalanib, Telatinib, Glesatinib, Lucitanib, Ilorasertib, Rebastinib, Golvatinib, Foretinib, Ningetinib, Tafetinib, Altiratinib, TAS-115, Chiauranib, Henatinib, 4SC-203, AAL-993, ACTB-1003, AEE-788, AMG-628, Arenobufagin, BAW2881, BIBF-1202, BMS-690514, BMS-794833, CEP-11981, CEP-5214, CP-547632, CYC116, DW532, ENMD-2076, FIIN-1, GFB-204, BFH-772, BMS599626, BMS690514, PP 121, MGCD 265 analogue, AC480, Ki 8751, KRN 633, WHI-P 154, TAK593, JI 101, AZD-2932, SCR-1481B1, Isoliquiritigenin, JNJ-26483327, KI-20227, LY2457546, ODM-203, OSI-930, PF-00337210, CGP41231, R1530, RAF265, SAR131675, Semaxinib, SIM010603, SKLB1002, SKLB610, SU 5205, SU11652, SU14813, SU-1498, SU-4312, SU5402, T-1840383, Tanshinone IIA, TAS-115, TG 100572, TG 100801, TG100572 HCl, Toceranib, Tyrosine phosphorylation inhibitor A9, Tesevatinib, XL-844, XL999, ZD4190 HCl, ZM-306416, ZM323881 HCl, ABT-510, NVP-ACC789, ADT-OH, BMS-645737, EG 00229, XL-820, SGI-7079, Endostatin and/or Taxifolin.

As used herein, the term "specifically binding" generally means that in a complicated mixture, the VEGFR inhibitor may specifically recognize and bind to VEGFR, but does not substantially recognize or bind to other components in the complicated mixture. For instance, the affinity of the inhibitor to VEGFR may be at least 2 times the affinity of the inhibitor to other non-specific binding ingredients (e.g., at least 2 times, 3 times, 4 times, 5 times, 6 times, 7 times, 8 times, 9 times, 10 times or more). In some embodiments, the equilibrium dissociate constant of the VEGFR inhibitor with VEGFR is less than or equal to 10⁻⁶ (e.g., less than or equal to 10⁻⁷ M, less than or equal to 10⁻⁸ M, less than or equal to 10⁻⁹ M, less than or equal to 10⁻¹⁰ M or less).

As used herein, the protein macromolecule that specifically binds to VEGFR may be an antibody, the antibody variant, fusion protein, derivative or a fragment thereof that targets VEGFR. For instance, it may be an antibody that specifically binds to VEGFR or a fragment of antigen binding thereof.

As used herein, the antibody generally refers to a polypeptide molecule capable of specifically recognizing and/or neutralizing a specific antigen. For instance, the antibody may comprise an immunoglobulin composed of at least two heavy (H) chains and two light (L) chains that are linked to each other via disulfide bond, and comprise any molecule containing an antigen binding fraction thereof. The term "antibody" may comprise a monoclonal antibody, an antibody fragment or an antibody derivative, including, but not limited to, a human antibody (full humanized antibody), a humanized antibody, a chimeric antibody, a single-stranded antibody (e.g., scFv), and an antibody fragment binding to an antigen (e.g., Fab, Fab' and (Fab)₂ fragment). Of those, the chimeric antibody may be an antibody in which a part of the amino acid sequence of each heavy chain or light chain is homologous to the corresponding amino acid sequence of an antibody derived from a species, or belongs to a particular classification, while the remainder segment of the chain is homologous to the corresponding sequence of another species. Of those, the humanized antibody may refer to a chimeric antibody which comprise a small amount of sequence derived from a non-human immunoglobulin, thereby decreasing the immunogenicity when a hetero-antibody is introduced into humans, but keeping a complete antigen binding affinity and specificity of the antibody. Of those, the full humanized antibody may comprise antibody produced by human or human immune cells, or derived from a non-human source (e.g., a genetically modified non-human animal by using a humanized antibody library), or other sequences encoding a human antibody.

As used herein, the fragment of antigen binding of an antibody may be one or more fragments having a specific antigen binding function. The antigen binding function of an antibody may be achieved by a full-length fragment of the antibody. The antigen binding function of the antibody may also be achieved by means of: a heavy chain containing Fv, ScFv, dsFv, Fab, Fab' or F(ab')₂ fragment, or a light chain containing Fv, ScFv, dsFv, Fab, Fab' or F(ab')₂ fragment. (1) Fab fragment, that is a monovalent fragment composed of VL, VH, CL and CH domains; (2) F(ab')₂ fragment, that is a divalent fragment containing two Fab fragments linked by a disulfide bond in the hinge region; (3) Fd fragment composed of VH and CH domains; (4) Fv fragment composed of VL and VH domains of one arm of the antibody; (5) dAb fragment composed of VH domain (Ward et al., (1989) Nature 341: 544-546); (6) isolated complementary determining region (CDR); (7) a combination of two or more isolated CDRs that may be optionally linked via a linker; (8) Camelized single domain antibody, that is an antibody comprising two heavy chain variable regions but not containing light chain and (9) nanobody, comprising a heavy chain variable region, CH2 and CH3. Moreover, it may further comprise monovalent single-stranded molecule Fv (scFv) formed by pairing VL and VH (scFv) (see, Bird et al. (1988) Science 242: 423-426; and Huston et al. (1988) Proc.Natl.Acad.Sci. 85: 5879-5883). The term "portion of antigen binding" may further comprise an immunoglobulin fusion protein comprising binding domains selected from the group consisting of (1) a peptide of binding domain fused with a peptide of immunoglobulin hinge region; (2) a heavy chain CH2 constant region of Immunoglobulin fused with a hinge region; and (3) a heavy chain CH3 constant region of immunoglobulin fused with a CH2 constant region.

For instance, the protein macromolecule that specifically binds to VEGFR may be Ramucirumab, a fragment of antigen binding or a functional variant thereof.

As used herein, the term "VEGF trapping agent" generally refers to an agent capable of trapping VEGF by binding thereof. For instance, the VEGF trapping agent may be selected from the group consisting of Bevacizumab and Aflibercept.
I As used herein, the term "an agent of reducing the expression of VEGF" generally refers to a substance capable of directly or indirectly decreasing the expression of VEGF in a subject. For instance, the agent of reducing the expression of VEGF may be selected from the group consisting of Temsirolimus and Thalidomide.

As used herein, the term "RNAi" generally refers to RNA interference, which is generally an exogenous or endogenous double-stranded RNA molecule or a small molecular RNA, and typically inhibits the expression or translation of a target gene through the specific degradation of RNAi caused by targeting mRNA. In general, RNAi comprises two types of small molecular RNAs: microRNA (miRNA) and small interference RNA (siRNA), which are capable of binding to other mRNA molecules, thereby increasing or decreasing the activity thereof, e.g., preventing mRNA from being translated to protein. In a eukaryotic animal, RNAi path cleaves a long double-stranded RNA (dsRNA) by RNaseIII enzyme (e.g., Dicer, DCL or Drosha) to double-stranded siRNA fragments having about 20-23 nucleotides in length. Each siRNA is resolved to two single-stranded RNAs (ssRNA), i.e., a passenger chain and a guide chain. The passenger chain is degraded, while the guide chain is integrated into an RNA inducing silencing complex (RISC). When the guide chain is complementary to an mRNA molecule, the RISC cleaves the mRNA molecule, thereby resulting in the degradation of the mRNA molecule.

As used herein, an RNAi that inhibits the expression of a VEGFR protein may inhibit the expression or translation of VEGFR by targeting a mRNA encoding VEGFR to specifically degrading the mRNA. As used herein, the RNAi inhibiting the expression of VEGF protein may inhibit the expression or translation of VEGF by targeting an mRNA encoding VEGF to specifically degrading the mRNA.

As used herein, the term "oligonucleotide" generally refers to an oligomer or polymer of ribonucleic acid (RNA) or deoxyribonucleic acid (DNA) or any mimic thereof or structurally modified nucleic acid. The term comprises oligonucleotides consisting of naturally occurring nucleobases, pentose and covalent nucleoside (backbone), and non-naturally occurring oligonucleotides having similar function. The modified or substituted oligonucleotide is generally superior to the naturally occurring forms as they have, e.g. increased cell uptake, increased affinity to target nucleic acid, and increased stability in the presence of nuclease.

As used herein, the term "antisense oligonucleotide" generally refers to a single-stranded oligonucleotide having nucleobase sequence that may at least partially hybridize with the corresponding region or fragment of the target nucleic acid. As used herein, the antisense oligonucleotide may comprise about 8 to about 50 nucleobases.

As used herein, the VEGFR inhibitor is capable of inhibiting VEGFR1, VEGFR2 and/or VEGFR3. For instance, the VEGFR inhibitor is capable of inhibiting one, two or three of VEGFR1, VEGFR2, and VEGFR3.

In some embodiments, the anti-tumor agent may include Ramucirumab, Bevacizumab, Regorafenib, Ponatinib, Cabozantinib, Lenvatinib, Sorafenib, Pazopanib, Apatinib, Axitinib, Nintedanib, Vandetanib, Sunitinib, Midostaurin, Tivozanib, Fruquintinib, Cediranib, Brivanib, Donafenib, Surufatinib, Anlotinib, Famitinib, Tesevatinib, Vorolanib, Motesanib, Linifanib, Semaxanib, Dovitinib, Orantinib, Vatalanib, Telatinib, Glesatinib, Lucitanib, Ilorasertib, Rebastinib, Golvatinib, Foretinib, Ningetinib, Tafetinib, Altiratinib, TAS-115, Chiauranib, Henatinib, 4SC-203, AAL-993, ACTB-1003, AEE-788, AMG-628, arenobufagin, BAW2881, BIBF-1202, BMS-690514, BMS-794833, CEP-11981, CEP-5214, CP-547632, CYC116, DW532, ENMD-2076, FIIN-1, GFB-204, BFH-772, BMS599626, BMS690514, PP 121, MGCD 265 analogue, AC480, Ki 8751, KRN 633, WHI-P 154, TAK593, JI 101, AZD-2932, SCR-1481B1, Isoliquiritigenin, JNJ-26483327, KI-20227, LY2457546, ODM-203, OSI-930, PF-00337210, CGP41231, R1530, RAF265, SAR131675, Semaxinib, SIM010603, SKLB1002, SKLB610, SU 5205, SU11652, SU14813, SU-1498, SU-4312, SU5402, T-1840383, tanshinone IIA, TAS-115, TG 100572, TG 100801, TG100572 HCl, Toceranib, Tyrosine phosphorylation inhibitor A9, Tesevatinib, XL-844, XL999, ZD4190 HCl, ZM-306416, ZM323881 HCl, ABT-510, NVP-ACC789, ADT-OH, BMS-645737, EG 00229, XL-820, SGI-7079, Endostatin, Taxifolin, Aflibercept, and/or a pharmaceutically acceptable salt of the above substances.

As used herein, the term "pharmaceutically acceptable salt" may refer to a pharmaceutically acceptable salt of the compound. In some embodiments, the pharmaceutically acceptable salt may be selected from the group consisting of Sorafenib tosylate, Sunitinib malate, Pazopanib hydrochloride, and Dovitinib (TKI258) lactate.

As used herein, the VEGFR inhibitor and/or the VEGF inhibitor may be administered in combination with one or more additional therapies. In some embodiments, the one or more additional therapies may comprise one or more additional anti-tumor therapy. For instance, the additional anti-tumor therapy may comprise cytotoxic anticancer agents, immunotherapeutic anticancer agents, or hormonotherapeutic anticancer agents. The additional anti-tumor therapies may further comprise radiotherapy or surgery therapy.

As used herein, when a VEGFR inhibitor and/or a VEGF inhibitor is administered in combination with additional anti-tumor therapies, they may be simultaneously administered to a subject or individually administered at intervals. For instance, the additional anti-tumor therapy may be a part of a single dosage form, and mixed with the VEGFR inhibitor and/or the VEGF inhibitor to form a single composition. As another example, the additional anti-tumor therapy may be a separate agent, which is administered separately from the VEGFR inhibitor and/or the VEGF inhibitor. As used herein, if the additional anti-tumor therapy and the VEGFR inhibitor and/or the VEGF inhibitor form a single composition, the VEGFR inhibitor and/or the VEGF inhibitor may be present and/or administered at a level of about 1-99% (e.g., about 5-95%, about 1%,about 5%, about 10%, about 20%, about 30%, about 40%, about 50%, about 60%, about 70%, about 80%, about 90%, about 95% or about 99%) in relation to the total dosage.

As used herein, the term "skin tissue disease or disorder" generally refers to the pathologic changes occurred in the morphology, structure and/or function of skin (including hair and nail). For instance, the skin tissue disease or disorder may comprise, but is not limited to rash, hand-foot syndrome, pruritus, erythema, dry skin, alopecia, paronychia, pigmentation disorder, and the like.

As used herein, the term "rash" generally refers to a skin change capable of affecting the color, appearance, or texture of skin. Rash may be localized to only a portion of the body, or affect the overall skin. Rash further comprises urticaria.

As used herein, the term "hand-foot syndrome" is also known as Hand Foot Syndrome (HFS), or Palmar Plantar Erythrodysesthesia (PPE) , or Hand-foot skin reaction (HFSR) , which was first described in 1984 by Jacob Lokich and Cery Moor of New England Diaconis Hospital of Harvard Medical School. The typical clinical manifestations are progressive. The primary clinical manifestations comprise finger (toe) fever, pain, erythematous swelling, serious person for desquamation, ulcer, and severe pain, etc. The pathologic manifestations of HFS primarily comprise, e.g., basal keratinocyte vacuolar degeneration, skin perivascular lymphocyte infiltration, keratinocyte apoptosis, and skin edema, and the like. For instance, HFS may comprise palmar or plantar dysesthesia, or acral erythema caused by chemotherapy, or the like. Cancer patients may develop the corresponding symptoms during the process of chemotherapy or molecular targeted therapy (e.g., the VEGFR inhibitor and/or the VEGF inhibitor).

Currently, there are a variety of classification methods for hand-foot syndrome (HFS), wherein the criterion of the American National Cancer Institute (NCI) is relatively commonly used. This criterion classifies the hand-foot syndrome as 3 Grades: Grade 1 is minimal skin changes or dermatitis with paresthesia (e.g., fingerprints disappearance, pigmentation, erythema, decrustation, paresthesia, dysesthesia, skin numbness, etc.), but does not affect daily activities; Grade 2 is the same level of skin change as Grade 1 with pain, slightly affects daily activities, and the skin surface is intact; and Grade 3 is ulcerative dermatitis or serious painful cutaneous lesions, seriously affects daily life, and has obvious tissue damage (e.g., desquamation, blisters, hemorrhage, edema, etc.).

Moreover, the World Health Organization (WHO) classifies HFS as four grades: Grade 1 is a feeling of dysesthesia, paresthesia, or tingling in the hands and feet; Grade 2 is discomfort when holding objects and walking, painless swelling, or erythema; Grade 3 is painful erythema and swelling of palms and soles, periungual erythema and swelling; and Grade 4 is decrustation, ulceration, blistering and severe pain.

As used herein, the term "erythema" generally refers to local or systemic red maculae caused by localized or systemic expansion of dermal papillary capillary network.

As used herein, the term "paronychia" generally refers to an infectious lesion of soft tissues around finger (toe) nails, which is generally caused by subcutaneous invasion and reproduction of bacteria through slight skin damage near the nails, and has clinical manifestations including painful redness and swelling of the affected area, with inflammatory exudation, and granulation tissue proliferation, etc.

As used herein, the term "pigmentation disorder" generally refers to a disorder in which the skin exhibits a lighter or darker color as compared with normal conditions, produces stains, or discolors. Hypopigmentation is due to the insufficiently production of pigments in the body, while hyperpigmentation is due to the excess production of pigments in the body.

As used herein, the term "sensory organ disease or disorder" generally refers to a pathologic change occurred in the morphology, structure, and/or function of organs including ears, eyebrows, eyes, nose, mouth, etc. For instance, the sensory organ disease or disorder may comprise but are not limited to oral mucositis, xerostomia (dry mouth), epistaxis, nasopharyngitis and/or cheilitis.

As used herein, the term "nasopharyngitis" generally refers to inflammatory reactions of nasopharyngeal mucosa, submucosa and lymphoid tissues, and may be classified to acute nasopharyngitis and chronic nasopharyngitis. Symptoms comprise, but are not limited to, nasopharyngeal dryness and discomfort, sticky secretions different to be coughed up, and are often accompanied with nausea, severe cases of hoarseness, sore throat, headache, dizziness, fatigue, indigestion, low-grade fever, as well as other local or systemic symptoms. Nasopharyngeal examination shows chronic mucosa congestion, hyperplasia, hypertrophy, secretion covering or dry scab, etc.

As used herein, the term "cheilitis" generally refers to inflammatory disease or disorder occurring in lips. For instance, it may comprise inflammations of perioral skin, vermilion border, and/or buccal mucosa, and the like. It may be classified into: acute cheilitis and chronic cheilitis, according to the course of disease; erosive cheilitis, eczematous cheilitis, desquamative cheilitis, according to the clinic symptom characteristics; and chronic non-specific cheilitis, cheilitis glandularis, cheilitis of benign lymph adenosis, cheilitis granulomatosa, Melkersson-Rosenthal Syndrome, actinic cheilitis and allergic cheilitis, etc., according to the etiology and pathology.

As used herein, the term "gastrointestinal disease or disorder" generally refers to the pathological change of morphology, structure and/or function of gastric or intestinal tissues (e.g., digestive tract tissues from gastric pylorus to anus). For instance, the gastrointestinal disease or disorder may comprise, but is not limited to, diarrhea, vomiting, nausea, anorexia, constipation and/or abdominal pain, and the like.

As used herein, the term "epithelial tissue" comprises one or more layers of free and surface-closed cells covering the whole body, comprising, skin, mucus, cavity, slurry and gland spaces. All the epithelial layers comprise two special domains, that is, the top domain facing the mucus (or cavity) space and the basolateral membrane facing the serosal (or deep) space. Thus, an important function of epithelial tissues is to provide a proper barrier to separate and control a variety of physiological processes between the two spaces. The epithelial tissue comprises epithelial cells and endothelial cells.

As used herein, the term "epithelial disease or disorder" generally refers to disease or disorder caused by lesion of epithelial cell and/or endothelia cell. For instance, the epithelial disease or disorder may comprise rash, acne, skin pruritus, alopecia, hair change, erythema, skin exfoliation, herpes, hirsutism, hyper-pigmentation, nail disorders, paronychia and schizonychia, dry skin, hypersensitivity, mucositis, nasopharyngitis, nasopharyngitis, xerostomia, cheilitis, oral mucositis and/or gastrointestinal mucosal injury. As another example, the epithelial disease or disorder may also comprise skin epithelial cell disease or disorder (e.g., rash, acne, rosacea, atopic dermatitis, contact dermatitis, seborrheic dermatitis, lupus, scleroderma, pemphigus, pigmentation, melasma, vitiligo, urticaria, tinea corporis, pruritus, alopecia, hair change, erythema, paronychia and schizonychia, dry skin, hypersensitivity and psoriasis), oral epithelial cell disease or disorder (e.g., pemphigus, herpes labialis, herpetic stomatitis, granulomatous cheilitis, oral mucositis , pemphigoid, Sjogren syndrome, Bechet disease and oral sarcoidosis, etc.), nasal epithelial cell disease or disorder (epistaxis, sinusitis, nasal furuncle and nasal polyps, etc.), gastric epithelial cell diseases or disorders (e.g., gastritis, intestinal metaplasia, gastric perforation, gastric fistula, gastric ulcer and gastrointestinal polyp) and/or small intestinal epithelial cell disease or disorder (e.g., enteritis, Crohn's disease, enterobrosis, intestinal fistula, enterelcosis, ulcerative colitis and NSAIDs enteropathy).

In the present application, the term "nitric oxide releasing moiety" generally refers to any substance capable of contributing to, producing and/or releasing nitric oxide. In some embodiments, the nitric oxide releasing moiety may directly contribute to, produce and/or release nitric oxide. For instance, the nitric oxide releasing moiety may stimulate other substances to contribute to, produce and/or release nitric oxide. For instance, the nitric oxide releasing moiety serve as a reactant of a chemical or enzyme-catalyzed reaction and contributes to, produces and/or releases nitric oxide via such reaction. In some embodiments, the nitric oxide releasing moiety serves a catalyst of a chemical or enzyme-catalyzed reaction, and stimulates other substances to contribute to, produce and/or release nitric oxide via such reaction. The nitric oxide releasing moiety may comprises nitric oxide itself.

As used herein, the item "organic molecular" generally refer to compound comprising carbon element, and not comprising oxide of carbon, carbonic acid, carbonate, cyanogen, cyanide, oxocyanide, cyanate, thiocyanogen, metal carbide and of like.

As used herein, the term "polymer" generally means that any compound with a molecular weight of greater than 2000 Daltons, greater than 3000 Daltons, greater than 4000 Daltons, greater than 5000 Daltons, greater than 6000 Daltons, greater than 7000 Daltons, greater than 8000 Daltons, greater than 9000 Daltons, greater than 10000 Daltons, greater than 12000 Daltons, greater than 15000 Daltons or greater than 20000 Daltons.

As used herein, the term "small molecule" generally means that any compound with a molecular weight of 2000 Daltons or less, 1500 Daltons or less, 1200 Daltons or less, 1000 Daltons or less, 900 Daltons or less, 800 Daltons or less, 700 Daltons or less, 600 Daltons or less, 500 Daltons or less, 400 Daltons or less, 300 Daltons or less, 200 Daltons or less or 100 Daltons or less.

As used herein, the nitric oxide releasing agent may have one or more groups as follow: diazeniumdiolate, hydroxyldiazenesulfonic acid, S-nitrosothiol, furoxan, oxime, N-nitrosoamine, N-hydroxylguanidine, diazeniumdiolate, nitrate, nitrite, nitrate ester, nitrite ester, sydnonimine, sydnone, oxatriazol-5-imine, oxatriazol-5-one, hydroxylamine, dioxadiazacyclobutene, N-hydroxylnitrosoamine, N-nitrosoimine, hydroxycarbamide and metal nitrosamino complex.

For instance, the nitric oxide releasing agent may has one or more groups selected from Table 1:

**Table 1**

| NO. | Compound | Structure of compound | Description |
|---|---|---|---|
| 1 | Diazeniumdiolate | 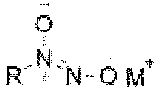 | Of those, R, R1, R2 may be organic chemical groups; |
| 2 | Diazeniumdiolate ester | 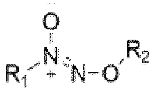 | M may be metal cation; |
| 3 | Hydroxyldiazenesulfonic acid | 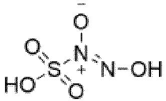 | X may be inorganic anion; |
| | | | a, b , n may be positive integer. |
| 4 | S-nitrosothiol | R-S-N=O | |
| 5 | Furoxan | 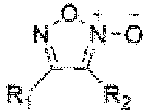 | |
| 6 | Oxime | 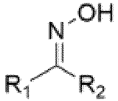 | |
| 7 | N-nitrosoamine | 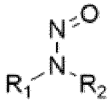 | |
| 8 | N-hydroxylguanidine | 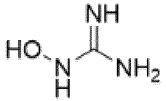 | |
| 9 | Nitrate | 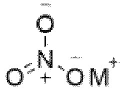 | |
| 10 | Nitrate ester | 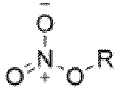 | |
| 11 | Nitrite | 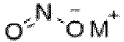 | |
| 12 | Nitrite ester | 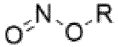 | |
| 13 | Sydnonimine | 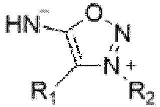 | |
| 14 | Sydnone | 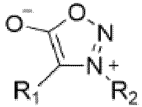 | |
| 15 | Oxatriazol-5-imine | 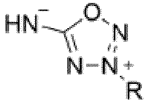 | |
| 16 | Oxatriazol-5-one | 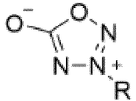 | |
| 17 | Hydroxylamine | H₂N-OH | |
| 18 | Dioxadiazacyclobutene | 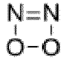 | |
| 19 | N-hydroxylnitrosoamine | 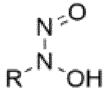 | |
| 20 | N-nitrosoimine | 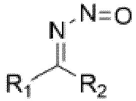 | |
| 21 | N- hydroxycarbamide | 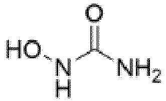 | |
| 22 | Metal nitrosamino complex | 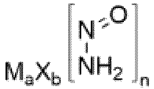 | |

As used herein, the term "prevent" may be interchangeably used with "prophylactically treat". In the present application, the term "prevent" generally refers to prevent the occurrence, reoccurrence or diffusion of diseases or one or more symptoms thereof by taking some actions in advance. As used herein, the term "treat" generally refers to eliminate or improve disease or ameliorate one or more symptoms associated with the diseases.

As used herein, the term "subject" generally refers to a human or non-human animal (including mammals, rodents, and birds, etc.) in need of accepting a diagnosis, prognosis, improvement, prevention, and/or treatment. For instance, the subject may be a livestock (such as cow, pig, goat, chicken, rabbit or horse), or rodents (such as rat and mouse), or primates (such as gorilla and monkey), domestic animals (such as dog and cat).

As used herein, the term "effective amount" refers to an amount of medicament capable of ameliorating or eliminating diseases or symptoms of the subject, or prophylactically inhibiting or prohibiting the occurrence of diseases or symptoms. In general, the effective amount may determine depending on the body weight, age, sex, diet, excretion rate, medical history, current therapy, time of administration, dosage form, administration mode, administration route, combination of medicaments, health conditions or cross infection potential of the subject, allergy, hypersensitivity, and side effects, and/or degree of epithelial (or endothelial) tissue disease development. The skilled man in the art (e.g., physicians or veterinarians) may decrease or increase the administration dosage in portion in accordance with these or other conditions.

As used herein, the term "does not substantially affect" may refer to, the therapeutic effect is equivalent, or not worse significantly. For instance, to any subject, the decreased tumor volume is equal or, or decreased by no less than about 5%, no less than about 4%, no less than about 3%, no less than about 2%, not less than about 1%, not less than about 0.5%, not less than about 0.1%, not less than about 0.01%, not less than about 0.001% or less.

As used herein, the term "susceptible to" generally refers to a subject have a greater probability of suffer a disease or disorder associated with administration of an anti-tumor agent. For instance, the greater probability may refer to the probability of a subject suffering the disease or disorder associated with administration of a VEGFR inhibitor and/or VEGF inhibitor increases by about at least 10%, at least 20%, at least 30%, at least 40%, at least 50%, at least 60%, at least 70%, at least 80%, at least 90%, at least 100%, at least 150%, at least 200% or higher, compared with that of a health subject.

As used herein, the term "features of skin tissue" generally refers to features capable of representing skin tissue disease or disorder. For instance, the features may comprise features capable of representing a disease or disorder associated with administration of the anti-tumor agent. For instance, the feature may comprise feature capable of representing rash associated with administration of the anti-tumor agent, hand-foot syndrome associated with administration of an anti-tumor agent, pruritus associated with administration of the anti-tumor agent, erythema and/or purpura associated with administration of the anti-tumor agent, dry skin and/or chapped associated with administration of the anti-tumor agent, alopecia associated with administration of the anti-tumor agent, paronychia associated with administration of the anti-tumor agent, and/or pigmentation associated with administration of the anti-tumor agent. For instance, the feature may comprise area and degree of erythema, the area and degree of purpura, the numbers and degree of pimples , the numbers and degree of pustule, the numbers and degree of nodule, the range and degree of skin swelling, the degree of skin ulcer, degree of xerosis, degree of skin chapped, degree of skin keratinization, degree of skin lichen, the area and degree of skin desquamation, state of skin tight, degree of skin pruritus, degree of vascular inflammation between dermis with hypodermic, the degree of necrotic skin tissue, the degree of skin ulcer, the area of livedo reticularis, the degree of skin pigmentation, the numbers of blister and bullae, part/area/degree of alopecia, degree of skin granulation, degree of skin seborrhea, degree of folliculitis, the degree of periungual swelling and redness, the degree of paronychia, the pigmentation of periungual skin, the degree of onychatrophia, onychatrophia or onychauxis, abnormal color of nail bed, nail stripe, nail ridge, nail pterygium and of like.

As used herein, the term "features of sensory organ" generally refers to the features representing sensory disease or disorder. For instance, the features may comprise the features representing the disease or disorder associated with administration of an anti-tumor agent. For instance, he features may comprise feature capable of representing an oral mucositis associated with administration of an anti-tumor agent, xerostomia associated with administration of an anti-tumor agent, epistaxis associated with the administration of an anti-tumor agent, nasopharyngitis associated with the administration of an anti-tumor agent, and /or cheilitis associated with administration of an anti-tumor agent. For instance, the feature may comprise congestion degree of oral mucosa, edema degree of oral mucosa, herpes degree of oral mucosa, ulcer degree of oral mucosa, degree of oral submucosa glandular defect, degree of lingual gland/ sublingual gland /parotid gland and atrophy, the degree of xerostomia, the degree of caries, the degree of tongue swelling, the degree of peripheral tooth mark, frequency of nosebleed, the amount of nosebleed, degree of oropharynx and nasopharynx mucosal edema, degree of oropharynx and nasopharynx mucosal herpes, degree of oropharynx and nasopharynx mucosal ulcer, degree of oropharynx and nasopharynx mucosal hyperplasia, degree of oropharynx and nasopharynx follicular hyperplasia, the degree of lip and around-lip swelling, the degree of lip and around-lip herpes, degree of lip and around-lip erythema, degree of lip and around-lip skin desquamation, degree of lip and around-lip skin lichenification and the degree of lip and around-lip skin erosion and of like.

As used herein, the term "features of gastrointestinal tract" generally refers to feature capable of representing gastrointestinal disease or disorder. For instance, the feature may comprise feature capable of representing a disease or disorder associated with administration of an anti-tumor agent. For instance, the feature may comprise capable of representing an esophagitis associated with administration of the anti-tumor agent, esogastritis associated with the administration of the anti-tumor agent, gastric ulcer associated with the administration of the anti-tumor agent, diarrhea associated with the administration of an anti-tumor agent, vomiting associated with the administration of the anti-tumor agent, nausea associated with the administration of the anti-tumor agent, anorexia associated with the administration of the anti-tumor agent, constipation associated with the administration of the anti-tumor agent, and/or abdominal pain associated with the administration of the anti-tumor agent . For instance, the feature may comprise degree of appetite loss, degree of stomach belching, degree of swallowing hard, degree of burning sensation of the sternum, degree of sternal pain, time and degree of upper abdominal pain (when hungry or satiety), degree of bloating, degree of diarrhea, numbers of defecation, defecation time, abdominal pain before defecation, rectal tenesmus, abnormal defecation (such as black-blood stool, fresh-blood stool, mucus stool, mucus-blood stool, watery stool, egg-dropping stool, etc.), vomiting frequency, vomitus situation, nausea, degree of malnutrition, degree of micronutrient deficiency, etc.

As used herein, the item "the location substantially not comprising tumor cells" generally refer to a tissue organ or location where the amount of cancer cell is low enough to be reckoned as substantially not comprised. For instance, the substantially not comprise may refer to the amount of cancer cells is less than 0.01%, less than 0.005%, less than 0.001%, less than 0.0001%, less than 0.00001% or less of the amount of overall cells of this location.

As used herein, the term of "no-cancer location" generally refer to a site that is not a cancer nidus or cancer metastatic site. For instance, the cancer nodus may be an occurrence site of cancer. For instance, the cancer metastatic site may be a place of the same tumor as the tumor of the occurrence site. For instance, the cancer metastatic site may be formed by a lymphatic metastasis, vascular metastasis or transcoelomic metastasis.

In the context of the present invention (especially in the context of the following claims, unless otherwise stated herein or clearly contradictory to the context, the terms "a" and "an" and "the" and "at least a/an/one" and similar referents are to be understood as comprising both singular and plural forms. Unless otherwise stated herein or clearly contradictory to the context, when the term "at least one" is followed by one or more of items as listed (for example, "at least one of A and B"), it is to be understood as one of the listed items (A or B) or any combination of two or more of the listed items (A and B).

Unless otherwise noted, the terms "comprise," "have," "include," and "contain," are intended to mean an open term (i.e., meaning "including, but not limited to"). However, in the present application, the expression "comprising" should be understood as also describing closed expressions such as "consisting of", "having" or "being".

In the present application, the term "EGFR" generally refers to Epidermal Growth Factor Receptor, also known as ErbB1 or HER1, that is a 170 kDa transmembrane glycoprotein encoded by c-erbB1 proto-oncogene. EGFR is a member of the human epidermal growth factor receptor (HER) family of receptor tyrosine kinase (RTK), and the family further comprises HER2 (ErbB2), HER3 (ErbB3) and HER4 (ErbB4). EGFR signaling is initiated by ligand binding, and then signal transduction cascade is initiated by inducing conformational changes in the receptor with other ErbB family members, homodimerization or heterodimerization, and trans-autophosphorylation of receptor (see, Ferguson et al., Annu Rev Biophys, 37: 353 to 73, 2008), thereby finally affecting a variety of cell functions (e.g., cell proliferation and survival). The expression of EGFR or the increased kinase activity thereof is associated with a series of human cancers (see, Mendelsohn et al., Oncogene 19: 6550-6565, 2000; GrUnwald et al., J Natl Cancer Inst 95: 851-67, 2003; Mendelsohn et al., Semin Oncol 33: 369-85, 2006). It has been reported that the increased expression of EGFR is found in numerous cancers, such as, glioma, breast cancer, ovarian cancer, cervical cancer, and the like.

In the present application, the term "EGFR inhibition" comprises the decreased EGFR activity, expression or quantity caused by any reasons (e.g., caused by treatment or the physical conditions of the subject itself). In some embodiments, EGFR inhibition is meant that the EGFR activity or quantity is decreased by at least 10%. In some embodiments, EGFR inhibition is generally meant that the EGFR activity or quantity is decreased by at least 20%, 40%, 50%, 80%, 90%, 95% or more. In some embodiments, the decrease is based on the comparison with the standard value of the same type of subjects (e.g., the same type of healthy persons or the same type of patients). In some embodiments, the decrease is based on the comparison with the value of the same subject earlier.

In the present application, the term "EGFR inhibitor" generally refers to any EGFR inhibitor that has been known in the art or will be found in the future, including any substance that causes an inhibition of a biological activity associated with the EGFR activity in a subject (including any inhibition of the downstream biological effect caused by the binding of EGFR with its natural ligand(s)) when the substance is administered to the subject. In some embodiments, the EGFR inhibitor comprises any reagent capable of blocking the EGFR activity or any downstream biological effect thereof during its use in the treatment of a cancer.

In the present application, the term "small molecular EGFR inhibitor" may comprise a small molecular EGFR inhibitor that binds reversibly to EGFR (e.g., Gefitinib, Erlotinib, Sapitinib and Icotinib), a small molecular EGFR inhibitor that binds irreversibly to EGFR (e.g., Afatinib, Dacomitinib, Lapatinib (e.g., Tykerb^{®}, GW572016 GlaxoSmithKline), Vandetanib (e.g., ZACTIMATM, ZD6474), Lenvatinib, Canertinib, Varlitinib and Neratinib) and/or a small molecular EGFR inhibitor that binds specifically to mutant EGFR (e.g., Osimertinib, Nazartinib, Rociletinib, Olmutinib, Avitinib and EAI045).

The term "pharmaceutically acceptable" as used herein generally refers to those compounds, materials, compositions and/or dosage forms that are suitable for use in contact with human and animal tissues, without excessive toxicity, irritation, allergic reactions, or other problems or complications within the scope of reasonable medical judgment, with a reasonable benefit/risk ratio. In some embodiments, pharmaceutically acceptable compounds, materials, compositions, and/or dosage forms are those approved by a regulatory agency (such as the U.S. Food and Drug Administration, China Food and Drug Administration, or the European Medicines Agency) or listed in a generally recognized pharmacopoeia (such as the United States Pharmacopoeia, the China Pharmacopoeia, or the European Pharmacopoeia) for use in animals (and more particularly in humans).

### DETAILED DESCRIPTION

In one aspect, the application provides a use of a NO-NSAID or a pharmaceutically acceptable salt thereof in preparing a medicament, the medicament is used for preventing, ameliorating and/or treating a disease or disorder in a subject associated with the administration of an anti-tumor agent.

In another aspect, the present application provides a method of preventing, ameliorating and/or treating a disease or disorder associated with administration of an anti-tumor agent in a subject, the method comprising administering to a subject in need thereof an effective amount of NO-NSAID compound or pharmaceutically acceptable salt thereof.

In another aspect, the present application provides a NO-NSAID compound, or pharmaceutically acceptable salt thereof, for use in preventing, ameliorating and/or treating a disease or disorder associated with administration of an anti-tumor agent in a subject.

In another aspect, the present application provides a pharmaceutical combination comprising: 1) an anti-tumor agent; and 2) a NO-NSAID compound.

In another aspect, the present application provides a use of a NO-releasing agent and an NSAID in the preparation a medicament, the medicament is used for preventing, ameliorating and/or treating a disease or disorder associated with the administration of an anti-tumor agent in a subject.

In another aspect, the present application provides a method of preventing, ameliorating and/or treating a disease or disorder associated with administration of an anti-tumor agent in a subject, the method comprising administering to a subject in need thereof an effective amount of a NO-releasing agent and an NSAID.

In another aspect, the present application provides a pharmaceutical combination comprising: 1) an anti-tumor agent; 2) a NO releasing agent; and 3) an NSAID.

### NO-NSAID

In any aspect of the present application, the NO-NSAID compound may comprises a nitric oxide (NO) releasing moiety and a nonsteroidal anti-inflammatory agent (NSAID) moiety, and the NO releasing moiety and the nonsteroidal anti-inflammatory agent moiety are covalently linked directly or via a spacer.

For example, in the NO-NSAID compound, the NO releasing moiety is linked to the NSAID moiety through a cleavable linker. In some embodiments, in the NO-NSAID compound, the NO-releasing moiety is linked to the NSAID moiety through a linker comprising an ester bond and/or a disulfide bond.

For example, in the NO-NSAID compound, a spacer may be included between the NO releasing moiety and the NSAID moiety. In some embodiments, the spacer comprises: one or more optionally substituted -CH2-, optionally substituted phenyl, and/or a combination thereof. For example, the spacer may comprise/can be: one or more -CH2- , one or more substituted -CH2- , one or more phenyl, one or more substituted phenyl, or any combination thereof.

The non-steroidal anti-inflammatory agent (NSAID) moiety may comprise a cyclooxygenase (COX) inhibitory moiety. The (COX) inhibitory moiety may comprise a small molecule COX inhibitory moiety that reversibly binds to COX and/or a small molecule COX inhibitory moiety that irreversibly binds to COX. The small molecule COX inhibitory moiety may has a molecular weight that is less than or equal to 2000 Daltons, less than or equal to 1500 Daltons, less than or equal to 1200 Daltons, less than or equal to 1000 Daltons, less than or equal to 900 Daltons, less than Or equal to 800 Daltons, less than or equal to 700 Daltons, less than or equal to 600 Daltons, less than or equal to 500 Daltons, less than or equal to 400 Daltons, less than or equal to 300 Daltons, less than or equal to 200 Daltons and/or less than or equal to 100 Daltons.

In some embodiments, the COX inhibitory moiety comprises a moiety capable of reducing the expression of the COX, and/or reducing the activity of the COX. For example, the COX inhibitory moiety can act directly on the COX protein and/ or the nucleic acid encoding the COX protein. For example, the COX inhibitory moiety can inhibit cyclooxygenase-1 (COX-1) and/or cyclooxygenase-2 (COX-2). In some embodiments, the COX inhibitory moiety selectively inhibits COX-2. In some embodiments, the COX inhibitory moiety can non-selectively inhibit COX-1 and COX-2.

For example, the COX inhibitory moiety may comprise one or more molecules, prodrugs thereof, active derivatives thereof, and/or active metabolites thereof, selected from the group consisting of: acetaminophen, acemetacin, aceclofenac, alminoprofen, arnfenac, bendazac, benoxaprofen, bromfenac, bucloxic acid, butibufen, carprofen, cinmetacin, clopirac, diclofenac, etodolac, felbinac, fenclozic acid, fenbufen, fenoprofen, fentiazac, flunoxaprofen, flurbiprofen, ibufenac, ibuprofen, indomethacin, isofezolac, isoxepac, indoprofen, ketoprofen, lonazolac, loxoprofen, metiazinic acid, mofezolac, miroprofen, naproxen, oxaprozin, pirozolac, pranoprofen, protizinic acid, salicylamide, sulindac, suprofen, suxibuzone, tiaprofenic acid, tolmetin, xenbucin, ximoprofen, zaltoprofen, zomepirac, aspirin, acemetcin, bumadizon, carprofenac, clidanac, diflunisal, enfenamic acid, fendosal, flufenamic acid , flunixin, gentisic acid, ketorolac, meclofenamic acid, mefenamic acid and mesalamine.

In some embodiments, the COX inhibitory moiety comprises one or more molecules, prodrugs thereof, active derivatives thereof, and/or active metabolites thereof, selected from the group consisting of naproxen, aspirin, diclofenac, ketoprofen, flurbiprofen and ibuprofen.

According to any aspect of the application, the NO-releasing moiety is capable of generating at least one of NO⁺, NO⁻, N₂O, NO, N₂O₃, NO₂, NO₃⁻, and NO₂⁻. For example, the NO-releasing moiety may generates NO directly or indirectly. For example, the NO-releasing moiety may comprises organic molecules, inorganic molecules, and/or polymers or nanomaterials.

In some embodiments, the NO-releasing moiety may comprises an component selected from the group consisting of: nitroglycerin, isosorbide mononitrate, butanediol mononitrate, pentaerythritol tetranitrate, isosorbide dinitrate, triethanolamine, nicorandil, propatyl nitrate, 5-amino-3-(4-morpholinyl)-1,2,3-oxadiazole, isopentyl nitrite, 3,3-bis(aminoethyl)-1-hydroxy-2-carbonyl-1-triazene (NOC-18), Sulfo NONOate disodium salt, S- nitrosoglutathione, S-nitroso-N-acetylpenicillamine, 4-phenyl-3-furoxancarbonitrile, ((±)-(E)-4-Ethyl-2-[(E)-hydroxyimino]-5-nitro-3-hexenamide), Streptozocin, NG-Hydroxy-L-arginine acetate salt, O₂ -(2,4-Dinitrophenyl) 1-[(4- ethoxycarbonyl)piperazin-1-yl]diazen-1-ium-1,2-diolate), N-Nitrosodibutylamine, 3-morpholinosydnonimine (SIN-1) , Linsidomine, Molsidomine, 3-(4-acetylphenyl)sydnone, Diethylamine NONOate /AM) and Itramin.

For example, the NO releasing moiety may omprises a component selected from the group consisting of nitroxyl complex, metal nitrosyl complex, metal nitrosamino complex, nitrates and nitrites.

In some embodiments, the NO-releasing moiety comprises a component selected from the group consisting of S-nitrosothiol nanosilica spheres, S-nitrosethanedithiol chitosan and oligopropylenediamine-grafted NONOate.

In some embodiments, the NO-releasing moiety has a molecular weight that is less than or equal to 2000 Daltons, less than or equal to 1500 Daltons, less than or equal to 1200 Daltons, less than or equal to 1000 Daltons, or less than or equal to 900 Daltons, less than or equal to 800 Dalton, less than or equal to 700 Dalton, less than or equal to 600 Dalton, less than or equal to 500 Dalton, less than or equal to 400 Dalton, less than or equal to 300 Dalton, less than or equal to 200 Daltons and/or less than or equal to 100 Daltons.

For example, the NO-releasing moiety may has one or more of the following groups: diazeniumdiolates, hydroxydiazenesulfonic acid, S-nitrosothiol, furoxans, oxime, N-nitrosamine, N-hydroxyguanidine, nitrate, nitrite, nitrate ester, nitrite ester, sydnonimines, sydnones, oxtriazole-5-imine, oxtriazol-5-one, hydroxylamine, dioxadiazetidine, N-hydroxynitrosamine, N- nitrosoimine, hydroxyurea and metal nitrosamino complexes.

In some embodiments, the NO-releasing moiety may comprise -NO₂ or -ONO₂.

In some embodiments, the NO-NSAID compound further comprises a H₂S releasing moiety. For example, it may be the NO-NSAID compound described in WO2013025790A2.

In some embodiments, the NO-NSAID compound comprises a structure represented by Formula (1): 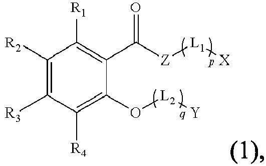 wherein:
Z is O or NH, R₁, R₂, R₃ and R₄ are each independently H, halogen, NO₂, N₃, C₁-C₁₀ alkyl, OR, OC(O)R, N(R)₂, NH-C(O)R, S(O)R or N=N-R, wherein each R is independently a C₁-C₁₀ alkyl or aryl group; L₁ comprises a structure selected from the group consisting of: -C(O)-, -(CH₂)ₘ-, -(CH₂)ₘ-O-, -(CH₂)ₘ-C(O)-, -(CH₂)ₘ-C(O)O-, -(CH₂)ₘ-OC(O)O-, -C(O)-(CH₂)ₘ-O-, -C(O)-(CH₂)ₘ-C(O)-, -OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7; L₂ comprises a structure selected from the group consisting of: -C(O)-, -(CH₂)ₘ-, -(CH₂)ₘ-O-, -(CH₂)ₘ-C(O)-, -(CH₂)ₘ-C(O)O-, -(CH₂)ₘ-OC(O)O-, -C(O)-(CH₂)ₘ-O-, -C(O)-(CH₂)ₘ-C(O)-, -OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7; P and q are each independently 0 or 1; X is the H₂S releasing moiety or the NO releasing moiety; Y is the NO releasing moiety or the H₂S releasing moiety, and Y and X are not simultaneously the NO releasing moiety nor simultaneously the H₂S releasing moiety; The NO releasing moiety is -C(O)-(CH₂)ₙ-ONO₂ or - (CH₂)ₙ-ONO₂, wherein n is 1, 2, 3, 4, 5, 6 or 7; and the H₂S releasing moiety is: 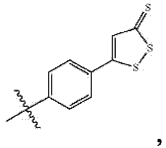 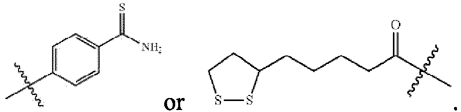 For example, the NO-NSAID compound may be the NO-NSAID compound described in US9688607B2.

For example, the NO-NSAID compound may comprises a structure represented by Formula (2): 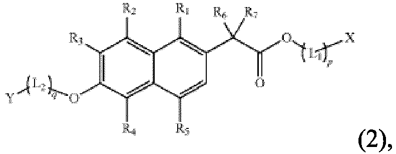 wherein:
p and q are each independently 0 or 1; L₁ and L₂ are each independently comprise a structure selected from the group consisting of: -C(O)-, -(CH₂)ₘ-, -(CH₂)ₘ-O-, -(CH₂)ₘ -C(O)-, -(CH₂)ₘ-C(O)O-, -(CH₂)ₘ -OC(O)O-, -C(O)-(CH₂)ₘ-O-, -C(O)-(CH₂)ₘ-C(O)-, -OC(O)-(CH₂)ₘ-O-, -OC(O)-(CH₂)ₘ-C(O)- and -OC(O)-(CH₂)ₘ-C(O)O-, wherein m is 1, 2, 3, 4, 5, 6 or 7; X is the H₂S releasing moiety or the NO releasing moiety; Y is the NO releasing moiety or the H₂S releasing moiety, and the Y and the X are not simultaneously the NO releasing moiety nor simultaneously not the H₂S releasing moiety; The NO releasing moiety is selected from: -NO, -C(O)-(CH₂)ₙ-ONO₂, -O-(CH₂)ₙ-ONO2, -(CH₂)ₙ-ONO₂, -C(O)-CH₂-C(CH₃)₂-SNO, -NH-CH₂-C(CH₃)₂-SNO, -CH₂-C(CH₃)₂-SNO, 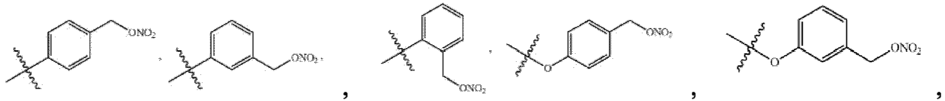 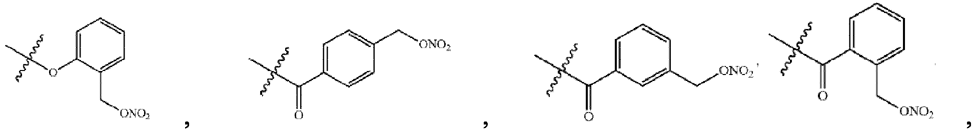 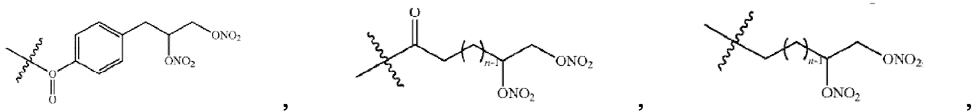 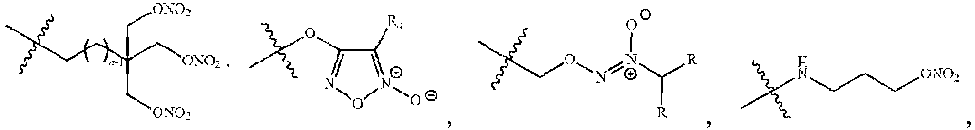 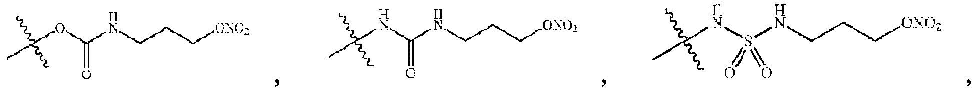 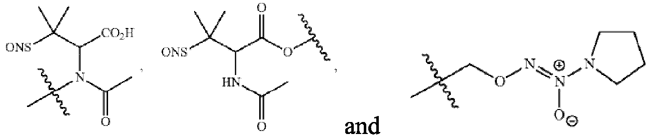 wherein n is 1, 2, 3, 4, 5, 6 or 7, Rₐ is H, C₁-C₁₀ alkyl, aryl, S(O)₂-aryl, CN or CON(R_{b})₂, and each R_{b} is independently H or C₁-C₁₀ alkyl; and the H₂S releasing moiety is selected from: 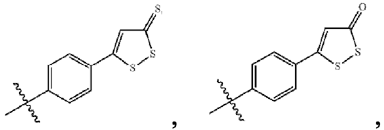 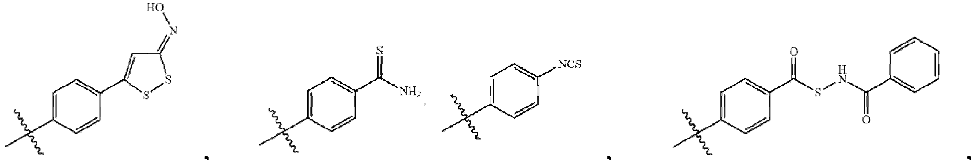 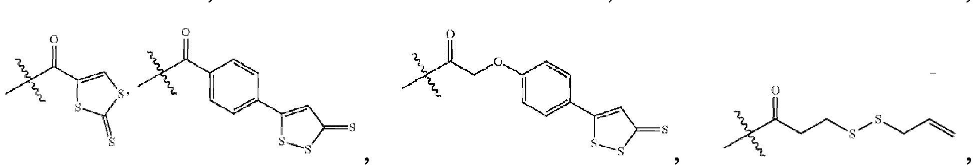 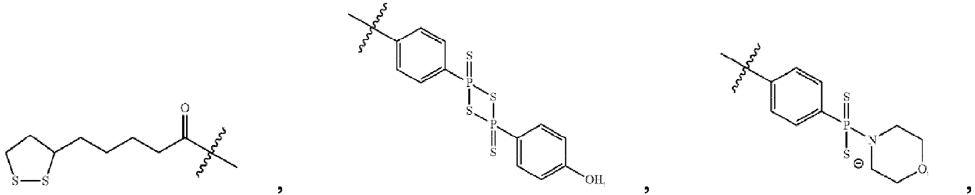 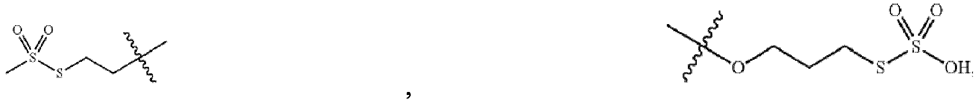 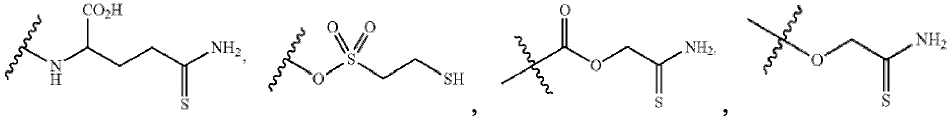 and 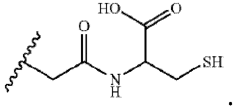 For example, the NO-NSAID compound may be a compound described in US10450260B2.

In some embodiments, the NO-NSAID compound comprises a structure selected from the group consisting of 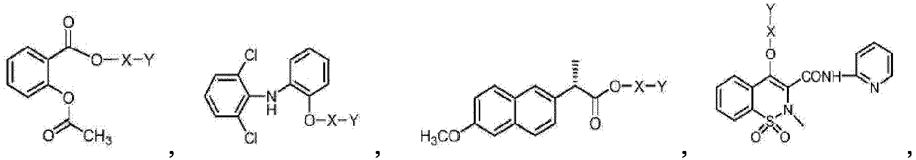 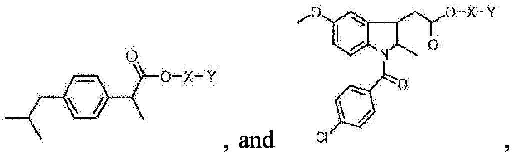 Wherein, X is -(CH₂)ₙ₁-Z-(CH₂)ₙ₂-, n1 and n2 are each independently 0, 1, 2, 3, 4 or 5; Z is O, N, S, C, 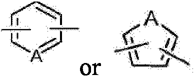 , A is O, N, S, or C; and Yis

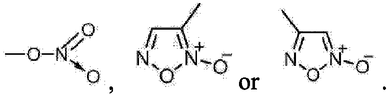

In some embodiments, the NO-NSAID compound comprises a structure represented by Formula (3):

M-X-Y-ONO₂ (3),

wherein M is a structure selected from the following group: 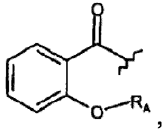 wherein R_{A} is a hydrogen atom or -C(O)CH₃; 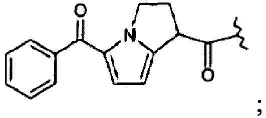 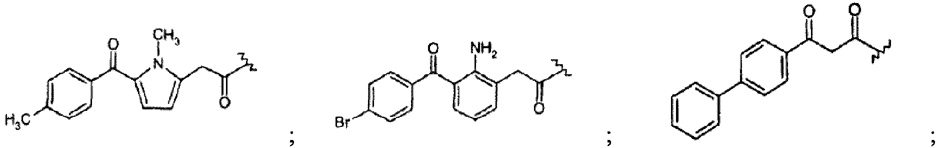 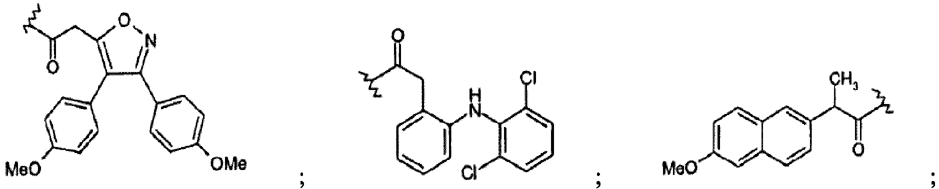 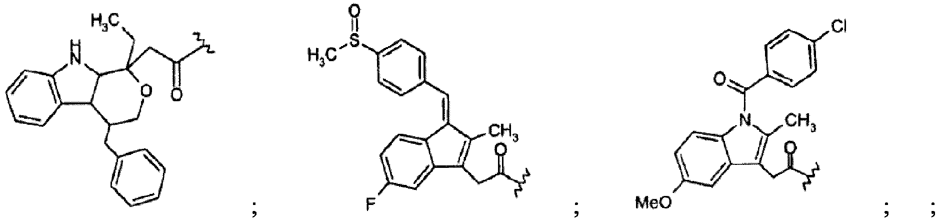 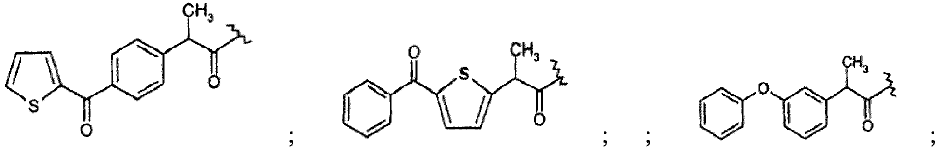 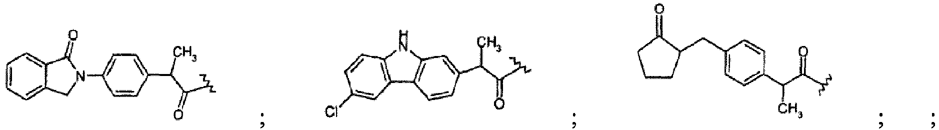 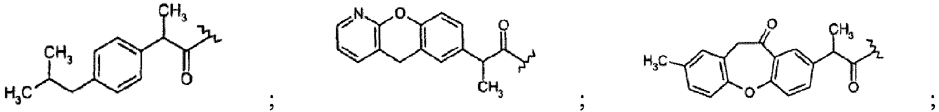 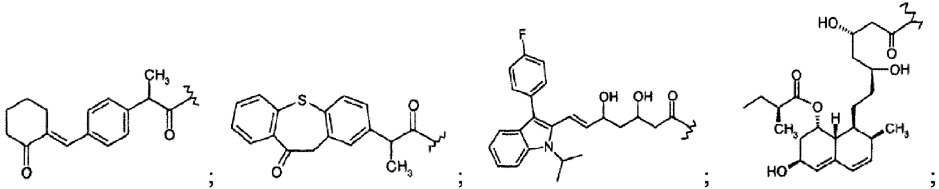 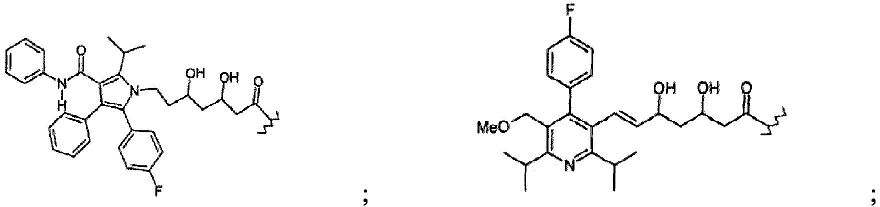 and 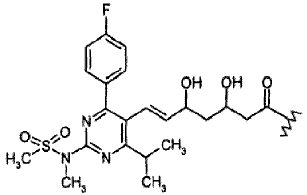 X is -O-, -S- or -NR¹-, wherein R¹ is H or a linear or branched C₁-C₆ alkyl group;
Y is a divalent group with the following definition:
   a)
      -Linear or branched C₁-C₂₀ alkylene, optionally substituted by one or more substituents selected from the group consisting of: halogen, hydroxyl, -ONO₂, -OC(O)(C₁-C₁₀ alkyl)-ONO₂ and -O(C₁-C₁₀ alkyl)-ONO₂;
      -C₅-C₇ cycloalkylene, which is optionally substituted by a linear or branched C₁-C₁₀ alkyl group;
   b) 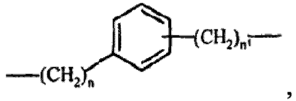 wherein n is an integer selected from 0-20, and n' is an integer from 1-20;
   c) 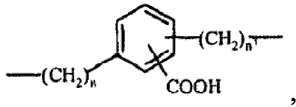 wherein n is an integer selected from 0-20, and n' is an integer from 1-20;
   d) 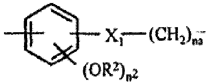 wherein X₁ is -OCO- or -COO-, and R² is H or CH₃, na is an integer from 1-20, and n² is an integer from 0-2;
   e) 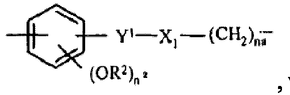 wherein Y¹ is -CH₂-CH₂-(CH₂)n²-, or -CH=CH-(CH₂)n²-, X₁, na, n² and R² are as defined above;
   f) 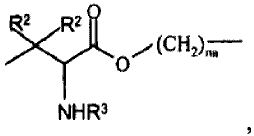 wherein na and R² are as defined above, and R³ is H or -COCH₃; or
   g) 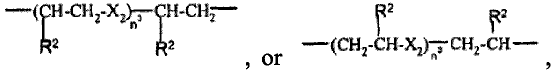 wherein X₂ is -O- or -S-, n³ is an integer from 1 to 6, and R² is defined as above.

In some embodiments, the NO-NSAID compound comprises a structure represented by Formula (3): M-X-Y-ONO₂ (3), wherein M is selected from the following structures: 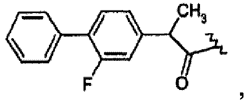 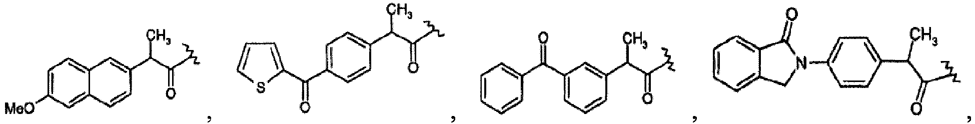 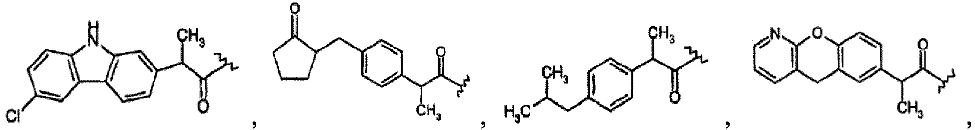 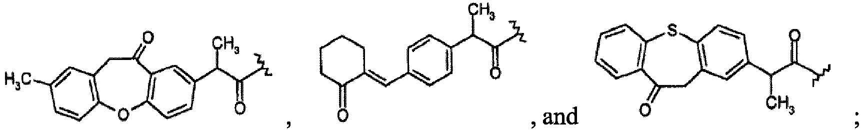 X is -O-;Yis a linear or branched C₁-C₁₀ alkylene group.

For example, the NO-NSAID compound may comprise a structure represented by formula (4):

A-(B)*_{b0}*-(D)*_{c0}*-NO₂ (4)

wherein: c0 is 0 or 1, b0 is 0 or 1, and at least one of c0 and b0 is not 1; A is R-T₁-, wherein R-T₁- is a free radical of the formula R-T₁-OH or a prodrug of acetylsalicylic acid, wherein T₁ = (CO), and R is the remaining radical of acetylsalicylic acid; B is T_{B}-X₂-T_{BI}-, TB ᵢₛ X, T_{BI} is (CO) or X, X is O, S, N or NR_{1C}, Ric is H or a linear or branched alkyl group having 1 to 5 carbon atoms; X₂ is a group of a compound of formula H-X₂-H selected from the group consisting of hydroxy acid, gallic acid, ferulic acid, gentisic acid, citric acid, caffeic acid, p-coumaric acid and vanillic acid; D is a divalent group: -T_{c}-Y-, wherein when b0 is 1, T_{c} is (CO) when T_{BI} is X , or T_{c} is X when T_{BI} is (CO), and X is O, S, N or NR_{1C}, Ric is H or a linear or branched alkyl group with 1 to 5 carbon atoms; when b0 is 0, T_{c} =X, and X is O, S, N or NR_{1C}, Ric is H or a linear or branched alkyl group with 1 to 5 carbon atoms; Y is Y*ₚ*, Y*_{O}* or Y*_{Ar}* , Y*ₚ* is 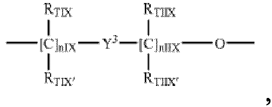 wherein nIX is an integer from 0 to 3, nIIX is an integer from 1 to 3, R*_{TIX}*, R_{*TIX*'}, R*_{TIIX}* and R*_{TIIX'}* are each independently H or linear or branched C₁-C₄ alkyl, Y ³ is a saturated, unsaturated or aromatic heterocyclic ring containing one or two nitrogen atoms and has 6 atoms; Y*_{O}* is selected from the group consisting of C₁-C₂₀ linear or branched alkyleneoxy, 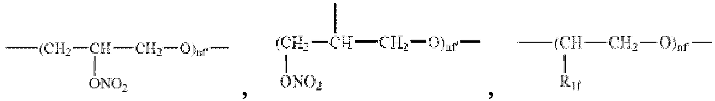 and 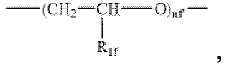 wherein nf is an integer from 1 to 6, and R_{1f} is H or CH ₃ , Y*_{Ar}* is 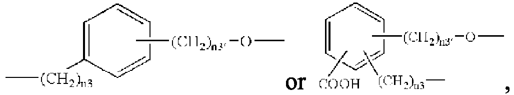 wherein n3 is an integer from 0 to 3, and n3' is an integer from 1 to 3; and When b0 is 1, c0 is 1 and Y is Y*_{O}*, or when b0 is 0, c0 is 1 and Y is Y*ₚ* or Y*_{Ar}*. For example, the NO-NSAID compound may comprise the NO-NSAID compound described in US7378437B2.

For example, the NO-NSAID compound may comprise a structure represented by Formula (5): A-X₁-L-(W)*ₚ*-NO₂(5). wherein: p is 0 or 1; A is R-T₁, wherein R is the residue of the prodrug and has the following formula: 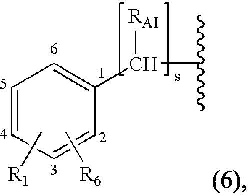 wherein s is 0 or 1; R_{AI} is H or CH₃; R₁ is OCOR₃, NHCOR₃, OH, CH₂CH(CH₃)₂, phenyl, benzoyl or 4,6-dichlorophenylamino, R₃ is a C₁-C₅ linear or branched residue; R₆ is H or halogen;

Alternatively, when R₁ and R₆ are located at the adjacent 4- and 5-positions on the aromatic ring of formula (6), the structure of formula (7) is formed: 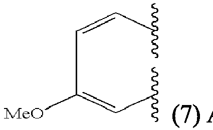 Alternatively, R has the structure of the following formula: 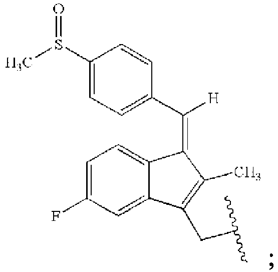 T₁ is (CO)t or (X)_{t'}, wherein X is O, S or NR_{1c}, R_{1c} is H or a linear or branched alkyl group having 1 to 5 carbon atoms, t and t' are 0 or 1, t is 1 when t' is 0, and t is 0 when t' is 1; X₁ is -T_{B}-Y-T_{BI}, wherein Ta and T_{BI} can be the same or different. When t is 0, T_{B} is (CO), when t' is 0, T_{B} is X, and X is as defined above; T_{BI} is (CO)ₜₓ or (X)ₜₓₓ, wherein tx and txx are 0 or 1, when txx is 0, tx is 1, when txx is 1, tx is 0, and X is as defined above; Y is a divalent linking group selected from the following: 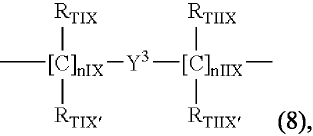 wherein: nIX is an integer from 0 to 3, nIIX is an integer from 1 to 3, R_{TIX}, R_{TIX'}, R_{TIIX} and R_{TIIX'} are each independently selected from H and C₁-C₄ linear or branched alkyl groups; Y³ is a saturated, unsaturated or aromatic heterocyclic ring with 5 or 6 atoms and containing 1 or 2 nitrogen atoms; C₁-C₂₀ linear or branched alkylene, which is optionally substituted with a substituent selected from the group consisting of: NHCOR₃, -NH₂ and -OH, R₃ is a C₁-C₅ linear or branched residue; a cycloalkylene group having 5 to 7 carbon atoms, optionally substituted with a C₁-C₂₀ linear or branched alkylene side chain, which is optionally substituted with a substituent selected from the group consisting of: NHCOR₃, -NH₂ and -OH, R₃ is a C₁-C₅ linear or branched residue, wherein one or more carbons atoms in the cycloalkylene ring are optionally replaced by a heteroatom; 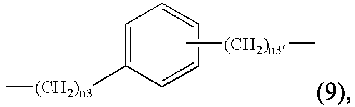 wherein n3 is an integer from 0 to 3, and n3' is an integer from 1 to 3; 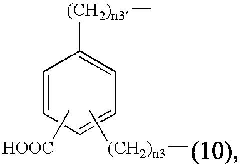 wherein n3 is an integer from 0 to 3, and n3' is an integer from 1 to 3; 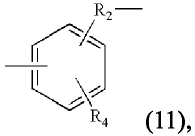 wherein R ₄ is hydroxyl, hydrogen or RsO, R₅ is C₁-C₁₀ linear, branched or cyclic alkyl group, and R₂ is C₂-C₁₀ linear or branched alkenylene containing one or more double bonds; 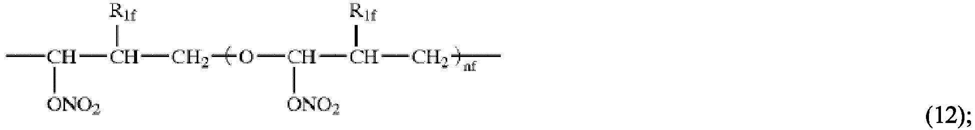 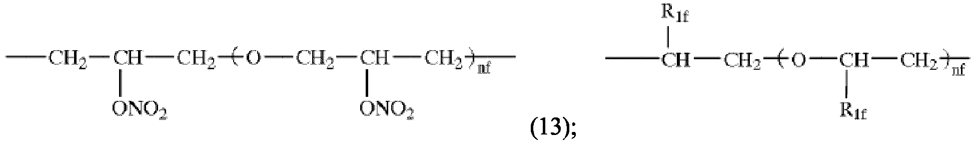 (14); and 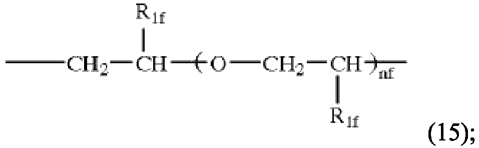 wherein R_{1f} is H or CH₃, and nf is an integer from 0 to 6; L is a covalent bond, CO or X, as defined above; and W is Y_{T}O, Y_{T} has the same meaning as Y, and in the compound of formula (5), Y and Y_{T} can be the same or different. For example, the NO-NSAID compound may comprise the NO-NSAID compound described in US20040023933A1.

For example, the NO-NSAID compound may comprise a structure represented by formula (5): A-X₁-L-(W)*ₚ*-NO₂(5), wherein: the p is 0 or 1, A is R-T₁-, wherein R has the structure of 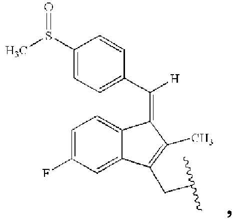 and T₁ is (CO)t or (X)_{t'}, t and t' are 0 or 1, t' is 0 when t is 1, and t' is 1 when t is 0; X₁ is -T_{B}-Y-T_{B1}-, wherein T_{B} and T_{B1} are the same or different, when t is 0, T_{B} is (CO), and when t' is 0, T_{B} is X; T_{B1} is (CO)ₜₓ or (X)ₜₓₓ, wherein tx and txx are each independently 0 or 1, tx is 1 when txx is 0, and tx is 0 when txx is 1; X is O, S or NR_{1C}, R_{1C} is H or a linear or branched alkyl group having 1 to 5 carbon atoms; Y is selected from 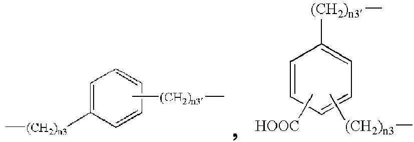 and 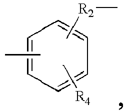 wherein n3 is an integer from 0 to 3, n3' is an integer from 0 to 3, R₄ is hydroxyl, hydrogen or R₅O-alkoxy, wherein R₅ is a C₁-C₁₀ linear, branched or cyclic alkyl, R₂ is C₂-C₁₀ linear or linear alkenylene and optionally contains one or more double bonds; L is a covalent bond, CO, or X, X is O, S, or NR_{1C}, and Ric is H or a linear or branched alkyl group with 1-5 carbon atoms; and W is Y_{T}O, Y_{T} is selected from 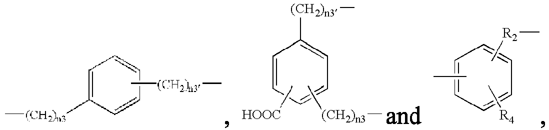 wherein n3 is an integer from 0 to 3, n3' is an integer from 0 to 3, R₄ is hydroxyl, hydrogen or RsO-alkoxy, wherein R₅ is C₁-C₁₀ linear, branched or cyclic alkyl, R₂ is C₂-C₁₀ linear or linear alkenylene and optionally contains one or more double bonds. For example, the NO-NSAID compound may comprise the NO-NSAID compound described in US7465803B2.

For example, the NO-NSAID compound may comprise a structure represented by Formula (16): 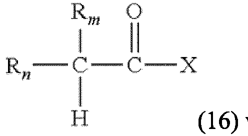 wherein Rₘ is H or lower alkyl, Rₙ is a structure selected from the following group: 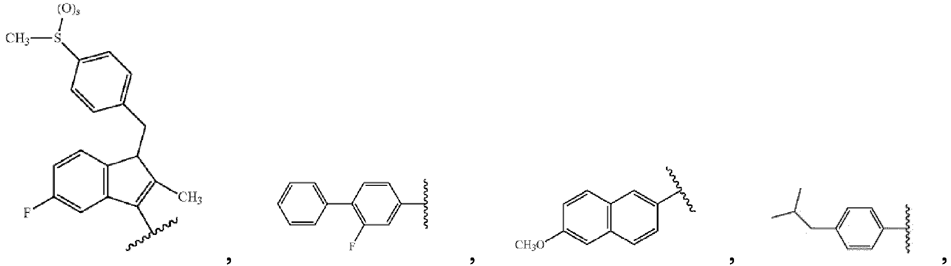 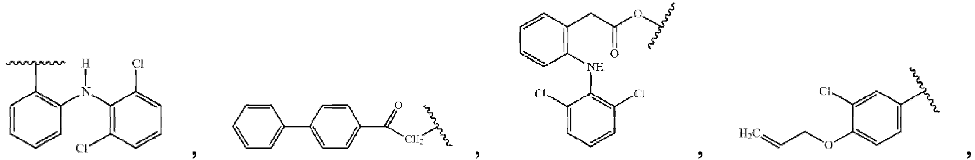 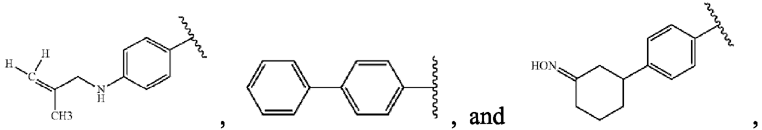 s is 0 or 1, X is -Y-(CR₄R_{4'})*ₒ*-C(R₄)(ONO₂)-(CR₄R_{4'})*_{q}*-(T)*ₒ*-(W)*_{q}*(T)*ₒ*-(CR₄R_{4'})*ₒ*-R₅;, each R₄ and R₄' is independently hydrogen, lower alkyl, -OH, -CH₂OH, -ONO₂, -NO₂ or -CH₂ONO₂, or R₄ and R₄' together with the carbon atom to which they are connected to form a cycloalkyl or heterocycle; W is a covalent bond or carbonyl group; each T is independently oxygen, (S(O)ₒ)ₒ or NRⱼ; Rⱼ is hydrogen, alkyl, aryl, heterocycle, alkylcarbonyl, alkylaryl, alkylsulfinyl, alkylsulfonyl, arylsulfinyl, arylsulfonyl, sulfonamido, N-alkylsulfonamido, N, N-diarylsulfonamido, N-arylsulfonamido, N-alkyl-N-arylsulfonamido, formamido and/or hydroxyl; each q is independently 1, 2 or 3; each o is independently 0, 1 or 2; Y is oxygen or sulfur; and R₅ is hydrogen, hydroxyl, alkyl, aryl, alkylsulfonyl, arylsulfonyl, carboxylic ester, alkylcarbonyl, arylcarbonyl, formamido, alkoxyalkyl, alkoxyaryl, cycloalkyl and/or heterocycle. For example, the NO-NSAID compound may comprise the NO-NSAID compound described in US8222277B2.

For example, the NO-NSAID compound may comprise a structure represented by Formula (17) or Formula (18):

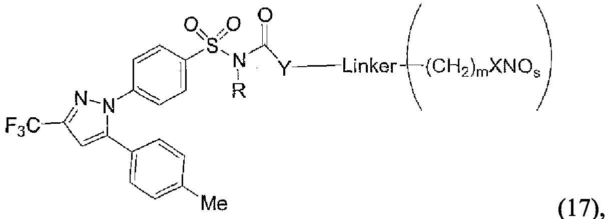

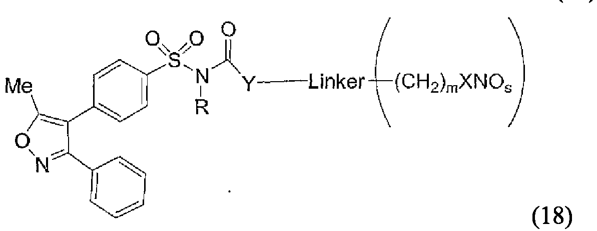

Each s is independently 1 or 2; k is 1, 2, 3 or 4; each m is independently 0, 1, 2, 3 or 4; each X is independently O or S; Y is a bond, S, O or NRi, wherein R₁ is hydrogen or C₁-C₆ alkyl; R is hydrogen or C₁-C₆ alkyl; the Linker is a linker, which is selected from the following group: 1)-(CH₂)ₙ, n is 0, 1, 2, 3 or 4; 2) C₃-C₆ cycloalkyl, optionally substituted with a group selected from the group consisting of: halogen, C₁-C₃ alkyl, C₁-C₃ alkoxy, hydroxyl, NO, CO₂, CF₃, CN, CH₂COOH, CH₂COO-C₁₋₃ alkyl and C₁-C₃ thioalkyl; 3) Aryl group, the aryl group is selected from phenyl and naphthyl, the aryl group is optionally substituted with a group selected from the following group: halogen, C₁-C₃ alkyl, C₁-C₃ alkoxy, hydroxyl, NO₂, CO₂, CF₃, CN, CH₂COOH, CH₂COO-C₁₋₃ alkyl and C₁-C₃ thioalkyl; and 4) Heteroaryl, which is optionally substituted by a group selected from the group consisting of: halogen, C₁-C₃ alkyl, C₁-C₃ alkoxy, hydroxyl, NO₂, CO₂, CF₃, CN, CH₂COOH, CH₂COO-C₁₋₃ alkyl and C₁-C₃ thioalkyl. For example, the NO-NSAID compound may comprise the NO-NSAID compound described in WO2004037798A1.

In some embodiments, the NO-NSAID compound comprises a structure represented by Formula (19):
A-T-Y-ONO₂ (19), wherein: A is a medicament selected from the following group: a residue of (A-OH or AH), a non-steroidal anti-inflammatory drug, an analgesic, an antipyretic and a COX-2 inhibitor; T is -O-, -NH-, -S-, -CO- or -(CH₂)ₙ₁OCO-, wherein n1 is an integer from 1 to 20; A is selected from the group consisting of: 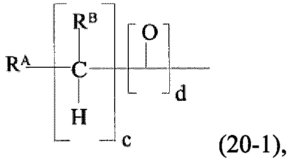 wherein c and d are each independently 0 or 1, R^{B} is selected from: H, linear or branched C₁-C₁₂ alkyl and C₂-C₁₂ alkenyl; when c is 0 and d is 1, R^{A} is a structure selected from the following group: 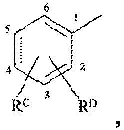 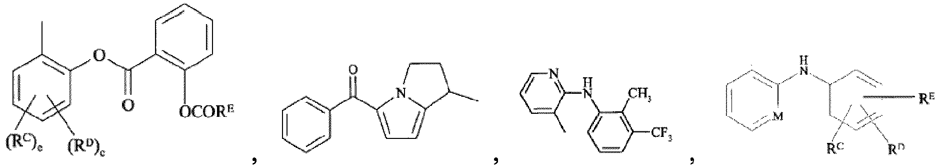 and 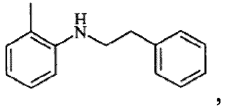 R^{c} is selected from the group consisting of H, halogen, amino, R^{E}CONH- and -OCOR^{E}, R^{D} is selected from the group consisting of H, OH, halogen, linear or branched C₁-C₄ alkyl, linear or branched C₁-C₄ alkoxy, trifluoromethyl, amino and mono- or di-(C₁-C₄) alkylamino, R^{E} is selected from H and linear or branched C₁-C₅ alkyl, e is 0 or 1, M is C or N; when c is 1, d is 1, and R^{B} is hydrogen, R^{A} is a structure selected from the following group: 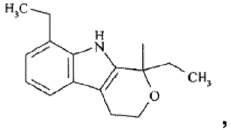 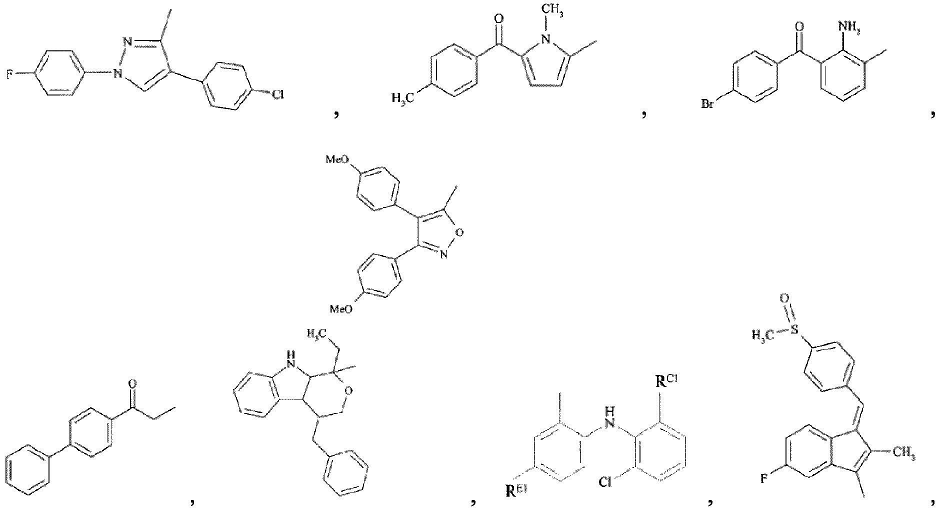 and 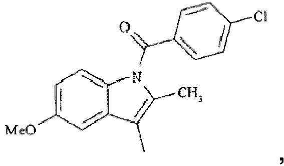 wherein R^{E1} is H or CH₃, and R^{C1} is Cl or F; when c is 1, d is 1, and R^{B} is CH₃, R^{A} is a structure selected from the following group: 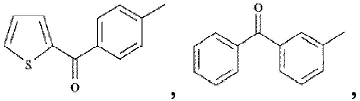 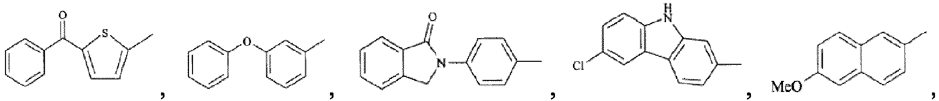 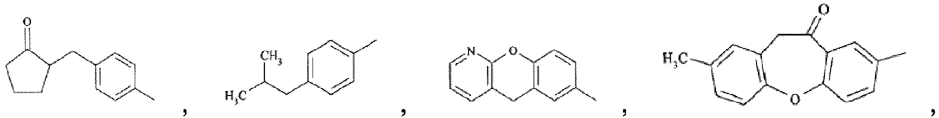 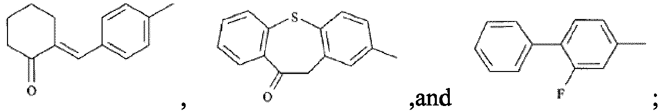 when c is 0 and d is 0, R^{A} is a structure selected from the following group: 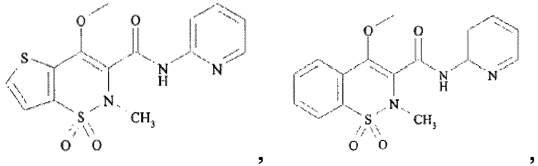 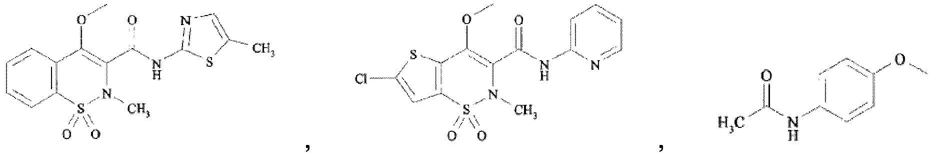 and 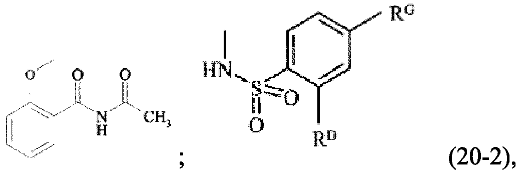 wherein: R^{D} as descried above,
R^{G} is a structure selected from the group consisting of: 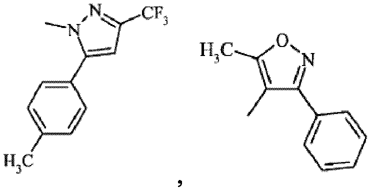 and 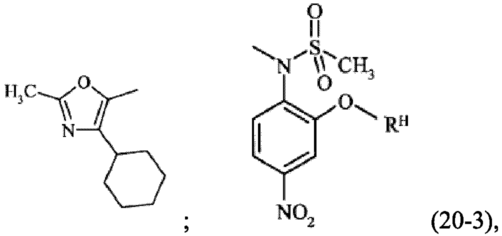 wherein R^{H} is phenyl or cyclohexyl; 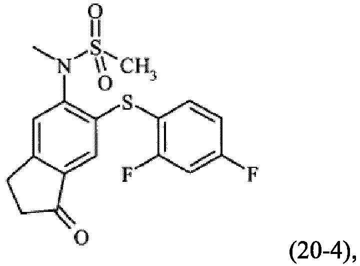
Y is a divalent group with the following meaning:
   a)
      - Linear or branched C₁-C₂₀ alkylene;
      - Cycloalkylene, the cycloalkylene has 5 to 7 carbon atoms in the ring, which is optionally substituted by the side chain R¹, wherein R¹ is a linear or branched alkyl having 1 to 10 carbon atoms;
   b) 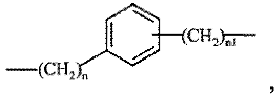 wherein n is an integer from 0 to 20, and n1 is an integer from 1 to 20;
   c) 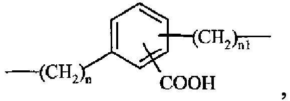 wherein n is an integer from 0 to 20, and n1 is an integer from 1 to 20;
   d) 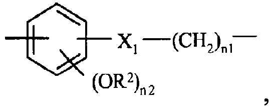 n1 is an integer from 1 to 20, and n2 is an integer from 0 to 2, X₁ is -OCO- or -COO-, and R² is H or CH₃;
   e) 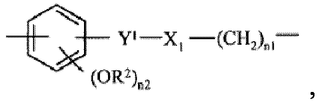 wherein n1, n2, R² and X₁ are as described above, Y¹ is - CH₂-CH₂- or -CH=CH-(CH₂)ₙ₂-;
   f) 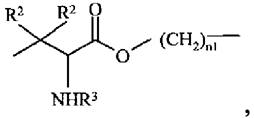 wherein n1 and R² are as described above, and R³ is H or COCH₃;
   g) 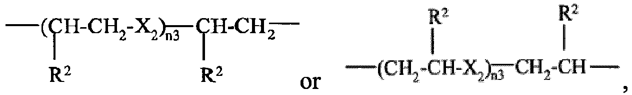 wherein X₂ is -O- or -S-, n3 is an integer from 1 to 6, and R² is as described above;
   h) 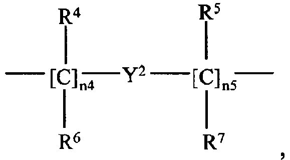 n4 is an integer from 0 to 10, n5 is an integer from 1 to 10, R⁴, R⁵, R⁶, R⁷ are each independently selected from H and linear or branched C₁-C₄ alkyl, and -ONO₂ is connected to 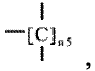 n5 is as described above, Y² is a 5- or 6-membered saturated, unsaturated or aromatic ring, and contains one or more heteroatoms selected from the group consisting of nitrogen, oxygen and sulfur.

In some embodiments, the NO-NSAID compound comprises a structure represented by Formula (21): 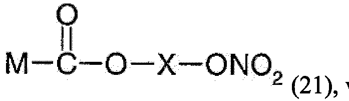 wherein X is a linker and M is a structure selected from the following group: 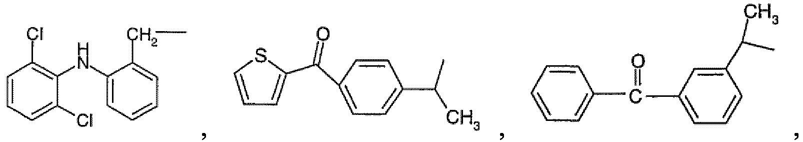 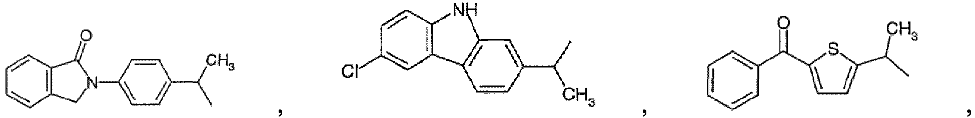 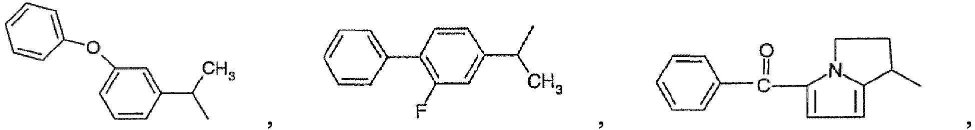 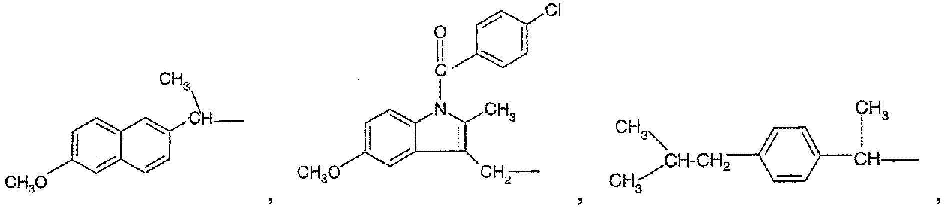 and

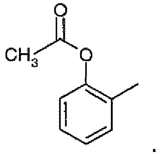

In some embodiments, the NO-NSAID compound comprises a compound selected from the group consisting of: 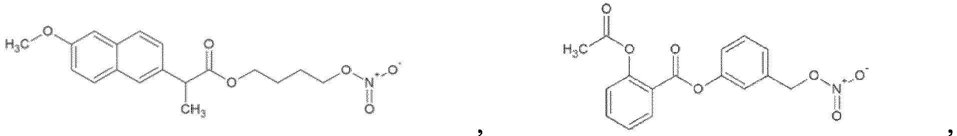 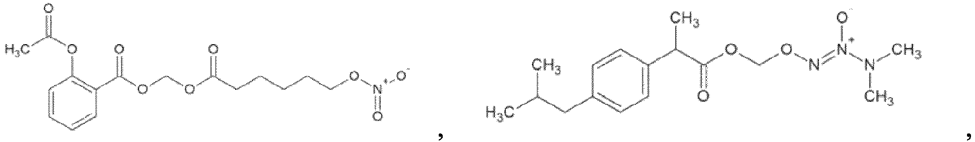 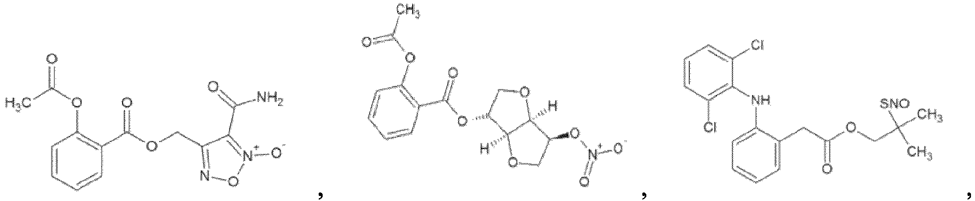 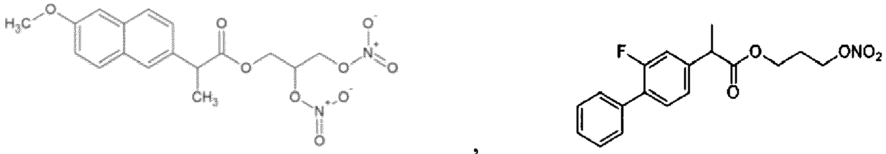 and

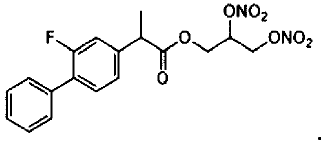

In the NO-NSAID compound described herein, the molar ratio of the NO-releasing moiety to the NSAID moiety may be from about 10:1 to about 1:10. For example, the molar ratio of the NO-releasing moiety to the NSAID moiety may be about 9:1 to about 1:1, about 8:1 to about 1:1, about 7:1 to about 1:1, about 6: 1 to about 1:1, about 5:1 to about 1:1, about 4:1 to about 1:1, about 3:1 to about 1:1, about 2:1 to about 1:1. In some embodiments, the molar ratio of the NO-releasing moiety to the NSAID moiety is about 1:1 to about 1:8, about 1:1 to about 1:7, about 1:1 to about 1:6 , about 1:1 to about 1:5, about 1:1 to about 1:4, about 1:1 to about 1:3, about 1:1 to about 1:2. In some embodiments, the molar ratio of the NO-releasing moiety to the NSAID moiety is about 3:1. In some embodiments, the molar ratio of the NO-releasing moiety to the NSAID moiety is about 2:1. In some embodiments, the molar ratio of the NO-releasing moiety to the NSAID moiety is about 1:1. In some embodiments, the molar ratio of the NO-releasing moiety to the NSAID moiety is about 1:2. In some embodiments, the molar ratio of the NO-releasing moiety to the NSAID moiety is about 1:3.

### Anti-tumor agent

According to any aspect of the present application, the anti-tumor agent comprises a small molecule compound, a small molecule conjugate, a protein and/or a polynucleotide.

For example, the anti-tumor agents may comprise a chemotherapeutic agent, a targeted therapeutic agent, and/or an immunotherapeutic agent.

In some embodiments, the anti-tumor agent is a targeted therapeutic agent. The targeted therapeutic agents may comprise a small molecule compound and/or antibody or antigen-binding fragment thereof. The antibody may comprise a monoclonal antibody, a multispecific antibody, a chimeric antibody, a humanized antibody, a fully human antibody and/or an antibody-drug conjugate. The antigen-binding fragment may comprise Fab, Fab', F(ab)₂, Fv fragment, F(ab')₂, scFv, di-scFv and/or dAb.

In some embodiments, the targeted therapeutic agent targets a molecule within a tumor cell, on the surface of a tumor cell, and/or in the tumor microenvironment. For example, the targeted therapeutic agent can target a protein and/or nucleic acid molecule. For example, the targeted therapeutic agent can target a tumor-associated antigen.

In some embodiments, the targeted therapeutic agent targets one or more targets selected from the group consisting of VEGF, EGFR, EGFR1, EGFR2, EGFR3, EGFR4, HER2, HER3, HER4, VEGFR, VEGFR1, VEGFR2, VEGFR3, VEGFR4 , PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic may inhibit the activity of one or more targets selected from the group consisting of: VEGF, EGFR, EGFR1, EGFR2, EGFR3, EGFR4, HER2, HER3, HER4, VEGFR, VEGFR1, VEGFR2, VEGFR3, VEGFR4 , PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic agent can reduce the expression of one or more targets selected from the group consisting of: VEGF, EGFR, EGFR1, EGFR2, EGFR3, EGFR4, HER2, HER3, HER4, VEGFR, VEGFR1, VEGFR2, VEGFR3, VEGFR4 , PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF -1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

For example, the targeted therapeutic agents may comprise a hormone, a signal transduction inhibitor, a gene expression modulator, an apoptosis inducer, an angiogenesis inhibitor, and/or a toxin delivery molecule.

In some embodiments, the targeted therapeutic agent is a tyrosinase inhibitor.

In some embodiments, the targeted therapeutic agent is a VEGFR inhibitor and/or a VEGF inhibitor. For example, the VEGFR inhibitor can inhibit VEGFR1, VEGFR2 and/or VEGFR3. In some embodiments, the targeted therapeutic agent is an EGFR inhibitor. In some embodiments, the targeted therapeutic agent is a BRAF inhibitor. In some embodiments, the targeted therapeutic agent is a PDGFR inhibitor. In some embodiments, the targeted therapeutic agent is a FGFR inhibitor. In some embodiments, the targeted therapeutic agent is an mTOR inhibitor. In some embodiments, the targeted therapeutic agent is a HER2 inhibitor.

For example, in any embodiment of the present application, the EGFR (eg, Her2) inhibitor may be selected from the following compounds and pharmaceutically acceptable salts thereof: afatinib, olmutinib, lapatinib, gefitinib and dacomitinib.

For example, in any embodiment of the present application, the VEGFR inhibitor and/or VEGF inhibitor may be selected from the following compounds and pharmaceutically acceptable salts thereof: ramucirumab, bevacizumab, anlotinib, regorafenib, cabozantinib, lenvatinib, sorafenib, fruquintinib, famitinib, apatinib, axitinib and nintedanib.

For example, in any embodiment of the present application, the BRAF inhibitor may be selected from the following compounds and pharmaceutically acceptable salts thereof: vemurafenib, encorafenib, selumetinib and dabrafenib.

For example, in any embodiment of the present application, the PDGFR inhibitor may be selected from the following compounds and pharmaceutically acceptable salts thereof: sunitinib and nintedanib.

For example, in any embodiment of the present application, the FGFR inhibitor may be selected from the following compounds and pharmaceutically acceptable salts thereof: erdafitinib and infigratinib.

For example, in any embodiment of the present application, the mTOR inhibitor may be selected from the following compounds and pharmaceutically acceptable salts thereof everolimus.

For example, the targeted therapeutic agent described in the present application may be selected from one or more of the following group: afatinib, dacomitinib, osimertinib, EAI045, gefitinib, almonertinib, pyrotinib, brigatinib, neratinib, olmutinib, bosutinib, icotinib, vandetanib, lapatinib, aflutinib, BPI-7711, mobocertinib, dovitinib, zorifertinib, varlitinib, orelabrutinib, acalabrutinib, brutinib, ibrutinib, dasatinib, pirtobrutinib, tolebrutinib, rilzabrutinib, fenebrutinib, evobrutinib, selumetinib, tivozanib, dovitinib, surufatinib, binimetinib, cobimetinib, trametinib, regorafenib, GSK-1120212, alpelisib, duvelisib, copanlisib, idelalisib, nortriptyline, inavolisib, dactolisib, apitolisib, parsaclisib, buparlisib, rigosertib, enzastaurin, paxalisib, leniolisib, ipatasertib, zotarolimus, sirolimus, everolimus, temsirolimus, sorafenib, apatinib, lenvatinib, sunitinib, cabozantinib, axitinib, nintedanib, brivanib, vatalanib, fruquintinib, dabrafenib, vemurafenib, encorafenib , pazopanib, crizotinib, panobinostat, erlotinib, rituximab, panitumumab, cetuximab, erfonrilimab, efactinib, cadonilimab, ramucirumab, bevacizumab, anlotinib, ponatinib, famitinib, erdafitinib, AZD4547, infigratinib, BCD -217, amivantamab, MCLA-129, EMB-01, LY3164530, JNJ-61186372, anti-EGFR and cMet bispecific antibody, GB263, their pharmaceutically acceptable salts and any combination thereof. Wherein, the EGFR and cMet bispecific antibody can be as described in WO2010115551A1, WO2014081954A1, or WO2015016559A1.

According to any aspect of the present application, the targeted therapeutic agent may be administered in combination with one or more other therapies.

In some embodiments, the anti-tumor agent is a chemotherapeutic agent. For example, the chemotherapeutic agent may comprise a pyrimidine nucleoside analog and/or a prodrug thereof.

In some embodiments, the chemotherapeutic agent comprises one or more selected from the group consisting of: capecitabine, cytarabine, docetaxel, doxorubicin, fluorouracil (5-FU), floxuridine, tegafur, idarubicin, paclitaxel, epirubicin, acelarin (NUC-1031), doxorubicin, leucovorin, cisplatin, paclitaxel, cyclophosphamide, vincristine and 5-FU prodrug.

In some embodiments, the chemotherapeutic agent comprises one or more selected from the group consisting of: tegafur, 5'-deoxy-fluorouridine, floxuridine, 2'-deoxy-fluorouridine, prodrug derivative of floxuridine, prodrug derivative of 2'-deoxy-fluorouridine, trifluoro-methyl-2'-deoxyuridine, 6-azauridine and/or 3-deazauridine.

For example, the chemotherapeutic agent may be administered in combination with one or more other therapies. In some embodiments, the one or more other therapies comprises one or more other anti-tumor therapies (eg, anti-tumor agents) described herein.

For example, the anti-tumor agent (e.g., a cytotoxic anti-cancer agent) may comprise alkylating agents, such as, nitrogen mustard, nitrogen mustard N-oxide hydrochloride, chlorambucil, cyclophosphamide, ifosfamide, thiotepa, isothiocyanate, busulfan, nimustine hydrochloride, mitropium bromide, melphalan, dacarbazine, ranimustine, sodium phopofol phosphate, ethylenetriamine, carmustine, lomustine, streptozotocin, pipobroman, ethoglucid, carboplatin, *cis-*platinum, miriplatin, nedaplatin, tinidamine, omustine, dichloropyridine, fupisitan, prednifixine, pumitepa, Ribomustin hydrochloride, temozolomide, diclofenac, trovafloxacin, zinostatin, simvastatin, penem, cystemustine and bizelesin; antimetabolites, such as, mercaptopurine, 6-mercaptopurine nucleoside, thioinosine, methotrexate, pemetrexed, entectine, cytarabine, oxaliplatin, tisabatin hydrochloride, 5-FU and derivatives thereof (e.g., fluorouracil, tegafur, UFT, dosiholu, carmofur, capecitabine, etc.), aminopterin, nazothioamine, calcium leucovorin, microphyllum, calcium folinate, calcium levofate, cladribine, emitoful, fludarabine, gemcitabine, hydroxylurea, pentostatin, piritrexim, iodouridine, nitoguanone, thiazolylfuran, vimalstat and bendamustine; anti-tumor antibiotics, such as, dactinomycin D, dactinomycin C, mitomycin C, chromomycin A3, bleomycin hydrochloride, bleomycin sulfate, cetiamycin hydrochloride, doxorubicin hydrochloride, mitoxantrone hydrochloride and idarubicin hydrochloride; and/or, etoposide, etoposide phosphate, vinblastine sulfate, vincristine sulfate, teniposide, paclitaxel, docetaxel and vinorelbine, and other plant-derived cytotoxic anticancer drugs; VEGF inhibitors such as bevacizumab, and VEGF inhibitors disclosed in PCT patent applications WO 2005/012359, WO 2005/044853, WO 98/45332, WO 96/30046, WO 94/10202, US patents US7,060,269, US6,582,959, US6,703,020, US6,054,297, US patent applications US2006/009360, US2005/0186208, US2003/0206899, US2003/0190317, US2003/0203409 and US2005/0112126.

In some embodiments, the anti-tumor agent may be an immunotherapy anti-tumor agent, which may comprise, for example: bropirimine, Krestin, etofuran, lentinan, ubenimex, interferon, interleukin, macrophage colony stimulating factor, granulocyte colony stimulating factor, erythropoietin, lymphotoxin, BCG vaccine, corynebacterium parvum, everolimus, levamisole, polysaccharide K, procodazole and/ or immune checkpoint inhibitors (eg, CTLA4 inhibitors, TIM-3 inhibitors, PD-1 inhibitors (eg, Nivolumab, Pembrolizumab, Pidilizumab, AMP514 (Amplimmune), AMP-224, and other PD-1 inhibitors disclosed in PCT patent applications WO2006/121168, WO2009/114335, WO2009/101611, US patent US 8609089, US patent applications US2010/028330, US2012/0114649), PD-L1 inhibitors (e.g., YW243.55.S70, MPDL3280A, MEDI-4736, MSB-0010718C, MDX-1105, and other PD-L1 inhibitors disclosed in PCT patent application WO2010/077634 and US patent US7,943,743)).

In some embodiments, the anti-tumor agent may hormonotherapeutic anticancer agent. For example, fusitatine, stilboestrol, chlorinated costen, medroxyprogesterone acetate, megestrol acetate, cyproterone acetate, cyproterone acetate, danazol, allylestrenol, progesterone, mepartricin, raloxifene or meloxifene, levofloxacin, antiestrogen (e.g., tamoxifen citrate, toremifene citrate, etc.), contraceptive, quanliehanwa, testolactone, aminosuccinimide, LH-RH agonists (e.g., goserelin acetate, buserelin, leuprorelin, etc.), droloxifene, epiandrostanol, ethinylestradiol sulfonate, fubenzole hydrochloride, anastrozole, letrozole, exemestane, vorozole, antiandrogen (e.g., flutamide, bicalutamide, nilutamide, etc.), 5α-reductase inhibitor (e.g., finasteride, Epristeride), corticosteroids (e.g., dexamethasone, prednisolone, betamethasone, triamcinolone, etc.) and/or androgen synthesis inhibitor (e.g., abiraterone, etc.).

### Diseases or disorders associated with anti-tumor agents

According to any aspect of the present application, the disease or disorder associated with the administration of the anti-tumor agent may be caused by the anti-tumor alone or may be caused by multiple treatment regimens, but including the anti-tumor agent.

In some embodiments, the disease or disorder is caused by administration of the anti-tumor agent. For example, the disease or disorder may onset or progression after administration of the anti-tumor agent.

In some embodiments, the subject did not suffer from the disease or disorder prior to administration of the anti-tumor agent.

For example, the disease or disorder may comprise an epithelial tissue disease or disorder. The epithelial tissue disease or disorder may comprise a disease or disorder associated with endothelial cell lesions, and/or a disease or disorder associated with epithelial cell lesions. For example, the epithelial cells may comprise skin epithelial cells, oral epithelial cells, nasal epithelial cells, gastric epithelial cells, and/or intestinal epithelial cells.

In some embodiments, the endothelial cells comprise vascular endothelial cells. The vascular endothelial cell lesions may comprise endothelial dysfunction. For example, the endothelial cell lesions may comprise degenerative vascular diseases (e.g., atherosclerosis, Mönckeberg arteriosclerosis, and arteriosclerosis (e.g., hyline degenerative arteriosclerosis and proliferative arteriosclerosis)), inflammatory vascular diseases (eg, infectious arteritis, syphilitic arteritis, giant cell arteritis, thromboangiitis obliterans, and rheumatic arteritis), functional vascular disease (eg, Raynaud's disease, acrocyanosis, and erythromelalgia) and/or congenital vascular disease (e.g., congenital arteriovenous fistula), etc.

In the present application, the epithelial cells may comprise skin epithelial cells, oral epithelial cells, nasal cavity epithelial cells, gastric epithelial cells and/or intestinal epithelial cells. For example, the epithelial cell lesions may comprise cutaneous epithelial cell lesions (e.g., rash, acne, rosacea, atopic dermatitis, contact dermatitis, seborrheic dermatitis, lupus, scleroderma, pemphigus, pigmentation, melasma, vitiligo, urticaria, tinea corporis, pruritus, alopecia, hair changes, erythema, paronychia and schizonychia, dry skin, hypersensitivity, and psoriasis), oral epithelial lesions (e.g., pemphigus, Cold sores, herpetic stomatitis, granulomatous cheilitis, oral ulcers, pemphigoid, Sjögren's syndrome, Behcet's syndrome and oral sarcoidosis, etc.), nasal epithelial cell lesions (epistaxis, sinusitis, nasal furuncle and nasal polyps, etc.), gastric epithelial cell lesions (e.g., gastritis, intestinal metaplasia, gastric perforation, gastric fistula, gastric ulcer, and gastrointestinal polyp) and/or small intestinal epithelial cell lesions (e.g., enteritis, Crohn's disease, enterobrosis, intestinal fistula, enterelcosis, ulcerative colitis and NSAIDs enteropathy), etc.

The inventors of the present application have discovered that anti-tumor agents can cause damage to endothelial cells and endothelial tissue, thereby causing diseases or disorders of skin tissues, oral tissues, nasal cavity tissues and/or gastrointestinal tissues. In the onset and progression of these diseases or disorders, the disease process typically begins with damage/lesion of endothelial cells and endothelial tissues, and epithelial cells may also exhibit pathological changes, ultimately manifesting in the patient as endothelial cell lesions associated with the administration of anti-tumor agents and/or epithelial cell lesions associated with the administration of anti-tumor agents.

For example, the disease or disorder may comprise a skin disease or disorder, a sensory disease or disorder, and/or a gastrointestinal disease or disorder.

In some embodiments, the skin disease or disorder comprises alopecia, body odor, bullous dermatitis, dry skin, eczema, erythema multiforme, erythroderma, lipoatrophy, hair color changes, abnormal hair texture, hirsutism, hyperhidrosis, hyperkeratosis, hypertrichosis, hypohidrosis, lipohypertrophy, nail changes, nail discoloration, nail loss, nail ridges, skin pain, hand-foot syndrome, photosensitivity, pruritus, purpura, acneiform rash, maculopapular rash, scalp pain, skin atrophy, skin hyperpigmentation, skin hypopigmentation, skin induration, skin ulcers, Stevens -Johnson syndrome, subcutaneous emphysema, telangiectasia, toxic epidermal necrosis, rash and/or urticaria.

In some embodiments, the skin disease or disorder is hand-foot syndrome.

In some embodiments, the present application relates to the use of the NO-NSAID compound to prevent, ameliorate and/or treat a disease or disorder (for example, hand-foot syndrome) in a subject associated with the administration of the anti-tumor agent (e.g., a VEGFR inhibitor and/or a VEGF inhibitor).

In some embodiments, the present application relates to the use of the NO-NSAID compound to prevent, ameliorate and/or treat a disease or disorder (e.g., rash) in a subject associated with the administration of the anti-tumor agent (e.g., EGFR inhibitors).

In some embodiments, the severity of the skin disease or disorder is based on NCI-CTCAE V5.0 grade 1 or above, grade 2 or above, grade 3 or above, grade 4 or above, or grade 5.

In some embodiments, the subject comprises a cancer patient. In some embodiments, the skin disease or disorder affects a different site than the cancer.

In the present application, the disease or disorder associated with the administration of an anti-tumor agent may exhibit statistically significant correlations with the anti-tumor agent. In some embodiments, the disease or disorder associated with administration of the anti-tumor agent may be caused by the anti-tumor agent. For example, the disease or disorder associated with the administration of the anti-tumor agent may comprise a skin disease or disorder, a sensory disease or disorder, and/or a gastrointestinal disease or disorder associated with the administration of the anti-tumor agent. For example, the skin disease or disorder, the sensory disease or disorder, and/or the gastrointestinal disease or disorder associated with the administration of the anti-tumor agent may comprise epithelial tissue diseases or disorders associated with the administration of the anti-tumor agent in the skin tissue, sensory features, and/or gastrointestinal tract. In some embodiments, the disease or disorder associated with the administration of the anti-tumor agent may comprise side effects or adverse reactions caused by the administration of the anti-tumor agent.

In the present application, the disease or disorder associated with the administration of the anti-tumor agent may be a new indication, which may be distinct from any previously known disease or disorder. For example, the diagnostic methods, treatment modalities, and/or symptoms of the disease or disorder associated with administration of the anti-tumor agent are unique. For instance, erythromycin ointment may treat rashes, but has no therapeutic effect on rashes associated with the administration of anti-tumor agents.

In some embodiments, the disease or disorder associated with the administration of the anti-tumor agent may comprise rash associated with the administration of the anti-tumor agent, hand-foot syndrome associated with the administration of the anti-tumor agent, pruritus associated with the administration of the anti-tumor agent, erythema associated with the administration of anti-tumor agents, dry skin associated with the administration of anti-tumor agents, alopecia associated with the administration of anti-tumor agents, paronychia associated with the administration of anti-tumor agents, pigmentation disorders associated with the administration of anti-tumor agents, oral ulcers associated with the administration of anti-tumor agents, xerostomia associated with the administration of anti-tumor agents, epistaxis associated with the administration of anti-tumor agents, nasopharyngitis associated with the administration of anti-tumor agents, cheilitis associated with the administration of anti-tumor agents, esophagitis associated with the administration of anti-tumor agents, esogastritis associated with the administration of anti-tumor agents, gastric ulcer associated with the administration of anti-tumor agents, diarrhea associated with the administration of anti-tumor agents, vomiting associated with the administration of anti-tumor agents, nausea associated with administration of an anti-tumor agent, anorexia associated with administration of an anti-tumor agent, constipation associated with administration of an anti-tumor agent, and/or, abdominal pain associated with administration of an anti-tumor agent. For example, the disease or disorder associated with administration of an anti-tumor agent comprises hand-foot syndrome associated with administration of an anti-tumor agent. For example, the severity of the disease or disorder associated with the administration of the anti-tumor agent is based on NCI-CTCAE V5.0 grade 1 or above, grade 2 or above, grade 3 or above, grade 4 or above, and/or grade 5.

In some embodiments, the disease or disorder may comprise rash, hand-foot syndrome, pruritus, erythema, dry skin, alopecia, paronychia, pigmentation disorders, oral ulcers, xerostomia, epistaxis, nasopharyngitis, cheilitis, esophagitis, esogastritis, gastric ulcer, diarrhea, vomiting, nausea, anorexia, constipation and/or abdominal pain. For example, the disease or disorder comprises hand-foot syndrome.

In some embodiments, the disease or disorder associated with the anti-tumor agent cannot effectively be treated or alleviated by administering an agent selected from the group consisting of: 1% sildenafil, urea cream, vaseline ointment, urea ointment, brimonidine ointment, vitamin B6 ointment, nicotine ointment, dexamethasone ointment, hydrocortisone ointment, vitamin K1 ointment (0.1%), erythromycin ointment and triamcinolone acetonide ointment.

In the present application, the severity of the disease or disorder may increase after the administrating the anti-tumor agent. For instance, the severity of the disease or disorder may increase by about 5% or above, about 10% or above, about 15% or above, about 20% or above, about 25% or above, about 30% or above, about 35% or above, about 40% or above, about 45% or above, about 50% or above, about 60% or above, about 70% or above, about 80% or above, about 90% or above, about 100% or above, about 200% or above or more.

As used herein, the subject may not suffer the disease or disorder before the administration of the anti-tumor agent. As used herein, the term "the subject did not suffer the disease or disorder" generally refers to the subject has no previous medical history related to the disease or disorder associated with administration of an anti-tumor agent. For instance, the subject doesn't suffer the disease or disorder associated with the administration of the anti-tumor agent for one day more, one week more, one month more, one year more, ten years more or since the birth of the subject before the administration of the anti-tumor agent.

### Pharmaceutical combinations and pharmaceutical compositions

According to any aspect of the application, the medicament does not substantially affect the therapeutic effect of the anti-tumor agent.

In the present application, the " does not substantially affect " may refer to compared to the therapeutic effect of using the anti-tumor agent alone, the therapeutic effect of using the NO-NSAID compound and the anti-tumor agent is equivalent, or not worse significantly. For instance, to any subject, compared to the therapeutic effect of using the anti-tumor agent alone, the decreased tumor volume caused by the using the NO-NSAID compound and the anti-tumor agent is equivalent, or decreased by not less than about 5%, not less than about 4%, not less than about 3%, not less than about 2%, not less than about 1%, not less than about 0.5%, not less than about 0.1%, not less than about 0.01% , not less than about 0.001% or less.

In some embodiments, the medicament or the NO-NSAID compound is prepared for topical administration. For example, the medicament or the NO-NSAID compound may be prepared for transdermal administration. In some embodiments, the medicament or the NO-NSAID compound is prepared for topical skin administration. In some embodiments, the medicament is prepared as a cream, lotion, gel, ointment, salve, spray, liposomal formulation, liniment, and/or aerosol. In some embodiments, the medicament or the NO-NSAID compound may be prepared as an ointment.

In some embodiments, the medicament or the NO-NSAID compound is an oral formulation. In some embodiments, the medicament or the NO-NSAID compound can be an injectable formulation. In some embodiments, the medicament or the NO-NSAID compound can be used for topical administration in the oral cavity.

In some embodiments, the administration site of the medicament is different from the administration site of the anti-tumor agent. In some embodiments, the administration site of the medicament is not the site of cancer occurrence or potential metastasis.

In some embodiments, the administration method of the medicament is different from the administration method of the anti-tumor agent.

In the medicament, the concentration of the NO-NSAID compound may be from about 0.0001 % to about 50%. For example, the concentration of the NO-NSAID compound may be from about 0.0001% (w/w) to about 40% (w/w), for instance, the concentration may be from about 0.0001% (w/w) to about 10% (w/w ), about 0.0001% (w/w) to about 9.5% (w/w), about 0.0001% (w/w) to about 9% (w/w), about 0.0001% (w/w) to about 8.5% (w/w), about 0.0001% (w/w) to about 8% (w/w), about 0.0001% (w/w) to about 7.5% (w/w), about 0.0001 % (w/w) to about 7% (w/w), about 0.0001 % (w/w) to about 6.5% (w/w), about 0.0001% (w/w) to about 6% (w/w), about 0.0001% (w/w) to about 5.5% (w/w), about 0.0001% (w/w) to about 5% (w/w), about 0.0001% (w/w) to about 4.5% (w/w), about 0.0001% (w/w) to about 4% (w/w), about 0.0001% (w/w) to about 3.5% (w/w), about 0.0001 % (w/w) to about 3% (w /w), about 0.0001% (w/w) to about 2.5% (w/w), about 0.0001% (w/w) to about 2% (w/w), about 0.0001% (w/w) to about 1.5% (w/w), about 0.0001% (w/w) to about 1% (w/w), about 0.0001% (w/w) to about 0.5% (w/w), about 0.0001% (w/ w) to about 0.01% (w/w) or less. In the medicament of the present application, the concentration of the NO-NSAID compound may be about 0.0001% (w/w) to about 1% (w/w), about 0.0001% (w/w) to about 0.9% (w/w), about 0.0001% (w/w) to about 0.6% (w/w), about 0.05% (w/w) to about 0.5% (w/w), about 0.05% (w/w) to about 0.4% (w/w), about 0.05% (w/w) to about 0.3% (w/w), about 0.05% (w/w) to about 0.2% (w/w), about 0.1% (w /w) to about 0.2% (w/w) or less. For example, the concentration of the NO-NSAID compound can be from about 0.5% to about 10%. In some embodiments, the concentration of the NO-NSAID compound is about 5% (w/w). In some embodiments, the concentration of the NO-NSAID compound is about 2.5% (w/w). In some embodiments, the concentration of the NO-NSAID compound is about 1% (w/w).

In the present application, one or more other active ingredients may also be included in the medicament. For example, the active ingredient may refer to a monomeric compound with medical utility or physiological activity. For example, the further active ingredient may be selected from the group consisting of: anti-inflammatory agents, analgesics, local anesthetics, antibiotics, antihistamines, preservatives, immunosuppressants and anti-hemorrhagic agents.

In the present application, the medicament may also comprise a pharmaceutically acceptable carrier. For example, the pharmaceutically acceptable carrier may be selected from the group consisting of fillers, binders, disintegrants, buffers, preservatives, lubricants, flavoring agents, thickeners, colorants, and emulsifiers.

In the pharmaceutical combinations and/or kits of the present application, in some embodiments, the anti-tumor agent and the NO-NSAID compound are not mixed with each other.

In some embodiments, the anti-tumor agent and the NO-NSAID compound are each independently present in separate containers. For example, the pharmaceutical combination may comprise 2 or more drugs independently packaged from each other, wherein at least one of the drugs contains the anti-tumor agent described herein, and at least one other drug contains the NO-NSAID compounds described herein.

In some embodiments, in the pharmaceutical combination, wherein the NO-NSAID compound in 2) is capable of preventing, ameliorating and/or treating a disease or disorder associated with the anti-tumor agent in 1).

In some embodiments, the NO-NSAID compound of 2) does not substantially affect the therapeutic effect of the anti-tumor agent of 1).

In some embodiments, in the pharmaceutical combination of the present application, the NO-NSAID compound of 2) is administered before, simultaneously with, or after the anti-tumor agent of 1).

### Prevention and/or treatment methods

In the present application, the subject may comprise humans or non-human animals. For example, the non-human animal may be selected from the group consisting of monkey, chicken, goose, cat, dog, mouse and rat. In addition, non-human animals may also comprise any animal species other than humans, such as livestock animals, or rodents, or primates, or domestic animals, or poultry animals. The person may be Caucasian, African, Asian, Semitic, or other races, or a hybrid of various races. For another example, the person may be an elderly, adult, teenager, child, or infant.

The effective dose in humans can be extrapolated based on effective doses observed in experimental animals. For example, Freireich et al. described the relationship between doses in animals and humans (based on milligrams per square meter of body surface area) (Freireich et al., Cancer Chemother. Rep. 50, 219 (1966)). Body surface area can be approximately determined from the patient's height and weight. See, for example, Scientific Tables, Geigy Pharmaceuticals, Ardsley, NY, 537 (1970).

In the methods of the present application, the anti-tumor agent can be caused by administering an anti-tumor agent to the subject.

For example, the NO-NSAID compound can be administered before, simultaneously with, or after the anti-tumor agent is administered to the subject. When the anti-tumor agent described herein is administered simultaneously with the NO-NSAID compound, the NO-NSAID compound is administered at a dosage level of about 0.0001-10% to the total dose (e.g., about 0.005-10%, about 0.01-10%, about 0.05-10%, about 0.1-10%, about 0.2-10%, about 0.3-10%, about 0.4-10%, about 0.5-10%, about 0.6-10%, about 0.7-10%, about 0.8 -10%, about 0.9-10%, about 0.95-10%, about 1-10%, about 2-10%, about 3-10%, about 5-10%, about 6-10%, about 8-10% or less). In embodiments where the NO-NSAID compound is administered separately from the anti-tumor agent, the NO-NSAID compound can be administered before or after the anti-tumor agent. The interval between administrations can be 1 minute, 2 minutes, 5 minutes, 10 minutes, 20 minutes, 30 minutes, 45 minutes, 1 hour, 2 hours, 3 hours, 4 hours, 5 hours, 6 hours, 12 hours, 18 hours, 1 day, 2 days, 3 days, 1 week, 2 weeks, 3 weeks, 1 month, 2 months, 3 months or longer.

The application also provides a method comprising administering an NO-NSAID compound to a subject, wherein the subject has been, is being and/or will be administered an anti-tumor agent and has or is susceptible to a disease or disorder associated with the administration of an anti-tumor agent.

The present application also provides a method for preventing or treating a disease or disorder, comprising administering an NO-NSAID compound to a subject susceptible to or suffering from the disease or disorder, wherein the subject has been, is being, and /or will be administered an anti-tumor agent.

In the present application, the subject may already suffer from a disease or disorder associated with administration of an anti-tumor agent, or the subject may have a greater probability of suffering from a disease or disorder associated with administration of an anti-tumor agent.

The present application also provides a method, comprising the following steps: 1) monitoring one or more side effects in a subject being administered an anti-tumor agent, such as characteristics of skin tissue, sensory organs and/or the gastrointestinal tract; 2) when the monitoring indicates that the subject is experiencing side effects associated with the administration of the anti-tumor agent, such as skin diseases or disorders, sensory organ diseases or disorders, and/or gastrointestinal diseases or disorders, administering an NO-NSAID compound to the subject.

In the present application, the method may further comprise continuing to monitor the skin disease or disorder, the sensory disease or disorder, and/or the gastrointestinal disease or disorder, and optionally reducing or discontinuing the anti-tumor agent. For example, the continued monitoring may refer to monitoring for about at least 1 day, at least 1 week, at least 10 days, at least 2 weeks, at least 3 weeks, at least 1 month, at least 3 months or longer after administration of the anti-tumor agent. For example, the reduction or discontinuation may refer to the dose of the anti-tumor agent administered to the subject is reduced by about at least 5%, at least 10%, at least 20%, at least 30%, at least 40%, at least 50%, at least 60%, at least 70%, at least 80%, at least 90%, at least 95%, at least 99% or 100% compared to the dose of the anti-tumor agent in step 1) of the method.

As used herein, the severity of the disease or disorder associated with administration of an anti-tumor agent may increase after administration of the anti-tumor agent. For example, the severity may be an increase of at least 5%, at least 10%, at least 20%, at least 30%, at least 40%, at least 50%, at least 60%, at least 70%, at least 80%, at least 90%, or more.

As used herein, the subject may did not suffered from the disease or disorder prior to administration of the anti-tumor agent.

In the present application, the NO-NSAID compound can be topically administered to the subject. For example, the NO-NSAID compound can be topically administered to a site in the subject that is essentially free of cancer cells. As another example, the NO-NSAID compound can be administered to a non-cancerous site of the subject.

Without being limited by any theory, the following examples are only for the purpose of illustrating the NO-NSAID compounds of the present application, preparation methods and uses, etc., and are not intended to limit the invention scope of the present application.

### Example

### List of NO-NSAID compounds

In the following examples, the NO-NSAID compounds used in the present application are shown in Table 2-1:

**table 2-1**

| Compound structure | Compound code |
|---|---|
| 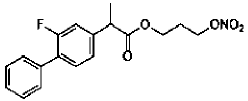 | A1 |
| 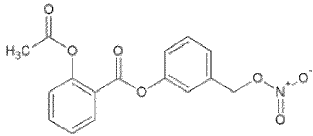 | A2 |
| 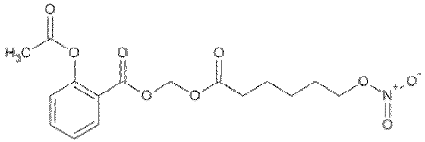 | A3 |
| 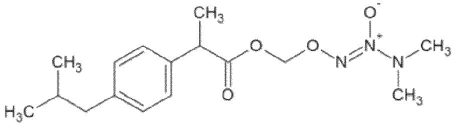 | A4 |
| 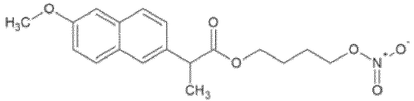 | A5 |
| 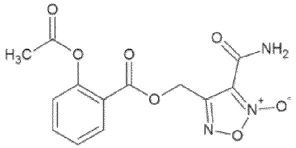 | A6 |
| 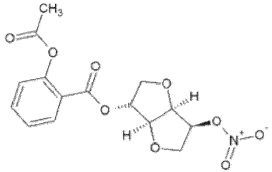 | A7 |
| 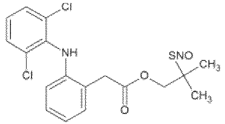 | A8 |
| 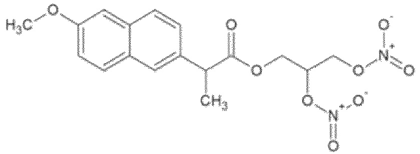 | A9 |
| 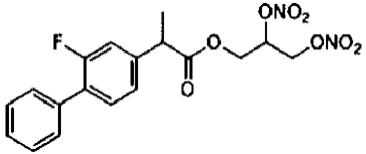 | A10 |

### NO-NSAIDs can prevent/treat hand-foot syndrome induced by anti-tumor agents in vivo

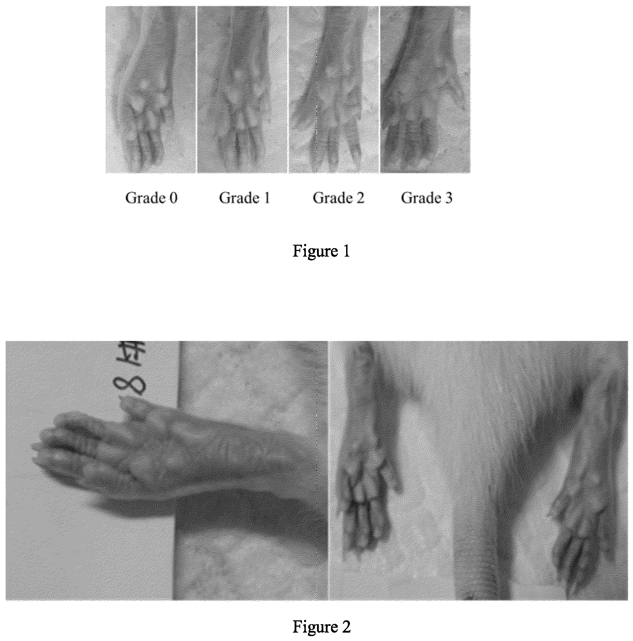

A rat animal model was constructed, and the anti-tumor agents shown in Table 2-2 were respectively administered to 8-week-old female SD rats by daily oral gavage. After several days, hand-foot syndrome symptoms appeared in the paws of the rats (as shown in Figure 1). Similar to humans, rats exhibit hand-foot syndrome symptoms after taking the anti-tumor agents, and the symptoms are very similar. Therefore, rats serve as an excellent animal model for simulating side effects (such as hand-foot syndrome) caused by anti-tumor agents.

After the rats (about 200 g) were fed and adapted for one week, they were divided into a NO-NSAID group and a control group, with 10 rats in each group, and a gavage administration test was conducted. The various anti-tumor agents were dissolve in a mixed solution of Cremophor EL: ethanol = 1:1. The anti-tumor agent solution were diluted with PBS to the required concentration (about 3 times with PBS solution) before gavage administration. The volume of each gavage administration should not exceed 2 mL, and the dosage is shown in Table 2-2. After gavage, an ointment containing NO-NSAID was applied to the hind paws of the rats, while the control group was applied with a blank ointment. After application, the rats were placed in a fixed cylinder for 4 hours. After 4 hours, the rats were released and the residual ointment was wiped off with clean water, then the rats were returned to the cage. The gavage frequency of inhibitors was shown in Table 2-2, and NO-NSAID was only applied once a day. The gavage and application operations were repeated daily until the end of the experiment. After 15-18 days of application, the number of rats in the treatment group that maintained normal or had significantly milder hand-foot syndrome than the blank control group was counted as the number of rats that effectively inhibited hand-foot syndrome.

Table 2-2 lists the animal experimental combinations of various anti-tumor agents and NO-NSAID ointments, as well as the corresponding experimental results (wherein the value in the control rate column = the hand-foot syndrome incidence rate in the blank group - the hand-foot syndrome incidence rate in the treatment group).

**Table 2-2 Experimental conditions and experimental results of Examples 1-14**

| Example | Anti-tumor agent | Dosage | Frequenc y | Treat ment | Concentrat ion wt% | Days | Control rate |
|---|---|---|---|---|---|---|---|
| | | | | A1 | 0.50% | | 42.86% |
| | | | | | 1% | | 42.86% |
| | | | | | 2.50% | | 60.00% |
| | | | | | 5% | | 83.33% |
| 1 | Sorafenib | 80mg/kg | Once a day | A2 | 1% | 5 | 50.00% |
| | | | | | 2% | | 50.00% |
| | | | | | 5% | | 66.67% |
| | | | | | 10% | | 80.00% |
| | | | | A3 | 1% | | 50.00% |
| | | | | | 3% | | 57.14% |
| | | | | A4 | 0.50% | | 42.86% |
| | | | | | 2% | | 71.43% |
| | | | | A5 | 0.50% | | 28.57% |
| | | | | | 1% | | 50.00% |
| | | | | | 2.50% | | 71.43% |
| | | | | | 5% | | 80.00% |
| | | | | A6 | 1% | | 66.67% |
| | | | | A7 | 1% | | 57.14% |
| | | | | A8 | 1% | | 42.86% |
| | | | | A9 | 1% | | 28.57% |
| | | | | | 5% | | 33.33% |
| | | | | A10 | 1% | | 28.57% |
| | | | | | 5% | | 40.00% |
| | | | | A1 | 0.50% | | 33.33% |
| | | | | | 1% | | 50.00% |
| | | | | | 2.50% | | 42.86% |
| | | | | | 5% | | 80.00% |
| | | | | A2 | 1% | | 57.14% |
| | | | | | 5% | | 66.67% |
| 2 | Regorafenib | 90mg/kg | Once a day | A3 | 1% | 18 | 42.86% |
| | | | | | 3% | | 71.43% |
| | | | | A5 | 0.50% | | 50.00% |
| | | | | | 1% | | 71.43% |
| | | | | | 2.50% | | 71.43% |
| | | | | | 5% | | 83.33% |
| 3 | Cabozantinib | 50mg/kg | | A1 | 1% | | 40.00% |
| | | | | | 2.50% | | 33.33% |
| | | | | | 5% | | 66.67% |
| | | | | A2 | 1% | | 28.57% |
| | | | | | 2.50% | | 42.86% |
| | | | | | 5% | | 66.67% |
| | | | | A6 | 1% | | 40.00% |
| | | | Once a day | | 3% | 18 | 42.86% |
| | | | | A7 | 1% | | 33.33% |
| | | | | | 3% | | 50.00% |
| | | | | A5 | 1% | | 42.86% |
| | | | | | 2.50% | | 60.00% |
| | | | | | 5% | | 57.14% |
| | | | | A8 | 1% | | 33.33% |
| | | | | | 5% | | 50.00% |
| | | | | A1 | 1% | | 33.33% |
| | | | | | 2.50% | | 66.67% |
| | | | | | 5% | | 66.67% |
| | | | | A2 | 1% | | 33.33% |
| 4 | Nintedanib | 110mg/kg | Once a day | | 3% | 18 | 50.00% |
| | | | | | 5% | | 66.67% |
| | | | | A5 | 1% | | 40.00% |
| | | | | | 2.50% | | 66.67% |
| | | | | | 5% | | 50.00% |
| 5 | Lenvatinib | 100mg/kg | Twice a day | A1 | 1% | 18 | 28.57% |
| | | | | | 2.50% | | 57.14% |
| | | | | | 5% | | 57.14% |
| | | | | A2 | 1% | | 42.86% |
| | | | | | 3% | | 42.86% |
| | | | | | 5% | | 71.43% |
| | | | | A5 | 1% | | 28.57% |
| | | | | | 2.50% | | 71.43% |
| | | | | | 5% | | 71.43% |
| 6 | Anlotinib | 20mg/kg | Once a day | A1 | 2.50% | 18 | 33.33% |
| | | | | A2 | 3% | | 66.67% |
| | | | | A5 | 2.50% | | 50.00% |
| 7 | Fruquintinib | 40mg/kg | Once a day | A1 | 2.50% | 16 | 57.14% |
| | | | | A2 | 3% | | 71.43% |
| | | | | A5 | 2.50% | | 42.86% |
| 8 | Apatinib | 105mg/kg | Once a day | A1 | 2.50% | 15 | 57.14% |
| | | | | A2 | 3% | | 50.00% |
| | | | | A5 | 2.50% | | 42.86% |
| 9 | Axitinib | 50mg/kg | Once a day | A1 | 2.50% | 18 | 60.00% |
| | | | | A2 | 3% | | 40.00% |
| | | | | A5 | 2.50% | | 66.67% |
| 10 | Lapatinib | 120mg/kg | Twice a day | A1 | 2.50% | 15 | 33.33% |
| | | | | A2 | 3% | | 33.33% |
| | | | | A5 | 2.50% | | 60.00% |
| 11 | Olmutinib | 100mg/kg | Once a day | A1 | 2.50% | 20 | 50.00% |
| | | | | A2 | 3% | | 60.00% |
| | | | | A5 | 2.50% | | 66.67% |
| 12 | Sunitinib | 120mg/kg | Once a day | A1 | 2.50% | 15 | 57.14% |
| | | | | A2 | 3% | | 42.86% |
| | | | | A5 | 2.50% | | 57.14% |
| 13 | Erdafitinib | 45mg/kg | Once a day | A1 | 2.50% | 20 | 42.86% |
| | | | | A2 | 3% | | 71.43% |
| | | | | A5 | 2.50% | | 57.14% |
| | | | | A7 | 3.00% | | 57.14% |
| 14 | Encorafenib | 80mg/kg | Once a day | A1 | 2.50% | 15 | 60.00% |
| | | | | A2 | 3% | | 42.86% |
| | | | | A5 | 2.50% | | 66.67% |
| | | | | A6 | 1% | | 40.00% |

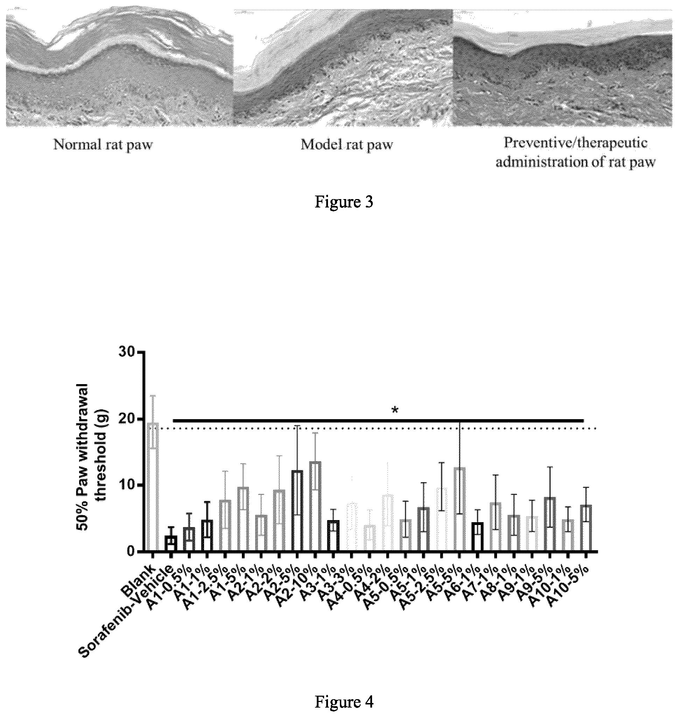

It can be seen from the results in Table 2-2 that the NO-NSAID ointment can effectively prevent and treat hand-foot syndrome caused by anti-tumor agents. At the same time, the results in Figure 2 also indicate that the NO-NSAID can effectively prevent and treat hand-foot syndrome caused by anti-tumor agents. After the experiment, paw samples were taken from rats in different groups for histological observation (Figure 3). The results showed that the NO-NSAID of the present application could effectively ameliorate hand-foot syndrome caused by anti-tumor agents, improve capillary conditions, and reduce keratinocyte necrosis, epidermal separation, inflammation and other phenomena. Furthermore, in the NO-NSAID compound of the present application, when the molar ratio of NSAID to NO is about 1:1, the control rate of hand-foot syndrome associated with the administration of anti-tumor agents is more excellent.

### NO-NSAID can prevent/treat hand-foot syndrome caused by macromolecular protein inhibitors in vivo

Ramucirumab or bevacizumab was diluted with saline to the required concentration. After the rats (about 200 g) were fed and adapted for one week, they were divided into groups, 10 rats in each group, for injection administration experiment. The diluted ramucirumab was intravenously infused for 60 minutes at a dose of 40 mg/kg, once a week, in combination with paclitaxel (10 mg/kg). Apply NO-NSAID ointment to the treatment group and blank ointment to the control group, following the same procedure as described above. The experiment lasts for 2-4 weeks, and observations are recorded.

Table 3 lists the animal experiments involving the administration of various macromolecular anti-tumor agents and NO-NSAID ointments, as well as the corresponding experimental results (wherein the value in the control rate column = the hand-foot syndrome mold incidence rate in the blank group - the hand-foot syndrome incidence rate in the treatment group).

**Table 3 Experimental conditions and experimental results of Examples 15-16**

| Examp le | Anti-tumor Agents | Dosage | Frequency | Treatme nt | Concentration wt % | Days | Control rate |
|---|---|---|---|---|---|---|---|
| 15 | Ramucirum ab | Combined with paclitaxel, injected for 60 minutes | Intraperiton eal injection once a week | A2 | 1% | 23 | 33.33 % |
| | | | | | 5% | | 66.67 % |
| | | | | A5 | 2.50% | | 33.33 % |
| | | | | | 5% | | 50.00 % |
| 16 | Bevacizum ab | 5-FU combinati on, injection 60-90 minutes | Tail vein injection once a week | A2 | 1% | 18 | 33.33% |
| | | | | | 5% | | 50.00% |
| | | | | A5 | 2.50% | | 50.00% |
| | | | | | 5% | | 66.67% |

It can be seen from the above experimental results that the NO-NSAID ointment of the present application has a certain preventive effect on hand-foot syndrome caused by macromolecular anti-tumor agents.

### NO-NSAID ointment alleviates the pain of hand-foot syndrome caused by anti-tumor agents

After conducting the above-mentioned preventive dosing experiments (Examples 1-14 involving the prevention part), pain analysis was performed on the rats using a mechanical sensitivity (Von Frey) pain assessment model. The experimental procedure are as follows: initially, let the rats adapt to the room for 1 hour. Subsequently, put the rats into an observation box with a metal mesh floor, and let the rats stay in the box for 20 minutes to adapt to the environment of the experimental platform. The pain of the paws was then detected using a Von Frey device, and the surface of the palm of the rat was stimulated with special cilia to detect the mechanical sensitivity of the animals, with a force of 0.4 g, 0.8 g, 1.5 g, 2.5 g, 4 g, 8 g, 10 g and 20 g (IITC Life Science, Woodland Hills, 2390 Series). After a specific pressure was applied, the rat's immediate withdrawal of the paw or licking was defined as a response, while the failure to withdraw the paw within 6 seconds was classified as no response. The rat movement response was considered an ambiguous response, the stimulation experiment was repeated in this case.

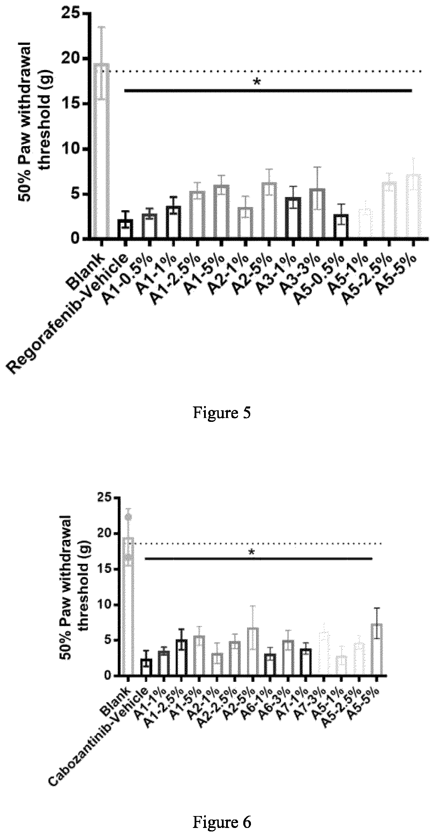

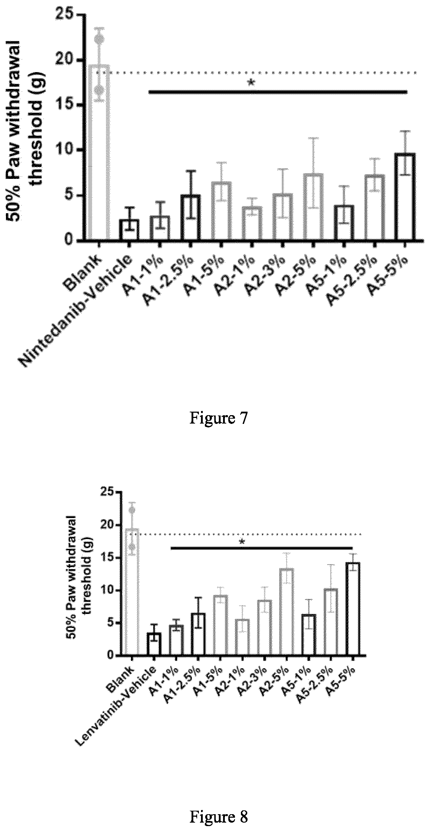

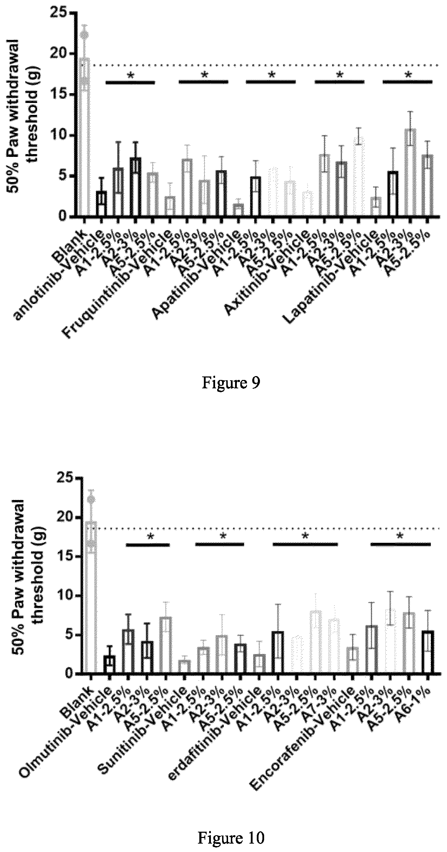

The results are shown in Figures 4-10, which show that NO-NSAID has a significant effect in alleviating the pain of hand-foot syndrome caused by anti-tumor agents.

### NO-NSAID ointment can effectively treat hand-foot syndrome caused by anti-tumor agents

After the rats (about 200 g) were fed and adapted for one week, the rats were divided into groups of 10 rats for gavage administration experiment. The anti-tumor agent was dissolved in a mixed solution of Cremophor EL: ethanol = 1:1, and diluted with PBS to the required concentration (diluted about 3 times with PBS solution) before gavage. The gavage volume did not exceed 2 mL, and the dosage was shown in Table 4. The anti-tumor agent was administered daily until the rat's exhibited symptoms of hand-foot syndrome, at which point treatment experiments commenced. Specific experimental procedures followed those outlined in the prevention experiment. After 5-13 days of topical application, the number of rats exhibiting restored or significantly reduced symptoms compared to pre-treatment was recorded as effective in treating hand-foot syndrome.

Table 4 lists the animal experiments using various anti-tumor agents and NO-NSAID ointment, as well as the corresponding experimental results (where the value in the control rate column = the incidence rate of hand-foot syndrome in the blank group - the incidence rate of hand-foot syndrome in the treatment group).

**Table 4 Experimental conditions and experimental results of Examples 17-26**

| Exampl e | Anti-tumor agent | Dosage | Frequenc y | Treat ment | Days | Concentrat ion wt % | Respons e rate |
|---|---|---|---|---|---|---|---|
| 17 | Sorafenib | 80mg/kg | Once every two days | A1 | 5 | 2.50% | 28.57% |
| | | | | A2 | 5 | 2.00% | 20.00% |
| | | | | A3 | 7 | 3% | 42.86% |
| | | | | A5 | 5 | 2.50% | 50.00% |
| 18 | Lenvatinib | 65mg/kg | Once a day | A1 | 9 | 2.50% | 40.00% |
| | | | | A2 | 9 | 3% | 33.33% |
| | | | | A5 | 12 | 2.50% | 42.86% |
| 19 | Regorafenib | 90mg/kg | Once every two days | A1 | 8 | 0.50% | 14.29% |
| | | | | A2 | 7 | 1% | 33.33% |
| | | | | A3 | 7 | 1% | 33.33% |
| | | | | A5 | 6 | 5% | 57.14% |
| 20 | Sunitinib | 120mg/kg | One e every two days | A1 | 9 | 2.50% | 28.57% |
| | | | | A2 | 7 | 3% | 33.33% |
| | | | | A5 | 7 | 2.50% | 33.33% |
| 21 | Cabozantinib | 50mg/kg | Once every two days | A1 | 11 | 1% | 28.57% |
| | | | | A2 | 13 | 1% | 14.29% |
| | | | | A5 | 11 | 1% | 33.33% |
| | | | | A7 | 13 | 3% | 40.00% |
| 22 | Fruquintinib | 40mg/kg | Once every two days | A1 | 8 | 2.50% | 42.86% |
| | | | | A2 | 8 | 3% | 50.00% |
| | | | | A5 | 8 | 2.50% | 28.57% |
| 23 | Apatinib | 105 mg/kg | One e every two days | A1 | 7 | 2.50% | 28.57% |
| | | | | A2 | 7 | 3% | 33.33% |
| | | | | A5 | 6 | 2.50% | 28.57% |
| 24 | Olmutinib | 100mg/kg | Once every two days | A1 | 12 | 2.50% | 42.86% |
| | | | | A5 | 10 | 2.50% | 50.00% |
| 25 | Erdafitinib | 45mg/kg | Once every two days | A2 | 13 | 3% | 28.57% |
| | | | | A5 | 13 | 2.50% | 40.00% |
| | | | | A7 | 11 | 3.00% | 33.33% |
| 26 | Encorafenib | 80mg/kg | Once every two days | A1 | 9 | 2.50% | 14.29% |
| | | | | A5 | 7 | 2.50% | 40.00% |
| | | | | A6 | 7 | 1% | 16.67% |

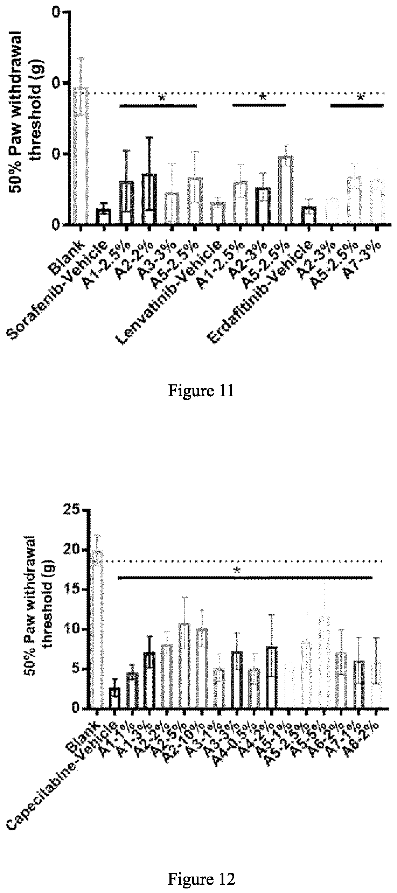

It can be seen from the results that the NO-NSAID of the present application can effectively treat hand-foot syndrome caused by anti-tumor agents. Additionally, pain relief was evaluated for some treatment groups, as shown in Figure 11. The results indicate that NO-NSAID ointment not only treats hand-foot syndrome caused by anti-tumor agents but also provides some pain relief.

### NO-NSAID ointment can prevent and/or treat chemotherapy-induced hand-foot syndrome in rat models

Rat animal model was constructed. Female SD rats were given chemotherapy drugs by gavage every day for 6 weeks (dosage and drugs shown in Table 5). After several days, the rats develop hand-foot syndrome in their paws.

After one week of acclimatization (about 200 g), the SD rats are divided into groups of 10 rats each, followed by gavage administration experiment. The chemotherapy drugs were dissolved, and each rat was given a gavage dose of 1 mL/100 g. The dosage is shown in Table 5. After gavage, the rats were fixed with a fixed cylinder. NO-NSAID ointment was applied to the paws of the rats in the medication group, and blank ointment was applied to the paws of the rats in the blank group (as blank control). Repeat the gavage and application test every day until the blank group exhibits significant hand-foot syndrome. At this point, the number of rats in the treatment group whose paw skin remains normal or shows significantly milder than those of the blank group were calculated as the number of rats effectively prevented from developing hand-foot syndrome.

Table 5 lists the animal experimental combinations of chemotherapy drugs and NO-NSAID ointment, as well as the corresponding experimental results (where the value in the control rate column = the incidence rate of hand-foot syndrome in the blank group - the incidence rate of hand-foot syndrome in the treatment group).

**Table 5 Experimental conditions and experimental results of Examples 27-30**

| Exampl e | Chemotherap y drug | Dosage | Frequenc y | Treatmen t | Concentratio nwt% | Days | Control rate |
|---|---|---|---|---|---|---|---|
| 27 | Capecitabine | 4000mg/kg | Once a day | A1 | 1% | 4 0 | 37.50 % |
| | | | | | 3% | | 44.44 % |
| | | | | A2 | 2% | | 22.22 % |
| | | | | | 5% | | 28.57 % |
| | | | | | 10% | | 33.33 % |
| | | | | A3 | 1% | | 28.57% |
| | | | | | 3% | | 37.50% |
| | | | | A4 | 0.50% | | 25.00 % |
| | | | | | 2% | | 57.14 % |
| | | | | A5 | 1% | | 12.50 % |
| | | | | | 2.50% | | 22.22 % |
| | | | | | 5% | | 50.00 % |
| | | | | A6 | 2% | | 42.86 % |
| | | | | A7 | 1% | | 22.22 % |
| | | | | A8 | 2% | | 25.00 % |
| 28 | Cytarabine | 120mg/kg | Twice a day | A2 | 2% | 30 | 14.29% |
| | | | | | 5% | | 42.86% |
| | | | | A4 | 0.50% | | 28.57% |
| | | | | A5 | 1% | | 14.29% |
| | | | | | 2.50% | | 28.57% |
| | | | | | 5% | | 57.14% |
| 29 | Doxorubicin | 7mg/kg | Once every three days | A1 | 1% | 30 | 50.00% |
| | | | | A5 | 1% | | 37.50% |
| | | | | | 2.50% | | 50.00% |
| 30 | Acelarin | 130mg/kg | Twice a week | A2 | 1% | 30 | 28.57% |
| | | | | A5 | 1% | | 28.57% |
| | | | | | 2.50% | | 57.14% |

It can be seen from the above results that the NO-NSAID ointment of the present application also has a certain preventive effect on chemotherapy-induced hand-foot syndrome, and at the same time, it can significantly ameliorate pain and inflammation (Figure 12).

### Comparison of Experimental Results of Different Mechanism Compounds in Preventing Anti-tumor Agent-Induced Hand-Foot Syndrome

After the rats (about 200 g) were acclimated for one week, they were divided into groups of 10 and then subjected to gavage administration experiments. The anti-tumor agent (the anti-tumor agent modeled by sorafenib) was dissolved in a mixed solution of Cremophor EL: ethanol =1:1, and diluted to the required concentration with PBS (diluted about 3 times with PBS solution) before gavage administration. The gavage volume did not exceed 2 mL each time, and the dosage was shown in Table 6. After gavage, the hind paws of the rats were treated with ointments of different mechanisms (NO-releasing agent group, NO-releasing agent combined with clinical drug group, NSAID group), while the control group was treated with a blank ointment. After the application of the ointment, the rats were placed in a fixed cylinder for 4 hours. After 4 hours, the rats were released, and the residual drugs in the application site were wiped off with clean water, and the rats were returned to the cage. The frequency of gavage administration of the inhibitor is shown in Table 6. Different treatment groups were only applied once a day. Gavage and ointment application were repeated every day until the end of the experiment. After 15-18 days of ointment application, the number of rats in the treatment group that remained normal or showed significantly less severe hand-foot syndrome compared to the blank control group was counted as the number of rats effectively inhibited from developing hand-foot syndrome.

**Table 6 lists the animal experimental combinations of various anti-tumor agents and ointments from different treatment groups, as well as the corresponding experimental results (where the value in the control rate column = the incidence rate of hand-foot syndrome in the blank group - the incidence rate of hand-foot syndrome in the treatment group).**

| Examp le | Anti-tumor agent | Dosage | Freque ncy | Treatment | Concentrati on wt% | Days | Control rate |
|---|---|---|---|---|---|---|---|
| 31 | Sorafenib | 80mg/kg | Once a day | Nitroglycerin | 0.20% | 15 | 60% |
| | | | Once a day | Isosorbide mononitrate/isosorbide dinitrate (1:1) mixed ointment | 0.20% | 15 | 50.00% |
| | | | | Acetylsalicylic acid | 1.00% | 15 | 14.29% |
| | | | | Ibuprofen | 2.00% | 15 | 0% |
| | | | | Naproxen | 2.50% | 15 | 16.67% |
| | | | | Nitroglycerin + Vitamin K1 | 0.2%+0.1% | 15 | 43% |
| | | | | Nitroglycerin + Erythromycin | 0.2%+1% | 15 | 50.00% |

It can be seen from the above results that NSAID ointment is either ineffective or has a very low efficiency in treating hand-foot syndrome caused by anti-tumor agents, and among the rats that showed response, the symptoms were only mildly alleviated. The results of the combination of nitroglycerin and clinical drugs indicate that the NO-releasing agent combined with other clinical drug mechanisms does not significantly improve the effectiveness of nitroglycerin in treating hand-foot syndrome.

In terms of pain scores, it can be observed that NO-NSAID at the same concentration is more effective in alleviating pain compared to NSAID. Additionally, NO-NSAID shows more significant pain relief compared to the combination of nitroglycerin and other drug mechanisms.

### The effect of NO-NSAID on ameliorating the cytotoxicity of anti-tumor agents on HUVEC cell proliferation

The cultured HUVEC cells were digested the suspended, counted, and seed into a 96-well plate with 5,000-10,000 cells per well. The wells are divided into the following groups: blank control group, anti-tumor agent group, anti-tumor agent + NO-NSAID group, anti-tumor agent solvent group, NO-NSAID control group. Wherein each well of each group contained a basic medium, and the final liquid volume contained in each well was about 100 µL. The grouping situation was as follows:
1) Blank control group: no solution was added except normally replacing the medium;
2) Anti-tumor agent group: added the anti-tumor agent solution (the final concentration was shown in Table 7-1 and Table 7-2, the solvent of the anti-tumor agent solution was DMSO);
3) Anti-tumor agent + NO-NSAID group: added the anti-tumor agent solution and the NO-NSAID solution (the final concentrations of anti-tumor agent and NO-NSAID were shown in Table 7-1 and Table 7-2, and according to the solubility of NO-NSAID, the solvent of NO-NSAID solution was selected as ethanol or sterile water, and minor volume differences in each group were adjusted by added the corresponding solvent);
4) Anti-tumor agent solvent group: an equal volume of DMSO as contained in the corresponding anti-tumor agent solution in Group 2) was added;
5) NO-NSAID control group: the same volume and type of solvent (for example, ethanol or sterile water) corresponding to the NO-NSAID contained in group 3) was added.

The anti-tumor agent solvent group was not involved in data processing and was only used as a reference for evaluating experimental system error. The NO-NSAID solvent control group was used for data correction to eliminate the influence of the solvent on the results.

After culturing for 24-48 hours, the Cell Counting Kit-8 (CCK-8) detection kit (C0037, Shanghai Beyotime Biotechnology Co., Ltd., Beyotime Biotechnology) was used to determine the cell viability. This allows for the calculation of the proliferative toxicity of anti-tumor agent on cell and the mitigating effect of NO-NSAID on proliferative toxicity. The results were statistically analyzed and plotted using GraphPad Prism 6.0 software and t test.

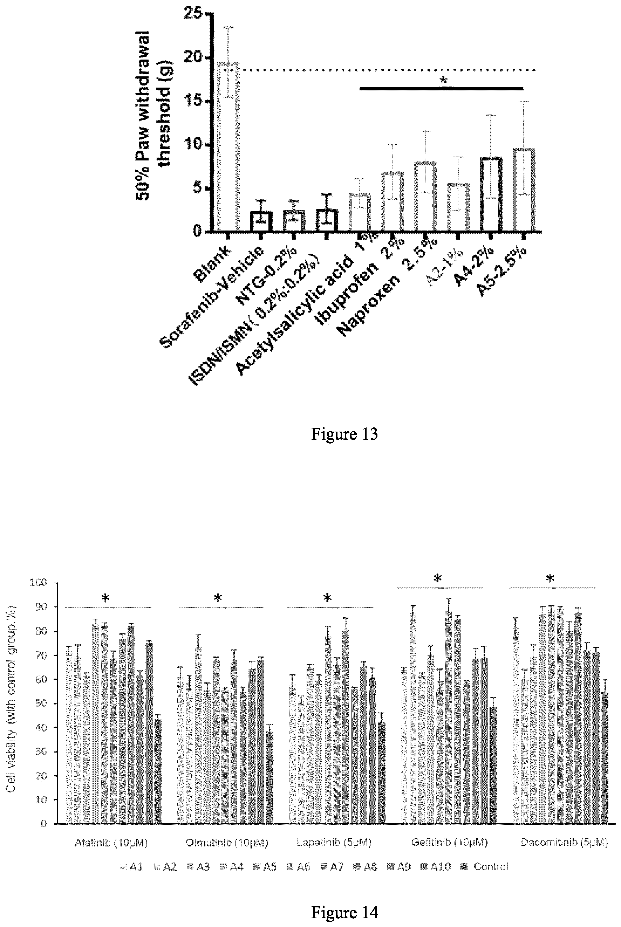

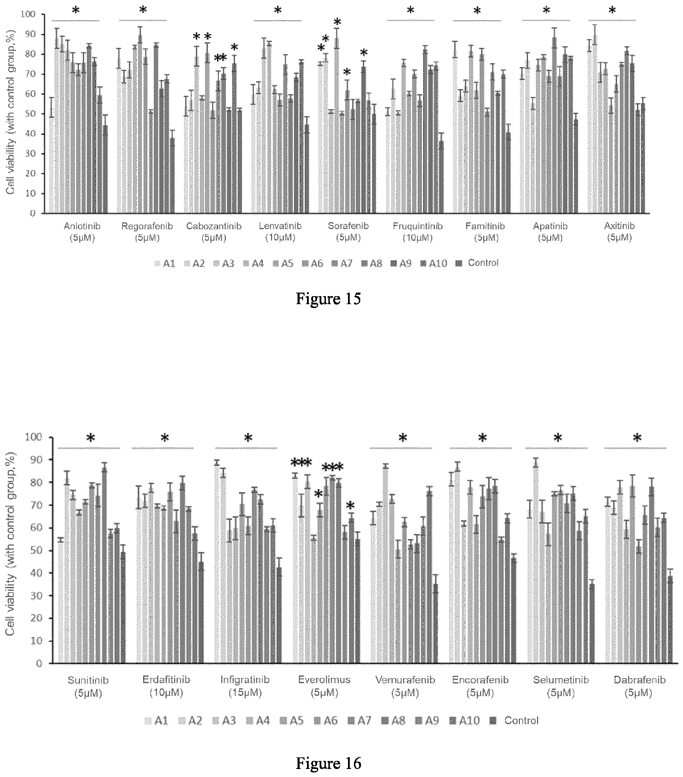

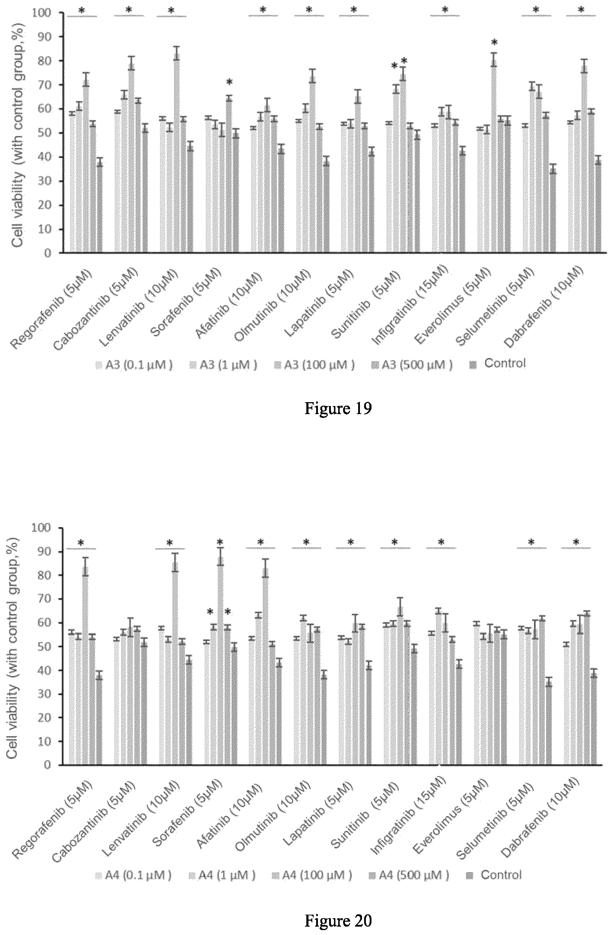

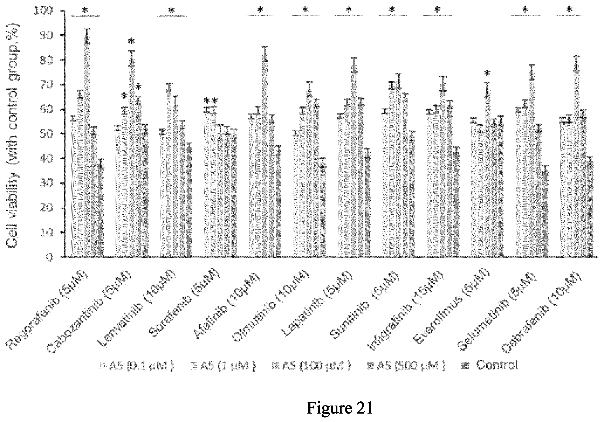

Table 7-1 and Table 7-2 list the various combinations of anti-tumor agents and NO-NSAID, as well as the corresponding experimental results (wherein, the data identification in the cell viability column indicates the percentage increase in viable cells in the anti-tumor agent and NO-NSAID groups compared to the anti-tumor agent alone. Figure 14 - Figure 21 respectively shows exemplary results of cell proliferative toxicity measured by the CCK-8 assay 24 hours after administration of anti-tumor agents and NO-NSAID to HUVEC cells. Among them, the horizontal axis represents different treatment groups and control groups; the vertical axis represents the cell viability (calculated as a percentage of the cell viability in the blank control group set at 100%). * Indicates P<0.05, which is significantly different from the corresponding anti-tumor agent administered alone group; t-test statistical test is used.

**Table 7-1 Experimental conditions and experimental results of Example 32**

| Example | Inhibitor | NO-NSAID | Final concentration | Response rate % (average) |
|---|---|---|---|---|
| 32 | Afatinib (10µM) | A1 | 100 µM | 50 |
| | Olmutinib (10µM) | | | 37 |
| | Lapatinib (5µM) | | | 27 |
| | Gefitinib (10µM) | | | 30 |
| | Dacomitinib (5µM) | | | 59 |
| | Anlotinib (5µM) | | | 16 |
| | Regorafenib (5µM) | | | 64 |
| | Cabozantinib (5µM) | | | 4 |
| | Lenvatinib (10µM) | | | 28 |
| | Sorafenib (5µM) | | | 51 |
| | Fruquintinib (10µM) | | | 23 |
| | Famitinib (5µM) | | | 70 |
| | Apatinib (10µM) | | | 43 |
| | Axitinib (10µM) | | | 65 |
| | Sunitinib (5µM) | | | 11 |
| | Erdafitinib (10µM) | | | 51 |
| | Infigratinib (15µM) | | | 80 |
| | Everolimus (5µM) | | | 62 |
| | Vemurafenib (5µM) | | | 45 |
| | Encorafenib (5µM) | | | 65 |
| | Selumetinib (5µM) | | | 51 |
| | Dabrafenib (10µM) | | | 53 |
| | Afatinib (10µM) | | | 46 |
| | Olmutinib (10µM) | | | 33 |
| | Lapatinib (5µM) | | | 16 |
| | Gefitinib (10µM) | | | 76 |
| 33 | Dacomitinib (5µM) | A2 | 100 µM | 12 |
| | Anlotinib (5µM) | | | 78 |
| | Regorafenib (5µM) | | | 50 |
| | Cabozantinib (5µM) | | | 10 |
| | Lenvatinib (10µM) | | | 34 |
| | Sorafenib (5µM) | | | 56 |
| | Fruquintinib (10µM) | | | 41 |
| | Famitinib (5µM) | | | 31 |
| | Apatinib (10µM) | | | 56 |
| | Axitinib (10µM) | | | 77 |
| | Sunitinib (5µM) | | | 65 |
| | Erdafitinib (10µM) | | | 49 |
| | Infigratinib (15µM) | | | 73 |
| | Everolimus (5µM) | | | 33 |
| | Vemurafenib (5µM) | | | 54 |
| | Encorafenib (5µM) | | | 76 |
| | Selumetinib (5µM) | | | 83 |
| | Dabrafenib (10µM) | | | 49 |
| 34 | Afatinib (10µM) | A3 | 100 µM | 32 |
| | Olmutinib (10µM) | | | 57 |
| | Lapatinib (5µM) | | | 40 |
| | Gefitinib (10µM) | | | 26 |
| | Dacomitinib (5µM) | | | 32 |
| | Anlotinib (5µM) | | | 73 |
| | Regorafenib (5µM) | | | 55 |
| | Cabozantinib (5µM) | | | 56 |
| | Lenvatinib (10µM) | | | 69 |
| | Sorafenib (5µM) | | | 3 |
| | Fruquintinib (10µM) | | | 22 |
| | Famitinib (5µM) | | | 39 |
| | Apatinib (10µM) | | | 15 |
| | Axitinib (10µM) | | | 35 |
| | Sunitinib (5µM) | | | 50 |
| | Erdafitinib (10µM) | | | 59 |
| | Infigratinib (15µM) | | | 28 |
| | Everolimus (5µM) | | | 56 |
| | Vemurafenib (5µM) | | | 80 |
| | Encorafenib (5µM) | | | 28 |
| | Selumetinib (5µM) | | | 49 |
| | Dabrafenib (10µM) | | | 64 |
| 35 | Afatinib (10µM) | A4 | 100 µM | 70 |
| | Olmutinib (10µM) | | | 28 |
| | Lapatinib (5µM) | | | 30 |
| | Gefitinib (10µM) | | | 42 |
| | Dacomitinib (5µM) | | | 71 |
| | Anlotinib (5µM) | | | 67 |
| | Regorafenib (5µM) | | | 74 |
| | Cabozantinib (5µM) | | | 13 |
| | Lenvatinib (10µM) | | | 74 |
| | Sorafenib (5µM) | | | 76 |
| | Fruquintinib (10µM) | | | 62 |
| | Famitinib (5µM) | | | 69 |
| | Apatinib (10µM) | | | 52 |
| | Axitinib (10µM) | | | 39 |
| | Sunitinib (5µM) | | | 34 |
| | Erdafitinib (10µM) | | | 45 |
| | Infigratinib (15µM) | | | 30 |
| | Everolimus (5µM) | | | 1 |
| | Vemurafenib (5µM) | | | 58 |
| | Encorafenib (5µM) | | | 59 |
| | Selumetinib (5µM) | | | 34 |
| | Dabrafenib (10µM) | | | 33 |
| 36 | Afatinib (10µM) | A5 | 100 µM | 69 |
| | Olmutinib (10µM) | | | 48 |
| | Lapatinib (5µM) | | | 62 |
| | Gefitinib (10µM) | | | 21 |
| | Dacomitinib (5µM) | | | 75 |
| | Anlotinib (5µM) | | | 57 |
| | Regorafenib (5µM) | | | 83 |
| | Cabozantinib (5µM) | | | 60 |
| | Lenvatinib (10µM) | | | 32 |
| | Sorafenib (5µM) | | | 1 |
| | Fruquintinib (10µM) | | | 37 |
| | Famitinib (5µM) | | | 35 |
| | Apatinib (10µM) | | | 59 |
| | Axitinib (10µM) | | | -2 |
| | Sunitinib (5µM) | | | 44 |
| | Erdafitinib (10µM) | | | 43 |
| | Infigratinib (15µM) | | | 48 |
| | Everolimus (5µM) | | | 28 |
| | Vemurafenib (5µM) | | | 24 |
| | Encorafenib (5µM) | | | 28 |
| | Selumetinib (5µM) | | | 61 |
| | Dabrafenib (10µM) | | | 65 |
| 37 | Afatinib (10µM) | A6 | 100 µM | 45 |
| | Olmutinib (10µM) | | | 28 |
| | Lapatinib (5µM) | | | 41 |
| | Gefitinib (10µM) | | | 77 |
| | Dacomitinib (5µM) | | | 75 |
| | Anlotinib (5µM) | | | 50 |
| | Regorafenib (5µM) | | | 66 |
| | Cabozantinib (5µM) | | | 0 |
| | Lenvatinib (10µM) | | | 23 |
| | Sorafenib (5µM) | | | 24 |
| | Fruquintinib (10µM) | | | 53 |
| | Famitinib (5µM) | | | 66 |
| | Apatinib (10µM) | | | 41 |
| | Axitinib (10µM) | | | 22 |
| | Sunitinib (5µM) | | | 58 |
| | Erdafitinib (10µM) | | | 56 |
| | Infigratinib (15µM) | | | 32 |
| | Everolimus (5µM) | | | 52 |
| | Vemurafenib (5µM) | | | 42 |
| | Encorafenib (5µM) | | | 51 |
| | Selumetinib (5µM) | | | 64 |
| | Dabrafenib (10µM) | | | 21 |
| 38 | Afatinib (10µM) | A7 | 100 µM | 59 |
| | Olmutinib (10µM) | | | 49 |
| | Lapatinib (5µM) | | | 66 |
| | Gefitinib (10µM) | | | 71 |
| | Dacomitinib (5µM) | | | 56 |
| | Anlotinib (5µM) | | | 56 |
| | Regorafenib (5µM) | | | 21 |
| | Cabozantinib (5µM) | | | 31 |
| | Lenvatinib (10µM) | | | 55 |
| | Sorafenib (5µM) | | | 5 |
| | Fruquintinib (10µM) | | | 32 |
| | Famitinib (5µM) | | | 17 |
| | Apatinib (10µM) | | | 78 |
| | Axitinib (10µM) | | | 44 |
| | Sunitinib (5µM) | | | 49 |
| | Erdafitinib (10µM) | | | 32 |
| | Infigratinib (15µM) | | | 60 |
| | Everolimus (5µM) | | | 60 |
| | Vemurafenib (5µM) | | | 27 |
| | Encorafenib (5µM) | | | 58 |
| | Selumetinib (5µM) | | | 55 |
| | Dabrafenib (10µM) | | | 44 |
| 39 | Afatinib (10µM) | A8 | 100 µM | 69 |
| | Olmutinib (10µM) | | | 27 |
| | Lapatinib (5µM) | | | 23 |
| | Gefitinib (10µM) | | | 19 |
| | Dacomitinib (5µM) | | | 72 |
| | Anlotinib (5µM) | | | 72 |
| | Regorafenib (5µM) | | | 75 |
| | Cabozantinib (5µM) | | | 38 |
| | Lenvatinib (10µM) | | | 24 |
| | Sorafenib (5µM) | | | 14 |
| | Fruquintinib (10µM) | | | 72 |
| | Famitinib (5µM) | | | 51 |
| | Apatinib (10µM) | | | 41 |
| | Axitinib (10µM) | | | 59 |
| | Sunitinib (5µM) | | | 74 |
| | Erdafitinib (10µM) | | | 63 |
| | Infigratinib (15µM) | | | 52 |
| | Everolimus (5µM) | | | 55 |
| | Vemurafenib (5µM) | | | 28 |
| | Encorafenib (5µM) | | | 60 |
| | Selumetinib (5µM) | | | 62 |
| | Dabrafenib (10µM) | | | 64 |
| 40 | Afatinib (10µM) | A9 | 100 µM | 32 |
| | Olmutinib (10µM) | | | 42 |
| | Lapatinib (5µM) | | | 40 |
| | Gefitinib (10µM) | | | 40 |
| | Dacomitinib (5µM) | | | 39 |
| | Anlotinib (5µM) | | | 58 |
| | Regorafenib (5µM) | | | 40 |
| | Cabozantinib (5µM) | | | 0 |
| | Lenvatinib (10µM) | | | 43 |
| | Sorafenib (5µM) | | | 48 |
| | Fruquintinib (10µM) | | | 56 |
| | Famitinib (5µM) | | | 33 |
| | Apatinib (10µM) | | | 62 |
| | Axitinib (10µM) | | | 45 |
| | Sunitinib (5µM) | | | 16 |
| | Erdafitinib (10µM) | | | 42 |
| | Infigratinib (15µM) | | | 29 |
| | Everolimus (5µM) | | | 7 |
| | Vemurafenib (5µM) | | | 39 |
| | Encorafenib (5µM) | | | 15 |
| | Selumetinib (5µM) | | | 36 |
| | Dabrafenib (10µM) | | | 35 |
| | Afatinib (10µM) | | | 56 |
| | Olmutinib (10µM) | | | 48 |
| | Lapatinib (5µM) | | | 32 |
| | Gefitinib (10µM) | | | 40 |
| | Dacomitinib (5µM) | | | 37 |
| | Anlotinib (5µM) | | | 27 |
| | Regorafenib (5µM) | | | 48 |
| | Cabozantinib (5µM) | | | 49 |
| | Lenvatinib (10µM) | | | 57 |
| | Sorafenib (5µM) | | | 13 |
| 41 | Fruquintinib (10µM) | A10 | 100 µM | 59 |
| | Famitinib (5µM) | | | 49 |
| | Apatinib (10µM) | | | 58 |
| | Axitinib (10µM) | | | -7 |
| | Sunitinib (5µM) | | | 21 |
| | Erdafitinib (10µM) | | | 23 |
| | Infigratinib (15µM) | | | 32 |
| | Everolimus (5µM) | | | 21 |
| | Vemurafenib (5µM) | | | 63 |
| | Encorafenib (5µM) | | | 33 |
| | Selumetinib (5µM) | | | 46 |
| | Dabrafenib (10µM) | | | 42 |

From the above results and the results shown in Figures 14 - 16, it can be observed that anti-tumor agents exhibit certain toxicity to HUVEC cells. However, following the addition of NO-NSAID, there is an increase in cell viability, indicating a significant alleviating effect of NO-NSAID on the proliferative toxicity induced by anti-tumor agents.

**Table 7-2 Response rates of different NO-NSAID compound concentrations on anti-tumor agents**

| | | | | | |
|---|---|---|---|---|---|
| | Inhibitor | NO-NSAID response rate % (average) | | | |
| | | A1 (0.1 µM ) | A1 (1 µM ) | A1 (100 µM ) | A1 (500 µM ) |
| | Regorafenib (5µM) | 21 | 27 | 64 | 42 |
| | Cabozantinib (5µM) | 10 | 10 | 4 | 15 |
| | Lenvatinib (10µM) | 21 | 13 | 28 | 10 |
| | Sorafenib (5µM) | 1 | 14 | 51 | 8 |
| 42 | Afatinib (10µM) | 13 | 20 | 50 | 25 |
| | Olmutinib (10µM) | 25 | 20 | 37 | 31 |
| | Lapatinib (5µM) | 27 | 23 | 27 | 24 |
| | Sunitinib (5µM) | 19 | 5 | 11 | 29 |
| | Infigratinib (15µM) | 23 | 14 | 80 | 25 |
| | Everolimus (5µM) | 4 | -2 | 62 | 20 |
| | Selumetinib (5µM) | 36 | 28 | 51 | 25 |
| | Dabrafenib (10µM) | 22 | 48 | 53 | 37 |
| 43 | Inhibitor | NO-NSAID response rate % (average) | | | |
| | | A2 (0.1 µM ) | A2 (1 µM ) | A2 (100 µM ) | A2 (500 µM ) |
| | Regorafenib (5µM) | 32 | 30 | 50 | 36 |
| | Cabozantinib (5µM) | 3 | 4 | 10 | 12 |
| | Lenvatinib (10µM) | 25 | 24 | 34 | 31 |
| | Sorafenib (5µM) | 5 | 20 | 56 | 17 |
| | Afatinib (10µM) | 15 | 17 | 46 | 38 |
| | Olmutinib (10µM) | 24 | 24 | 33 | 23 |
| | Lapatinib (5µM) | 14 | 32 | 16 | 20 |
| | Sunitinib (5µM) | 13 | 7 | 65 | 2 |
| | Infigratinib (15µM) | 30 | 18 | 73 | 17 |
| | Everolimus (5µM) | -11 | -6 | 33 | -9 |
| | Selumetinib (5µM) | 34 | 48 | 83 | 30 |
| | Dabrafenib (10µM) | 23 | 36 | 49 | 30 |
| | Inhibitors | NO-NSAID response rate % (average) | | | |
| | | A3 (0.1 µM ) | A3 (1 µM ) | A3 (100 µM ) | A3 (500 µM ) |
| | Regorafenib (5µM) | 32 | 37 | 55 | 26 |
| | Cabozantinib (5µM) | 14 | 29 | 56 | 24 |
| | Lenvatinib (10µM) | 21 | 14 | 69 | 20 |
| | Sorafenib (5µM) | 13 | 7 | 3 | 29 |
| 44 | Afatinib (10µM) | 15 | 24 | 32 | 22 |
| | Olmutinib (10µM) | 27 | 35 | 57 | 23 |
| | Lapatinib (5µM) | 20 | 20 | 40 | 19 |
| | Sunitinib (5µM) | 10 | 37 | 50 | 7 |
| | Infigratinib (15µM) | 18 | 28 | 28 | 20 |
| | Everolimus (5µM) | -8 | -8 | 56 | 1 |
| | Selumetinib (5µM) | 28 | 53 | 49 | 34 |
| | Dabrafenib (10µM) | 25 | 30 | 64 | 33 |
| 45 | Inhibitor | NO-NSAID response rate % (average) | | | |
| | | A4 (0.1 µM ) | A4 (1 µM ) | A4 (100 µM ) | A4 (500 µM ) |
| | Regorafenib (5µM) | 29 | 26 | 74 | 26 |
| | Cabozantinib (5µM) | 3 | 9 | 13 | 11 |
| | Lenvatinib (10µM) | 24 | 15 | 74 | 14 |
| | Sorafenib (5µM) | 4 | 17 | 76 | 16 |
| | Afatinib (10µM) | 18 | 35 | 70 | 14 |
| | Olmutinib (10µM) | 25 | 38 | 28 | 30 |
| | Lapatinib (5µM) | 20 | 17 | 30 | 28 |
| | Sunitinib (5µM) | 19 | 20 | 34 | 20 |
| | Infigratinib (15µM) | 23 | 39 | 30 | 18 |
| | Everolimus (5µM) | 10 | -2 | 1 | 4 |
| | Selumetinib (5µM) | 35 | 33 | 34 | 41 |
| | Dabrafenib (10µM) | 20 | 34 | 33 | 41 |
| | Inhibitor | NO-NSAID response rate % (average) | | | |
| | | A5 (0.1 µM ) | A5 (1 µM ) | A5 (100 µM ) | A5 (500 µM ) |
| | Regorafenib (5µM) | 29 | 46 | 83 | 22 |
| | Cabozantinib (5µM) | 1 | 15 | 60 | 24 |
| | Lenvatinib (10µM) | 11 | 44 | 32 | 16 |
| | Sorafenib (5µM) | 20 | 20 | 1 | 3 |
| | Afatinib (10µM) | 24 | 28 | 69 | 22 |
| 46 | Olmutinib (10µM) | 19 | 34 | 48 | 39 |
| | Lapatinib (5µM) | 26 | 35 | 62 | 36 |
| | Sunitinib (5µM) | 20 | 40 | 44 | 31 |
| | Infigratinib (15µM) | 28 | 30 | 48 | 34 |
| | Everolimus (5µM) | 0 | -7 | 28 | -2 |
| | Selumetinib (5µM) | 38 | 42 | 61 | 26 |
| | Dabrafenib (10µM) | 27 | 28 | 65 | 31 |

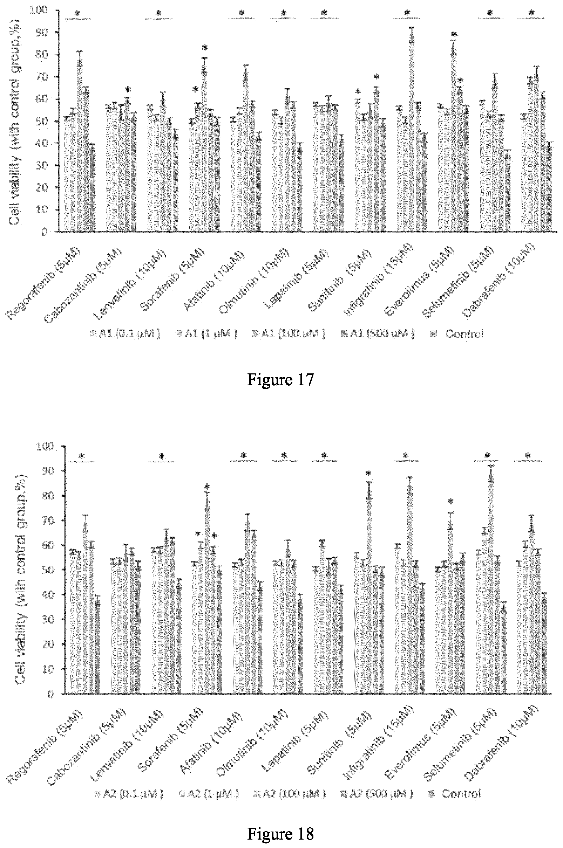

From Table 7-2 and the results shown in Figures 17-21, it is evident that the same NO-NSAID at various concentrations can alleviate the proliferative toxicity of anti-tumor agents on HUVEC cells to varying degrees.

### The effect of NO-NSAID on alleviating the proliferative toxicity of anti-tumor agents on human immortalized epidermal keratinocytes (HaCaT), human oral mucosal epithelial keratinocytes (HOK), human intestinal epithelial cells (FHs 74 Int), and gastric epithelial cells (GES-1)

Experiments were conducted using various epithelial cells: Examples 47-51 used human immortalized keratinocytes (HaCaT) with corresponding results in Table 8-1; Examples 52-56 used human oral keratinocytes (HOK) with results in Table 8-2; Examples 57-61 used gastric epithelial cells (GES-1) with results in Table 8-3; Examples 62-66 used human intestinal epithelial cells (FHs 74 Int) with results in Table 8-4.

The cultured cells were digested the suspended, counted, and seed into a 96-well plate with 5,000-10,000 cells per well. The wells are divided into the following groups: blank control group, anti-tumor agent group, anti-tumor agent + NO-NSAID group, anti-tumor agent solvent group, NO-NSAID solvent control group. Wherein each well of each group contained a basic medium, and the final liquid volume contained in each well was about 100 µL. The grouping situation was as follows:
1) Blank control group: no solution was added except normally replacing the medium;
2) Anti-tumor agent group: added the anti-tumor agent solution (the final concentration was shown in Table 8-1 to Table 8-4, the solvent of the anti-tumor agent solution was DMSO);
3) Anti-tumor agent + NO-NSAID group: added the anti-tumor agent solution and the NO-NSAID solution (the final concentrations of anti-tumor agent and NO-NSAID were shown in Table 8-1 to Table 8-4, and according to the solubility of NO-NSAID, the solvent of NO-NSAID solution was selected as ethanol or sterile water, and minor volume differences in each group were adjusted by added the corresponding solvent);
4) Anti-tumor agent solvent group: an equal volume of DMSO as contained in the corresponding anti-tumor agent solution in Group 2) was added;
5) NO-NSAID control group: the same volume and type of solvent (for example, ethanol or sterile water) corresponding to the NO-NSAID contained in group 3) was added.

The anti-tumor agent solvent group was not involved in data processing and was only used as a reference for evaluating experimental system error. The NO-NSAID solvent control group was used for data correction to eliminate the influence of the solvent on the results.

After culturing for 24-48 hours, the Cell Counting Kit-8 (CCK-8) detection kit (C0037, Shanghai Beyotime Biotechnology Co., Ltd., Beyotime Biotechnology) was used to determine the cell viability. This allows for the calculation of the proliferative toxicity of anti-tumor agent on cell and the mitigating effect of NO-NSAID on proliferative toxicity. The results were statistically analyzed and plotted using GraphPad Prism 6.0 software and t test.

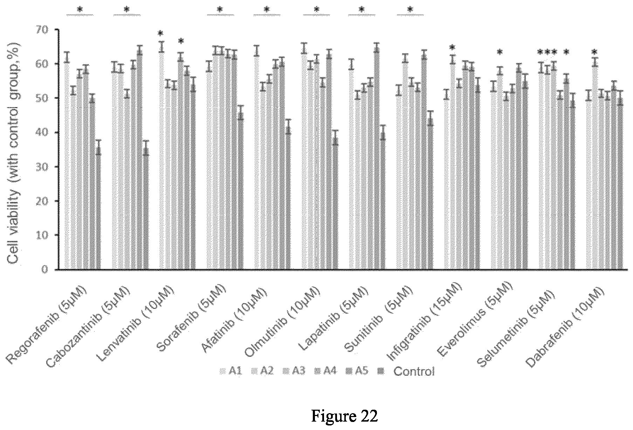

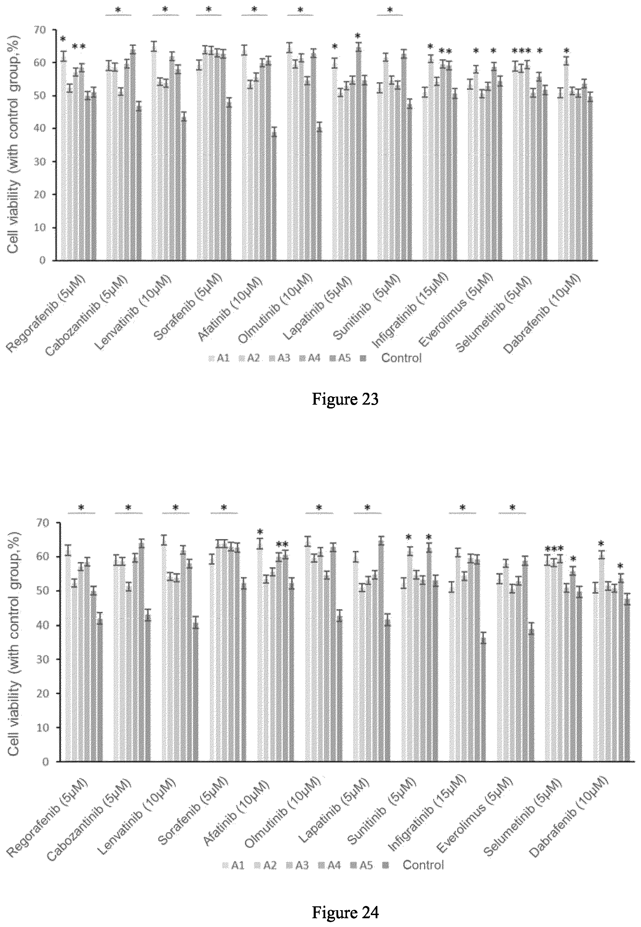

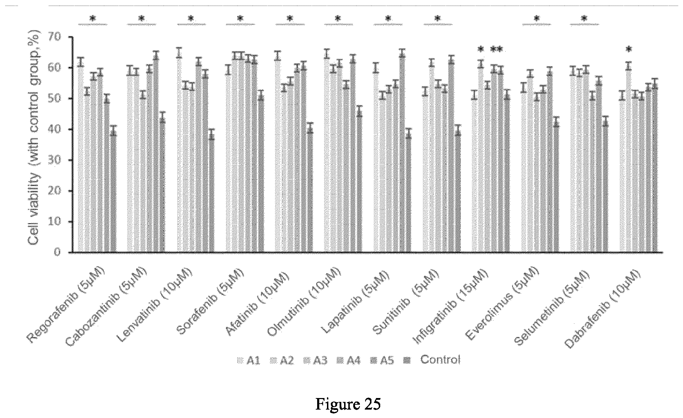

Table 8-1 to Table 8-4 list the various combinations of anti-tumor agents and NO-NSAID, as well as the corresponding experimental results (wherein, the data identification in the cell viability column indicates the percentage increase in viable cells in the anti-tumor agent and NO-NSAID groups compared to the anti-tumor agent alone. Figure 22 represents the experimental results on HaCaT cells, Figure 23 represents the experimental results on HOK cells, Figure 24 represents the experimental results on GES-1 cells, and Figure 25 represents the experimental results on FHs 74 Int cells. Among them, the horizontal axis represents different treatment groups and control groups; the vertical axis represents the cell viability (calculated as a percentage of the cell viability in the blank control group set at 100%). * Indicates P<0.05, which is significantly different from the corresponding anti-tumor agent administered alone group; t-test statistical test is used.

**Table 8-1 Experimental conditions and experimental results of Examples 47-51**

| | Inhibitor | NO-NSAID | Final concentration | Response rate % (average) |
|---|---|---|---|---|
| 47 | Regorafenib (5µM) | A1 | 100 µM | 41 |
| | Cabozantinib (5µM) | | | 36 |
| | Lenvatinib (10µM) | | | 24 |
| | Sorafenib (5µM) | | | 25 |
| | Afatinib (10µM) | | | 38 |
| | Olmutinib (10µM) | | | 42 |
| | Lapatinib (5µM) | | | 33 |
| | Sunitinib (5µM) | | | 15 |
| | Infigratinib (15µM) | | | -6 |
| | Everolimus (5µM) | | | -3 |
| | Selumetinib (5µM) | | | 19 |
| | Dabrafenib (10µM) | | | 2 |
| 48 | Regorafenib (5µM) | A2 | 100 µM | 26 |
| | Cabozantinib (5µM) | | | 36 |
| | Lenvatinib (10µM) | | | 1 |
| | Sorafenib (5µM) | | | 33 |
| | Afatinib (10µM) | | | 20 |
| | Olmutinib (10µM) | | | 34 |
| | Lapatinib (5µM) | | | 18 |
| | Sunitinib (5µM) | | | 31 |
| | Infigratinib (15µM) | | | 16 |
| | Everolimus (5µM) | | | 7 |
| | Selumetinib (5µM) | | | 18 |
| | Dabrafenib (10µM) | | | 21 |
| 49 | Regorafenib (5µM) | A3 | 100 µM | 33 |
| | Cabozantinib (5 µM) | | | 24 |
| | Lenvatinib (10µM) | | | 0 |
| | Sorafenib (5µM) | | | 33 |
| | Afatinib (10µM) | | | 24 |
| | Olmutinib (10µM) | | | 37 |
| | Lapatinib (5µM) | | | 22 |
| | Sunitinib (5µM) | | | 19 |
| | Infigratinib (15µM) | | | 1 |
| | Everolimus (5µM) | | | -10 |
| | Selumetinib (5µM) | | | 20 |
| | Dabrafenib (10µM) | | | 3 |
| | Regorafenib (5µM) | A4 | 100 µM | 36 |
| | Cabozantinib (5µM) | | | 37 |
| | Lenvatinib (10µM) | | | 17 |
| | Sorafenib (5µM) | | | 32 |
| | Afatinib (10µM) | | | 31 |
| 50 | Olmutinib (10µM) | | | 26 |
| | Lapatinib (5µM) | | | 25 |
| | Sunitinib (5µM) | | | 16 |
| | Infigratinib (15µM) | | | 12 |
| | Everolimus (5µM) | | | -4 |
| | Selumetinib (5µM) | | | 3 |
| | Dabrafenib (10µM) | | | 1 |
| 51 | Regorafenib (5µM) | A5 | 100 µM | 22 |
| | Cabozantinib (5µM) | | | 44 |
| | Lenvatinib (10µM) | | | 9 |
| | Sorafenib (5µM) | | | 31 |
| | Afatinib (10µM) | | | 32 |
| | Olmutinib (10µM) | | | 40 |
| | Lapatinib (5µM) | | | 41 |
| | Sunitinib (5µM) | | | 33 |
| | Infigratinib (15µM) | | | 12 |
| | Everolimus (5µM) | | | 9 |
| | Selumetinib (5µM) | | | 13 |
| | Dabrafenib (10µM) | | | 7 |

From the results in Table 8-1 and Figure 22, it can be observed that the anti-tumor agent exhibits proliferative toxicity to skin cells (HaCaT), whereas NO-NSAID significantly alleviates the proliferative toxicity caused by the anti-tumor agent.

**Table 8-2 Experimental conditions and experimental results of Examples 52-56**

| | Inhibitor | NO-NSAID | Final concentration | Response rate % (average) |
|---|---|---|---|---|
| | Regorafenib (5µM) | | | 22 |
| | Cabozantinib (5µM) | | | 23 |
| | Lenvatinib (10µM) | | | 38 |
| | Sorafenib (5µM) | | | 22 |
| | Afatinib (10µM) | | | 41 |
| 52 | Olmutinib (10µM) | A1 | 100 µM | 40 |
| | Lapatinib (5µM) | | | 12 |
| | Sunitinib (5µM) | | | 9 |
| | Infigratinib (15µM) | | | 1 |
| | Everolimus (5µM) | | | -2 |
| | Selumetinib (5µM) | | | 15 |
| | Dabrafenib (10µM) | | | 2 |
| 53 | Regorafenib (5µM) | A2 | 100 µM | 3 |
| | Cabozantinib (5µM) | | | 22 |
| | Lenvatinib (10µM) | | | 19 |
| | Sorafenib (5µM) | | | 31 |
| | Afatinib (10µM) | | | 24 |
| | Olmutinib (10µM) | | | 32 |
| | Lapatinib (5µM) | | | -8 |
| | Sunitinib (5µM) | | | 27 |
| | Infigratinib (15µM) | | | 21 |
| | Everolimus (5µM) | | | 8 |
| | Selumetinib (5µM) | | | 13 |
| | Dabrafenib (10µM) | | | 22 |
| | Regorafenib (5µM) | | | 12 |
| | Cabozantinib (5µM) | | | 8 |
| | Lenvatinib (10µM) | | | 18 |
| | Sorafenib (5µM) | | | 30 |
| | Afatinib (10µM) | | | 27 |
| | Olmutinib (10µM) | | | 35 |
| 54 | Lapatinib (5µM) | A3 | 100 µM | -4 |
| | Sunitinib (5µM) | | | 14 |
| | Infigratinib (15µM) | | | 7 |
| | Everolimus (5µM) | | | -8 |
| | Selumetinib (5µM) | | | 16 |
| | Dabrafenib (10µM) | | | 4 |
| 55 | Regorafenib (5µM) | A4 | 100 µM | 15 |
| | Cabozantinib (5µM) | | | 24 |
| | Lenvatinib (10µM) | | | 33 |
| | Sorafenib (5µM) | | | 29 |
| | Afatinib (10µM) | | | 34 |
| | Olmutinib (10µM) | | | 24 |
| | Lapatinib (5µM) | | | 0 |
| | Sunitinib (5µM) | | | 11 |
| | Infigratinib (15µM) | | | 18 |
| | Everolimus (5µM) | | | -3 |
| | Selumetinib (5µM) | | | -2 |
| | Dabrafenib (10µM) | | | 2 |
| | Regorafenib (5µM) | | | -2 |
| | Cabozantinib (5µM) | | | 32 |
| | Lenvatinib (10µM) | | | 25 |
| | Sorafenib (5µM) | | | 28 |
| | Afatinib (10µM) | | | 36 |
| 56 | Olmutinib (10µM) | A5 | 100 µM | 38 |
| | Lapatinib (5µM) | | | 22 |
| | Sunitinib (5µM) | | | 29 |
| | Infigratinib (15µM) | | | 17 |
| | Everolimus (5µM) | | | 10 |
| | Selumetinib (5µM) | | | 8 |
| | Dabrafenib (10µM) | | | 8 |

From the results in Table 8-2 and Figure 23, it can be observed that the anti-tumor agent exhibits proliferative toxicity to human oral mucosal epithelial keratinocytes (HOK), whereas NO-NSAID significantly alleviates the proliferative toxicity caused by the anti-tumor agent.

**Table 8-3 Experimental conditions and experimental results of Examples 57-61**

| | Inhibitor | NO-NSAID | Final concentration | Response rate % (average) |
|---|---|---|---|---|
| | Regorafenib (5µM) | | | 34 |
| | Cabozantinib (5µM) | | | 28 |
| | Lenvatinib (10µM) | | | 41 |
| | Sorafenib (5µM) | | | 15 |
| | Afatinib (10µM) | | | 24 |
| 57 | Olmutinib (10µM) | A1 | 100 µM | 38 |
| | Lapatinib (5µM) | | | 31 |
| | Sunitinib (5µM) | | | -2 |
| | Infigratinib (15µM) | | | 23 |
| | Everolimus (5µM) | | | 24 |
| | Selumetinib (5µM) | | | 18 |
| | Dabrafenib (10µM) | | | 6 |
| | Regorafenib (5µM) | | | 18 |
| | Cabozantinib (5µM) | | | 27 |
| | Lenvatinib (10µM) | | | 23 |
| | Sorafenib (5µM) | | | 24 |
| | Afatinib (10µM) | | | 3 |
| 58 | Olmutinib (10µM) | A2 | 100 µM | 30 |
| | Lapatinib (5µM) | | | 16 |
| | Sunitinib (5µM) | | | 18 |
| | Infigratinib (15µM) | | | 39 |
| | Everolimus (5µM) | | | 31 |
| | Selumetinib (5µM) | | | 17 |
| | Dabrafenib (10µM) | | | 25 |
| 59 | Regorafenib (5µM) | A3 | 100 µM | 26 |
| | Cabozantinib (5µM) | | | 14 |
| | Lenvatinib (10µM) | | | 22 |
| | Sorafenib (5µM) | | | 24 |
| | Afatinib (10µM) | | | 7 |
| | Olmutinib (10µM) | | | 33 |
| | Lapatinib (5µM) | | | 20 |
| | Sunitinib (5µM) | | | 4 |
| | Infigratinib (15µM) | | | 28 |
| | Everolimus (5µM) | | | 19 |
| | Selumetinib (5µM) | | | 19 |
| | Dabrafenib (10µM) | | | 7 |
| | Regorafenib (5µM) | | | 29 |
| | Cabozantinib (5µM) | | | 29 |
| | Lenvatinib (10µM) | | | 36 |
| | Sorafenib (5µM) | | | 22 |
| | Afatinib (10µM) | | | 16 |
| | Olmutinib (10µM) | | | 21 |
| 60 | Lapatinib (5µM) | A4 | 100 µM | 22 |
| | Sunitinib (5µM) | | | 0 |
| | Infigratinib (15µM) | | | 36 |
| | Everolimus (5µM) | | | 23 |
| | Selumetinib (5µM) | | | 2 |
| | Dabrafenib (10µM) | | | 6 |
| 61 | Regorafenib (5µM) | A5 | 100 µM | 14 |
| | Cabozantinib (5µM) | | | 37 |
| | Lenvatinib (10µM) | | | 29 |
| | Sorafenib (5µM) | | | 22 |
| | Afatinib (10µM) | | | 18 |
| | Olmutinib (10µM) | | | 35 |
| | Lapatinib (5µM) | | | 40 |
| | Sunitinib (5µM) | | | 21 |
| | Infigratinib (15µM) | | | 36 |
| | Everolimus (5µM) | | | 33 |
| | Selumetinib (5µM) | | | 12 |
| | Dabrafenib (10µM) | | | 12 |

From the results in Table 8-3 and Figure 24, it can be observed that the anti-tumor agent exhibits proliferative toxicity to human small intestinal epithelial cells (FHs 74 Int), whereas NO-NSAID significantly alleviates the proliferative toxicity caused by the anti-tumor agent.

**Table 8-4 Experimental conditions and experimental results of Examples 62-66**

| | Inhibitor | NO-NSAID | Final concentration | Response rate % (average) |
|---|---|---|---|---|
| | Regorafenib (5µM) | | | 37 |
| | Cabozantinib (5µM) | | | 27 |
| | Lenvatinib (10µM) | | | 43 |
| | Sorafenib (5µM) | | | 17 |
| | Afatinib (10µM) | | | 39 |
| 62 | Olmutinib (10µM) | A1 | 100 µM | 34 |
| | Lapatinib (5µM) | | | 35 |
| | Sunitinib (5µM) | | | 21 |
| | Infigratinib (15µM) | | | 0 |
| | Everolimus (5µM) | | | 19 |
| | Selumetinib (5µM) | | | 29 |
| | Dabrafenib (10µM) | | | -9 |
| 63 | Regorafenib (5µM) | A2 | 100 µM | 21 |
| | Cabozantinib (5µM) | | | 26 |
| | Lenvatinib (10µM) | | | 26 |
| | Sorafenib (5µM) | | | 26 |
| | Afatinib (10µM) | | | 22 |
| | Olmutinib (10µM) | | | 25 |
| | Lapatinib (5µM) | | | 20 |
| | Sunitinib (5µM) | | | 36 |
| | Infigratinib (15µM) | | | 20 |
| | Everolimus (5µM) | | | 27 |
| | Selumetinib (5µM) | | | 27 |
| | Dabrafenib (10µM) | | | 13 |
| 64 | Regorafenib (5µM) | A3 | 100 µM | 29 |
| | Cabozantinib (5µM) | | | 13 |
| | Lenvatinib (10µM) | | | 25 |
| | Sorafenib (5µM) | | | 26 |
| | Afatinib (10µM) | | | 26 |
| | Olmutinib (10µM) | | | 29 |
| | Lapatinib (5µM) | | | 23 |
| | Sunitinib (5µM) | | | 25 |
| | Infigratinib (15µM) | | | 6 |
| | Everolimus (5µM) | | | 14 |
| | Selumetinib (5µM) | | | 29 |
| | Dabrafenib (10µM) | | | -8 |
| 65 | Regorafenib (5µM) | A4 | 100 µM | 31 |
| | Cabozantinib (5µM) | | | 28 |
| | Lenvatinib (10µM) | | | 38 |
| | Sorafenib (5µM) | | | 24 |
| | Afatinib (10µM) | | | 33 |
| | Olmutinib (10µM) | | | 16 |
| | Lapatinib (5µM) | | | 26 |
| | Sunitinib (5µM) | | | 23 |
| | Infigratinib (15µM) | | | 17 |
| | Everolimus (5µM) | | | 18 |
| | Selumetinib (5µM) | | | 14 |
| | Dabrafenib (10µM) | | | -9 |
| | Regorafenib (5µM) | | | 17 |
| | Cabozantinib (5µM) | | | 36 |
| | Lenvatinib (10µM) | | | 32 |
| | Sorafenib (5µM) | | | 24 |
| | Afatinib (10µM) | | | 34 |
| 66 | Olmutinib (10µM) | A5 | 100 µM | 31 |
| | Lapatinib (5µM) | | | 43 |
| | Sunitinib (5µM) | | | 38 |
| | Infigratinib (15µM) | | | 16 |
| | Everolimus (5µM) | | | 28 |
| | Selumetinib (5µM) | | | 23 |
| | Dabrafenib (10µM) | | | -3 |

From the results in Table 8-4 and Figure 25 it can be observed that the anti-tumor agent exhibits proliferative toxicity to human gastric epithelial cells (GES-1), whereas NO-NSAID significantly alleviates the proliferative toxicity caused by the anti-tumor agent.

## Claims

1. A use of a NO-NSAID compound or a pharmaceutically acceptable salt thereof in preparing a medicament, said medicament is used for preventing, ameliorating and/or treating a disease or disorder in a subject associated with administration of an anti-tumor agent.

2. The use of claim 1, wherein said NO-NSAID compound comprises a nitric oxide (NO) releasing moiety and a non-steroidal anti-inflammatory agent (NSAID) moiety, and the NO-releasing moiety is covalently directly linked to the NSAID moiety or linked through a spacer.

3. The use of claim 2, wherein said NSAID moiety comprises a cyclooxygenase (COX) inhibitory moiety.

4. The use of claim 3, wherein said COX inhibitory moiety comprises a moiety that reduces the expression of COX, and/or a moiety that decreases the activity of COX.

5. The use of any one of claims 3-4, wherein said COX inhibitory moiety acts directly on a COX protein and/or a nucleic acid encoding a COX protein.

6. The use of any one of claims 3-5, wherein said COX inhibitory moiety is capable of inhibiting cyclooxygenase-1 (COX-1) and/or cyclooxygenase-2 (COX-2).

7. The use of any one of claims 3-6, wherein said COX inhibitory moiety is capable of selectively inhibiting COX-2.

8. The use of any one of claims 3-6, wherein said COX inhibitory moiety is capable of non-selectively inhibiting COX-1 and COX-2.

9. The use of any one of claims 3-8, wherein said COX inhibitory moiety comprises one or more molecules selected from the group consisting of, its prodrug, its active derivative and/or its active metabolite: acetaminophen, acemetacin, aceclofenac, alminoprofen, amfenac, bendazac, benoxaprofen, bromfenac , bucloxic acid, butibufen, carprofen, cinmetacin, clopirac, diclofenac, etodolac , felbinac, fenclozic acid, fenbufen, fenoprofen, fentiazac, flunoxaprofen, flurbiprofen , ibufenac, ibuprofen, indomethacin, isofezolac, isoxepac, indoprofen, ketoprofen, lonazolac, loxoprofen, metiazinic acid, mofezolac, miroprofen, naproxen, oxaprozin, pirozolac, pranoprofen, protizinic acid, salicylamide, sulindac , suprofen, suxibuzone, tiaprofenic acid, tolmetin, xenbucin, ximoprofen, zaltoprofen, zomepirac, aspirin, acemetcin, bumadizon, carprofenac, clidanac, diflunisal, enfenamic acid, fendosal, flufenamic acid, flunixin, gentisic acid, ketorolac, meclofenamic acid, mefenamic acid acid and mesalamine.

10. The use of any one of claims 3-9, wherein said COX inhibitory moiety comprises one or more molecules selected from the group consisting of its prodrug, its active derivative and/or its active metabolite: naproxen, aspirin, diclofenac, ketoprofen, flurbiprofen and ibuprofen.

11. The use of any one of claims 2-10, wherein said NO releasing moiety is capable of generating at least one of NO⁺, NO⁻, NzO, NO, N₂O₃, NOz, NO₃⁻ and NO₂⁻.

12. The use of any one of claims 2-11, wherein said NO releasing moiety is capable of generating NO directly or indirectly.

13. The use of any one of claims 2-12, wherein said NO releasing moiety comprises organic molecules, inorganic molecules and/or polymers or nanomaterials.

14. The use of any one of claims 2-13, wherein said NO releasing moiety comprises an component selected from the group consisting of: nitroglycerin, isosorbide mononitrate, butylene glycol mononitrate, pentaerythritol tetranitrate, isosorbide dinitrate, trinitroethanolamine, nicorandil, propatylnitrate, 5-amino-3-(4-morpholino)-1,2,3-oxadiazole, isoamyl nitrite , 3,3-bis(aminoethyl)-1-hydroxy-2-carbonyl-1-triazene (NOC-18), Sulfo NONOate disodium salt, S-Nitrosoglutathione, S-Nitroso-N-acetylpenicillamine, 4-Phenyl-3-furoxancarbonitrile, (±)-(E)-4-Ethyl-2-[(E)-hydroxyimino]-5-nitro-3-hexenamide, Streptozocin, NG-Hydroxy-L-arginine acetate salt, Oz -(2,4 -Dinitrophenyl) 1-[(4-ethoxycarbonyl)piperazin-1-yl]diazen-1-ium-1,2-diolate, N-nitrosodibutylamine, 3-morpholinosydnonimine (SIN-1), Linsidomine, Molsidomine, 3-(4-acetylphenyl)sydnone, Diethylamine NONOate/AM and Itramin.

15. The use of any one of claims 2-14, wherein said NO releasing moiety comprises a component selected from the group consisting of: nitroxyl complex, metal nitrosyl complex, metal nitrosamine complex, nitrates, and nitrites.

16. The use of any one of claims 2-15, wherein said NO releasing moiety comprises a component selected from the group consisting of S-nitrosothiol nanosilica spheres, S-nitrosethanedithiol chitosan and oligopropylenediamine-grafted NONOate.

17. The use of any one of claims 2-16, wherein said NO releasing moiety has a molecular weight that is less than or equal to 2000 Daltons, less than or equal to 1500 Daltons, less than or equal to 1200 Daltons, less than or equal to 1000 Daltons, less than or equal to 900 Daltons, less than or equal to 800 Daltons, less than or equal to 700 Daltons, less than or equal to 600 Daltons, less than or equal to 500 Daltons, less than or equal to 400 Daltons, less than or equal to 300 Daltons, less than or equal to 200 Daltons, and/or less than or equal to 100 Daltons.

18. The use of any one of claims 2-17, wherein said NO releasing moiety has one or more of the following groups: diazeniumdiolates, hydroxydiazenesulfonic acid, S-nitrosothiol, furoxans, oxime, N-nitrosamine, N-hydroxyguanidine, nitrate, nitrite, nitrate ester, nitrite ester, sydnonimines, sydnones, oxtriazole- 5-imine, oxtriazol-5-one, hydroxylamine, dioxadiazetidine, N-hydroxynitrosamine, N- nitrosoimine, hydroxyurea and metal nitrosamino complexes.

19. The use of any one of claims 2-18, wherein said NO releasing moiety comprises -NO₂ or -ONO₂.

20. The use of any one of claims 2-19, wherein in the NO-NSAID compound, said NO releasing moiety is linked to said NSAID moiety through a cleavable linker.

21. The use of any one of claims 2-20, wherein in the NO-NSAID compound, said NO releasing moiety is linked to said NSAID moiety through a linker comprising an ester bond and/or a disulfide bond.

22. The use of any one of claims 2-21, wherein in said NO-NSAID compound, a spacer is included between the NO releasing moiety and the NSAID moiety.

23. The use of claim 22, wherein said spacer comprises: one or more optionally substituted -CH ₂ -, optionally substituted phenyl and/or a combination thereof.

24. The use of any one of claims 1-23, wherein said NO-NSAID compound further comprises a H₂S releasing moiety.

25. The use of any one of claims 1-24, wherein said NO-NSAID compound comprises a structure represented by formula (3):
M-X-Y-ONO₂ (3),
wherein M is a structure selected from the following group: 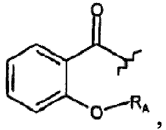 wherein R_{A} is a hydrogen atom or -C(O)CH₃; 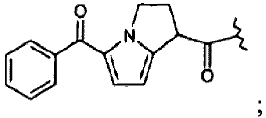 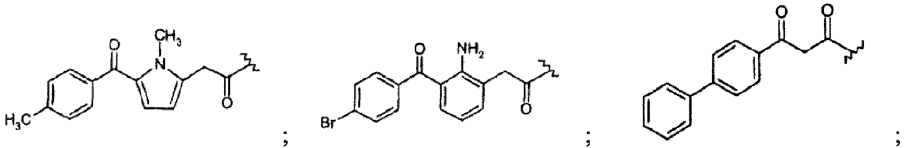 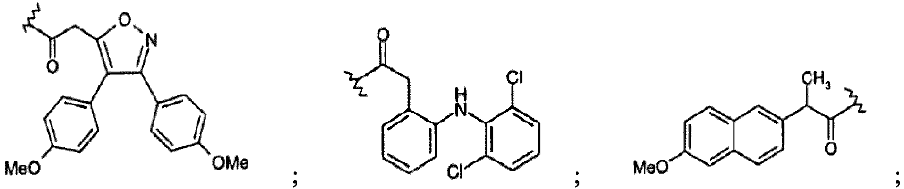 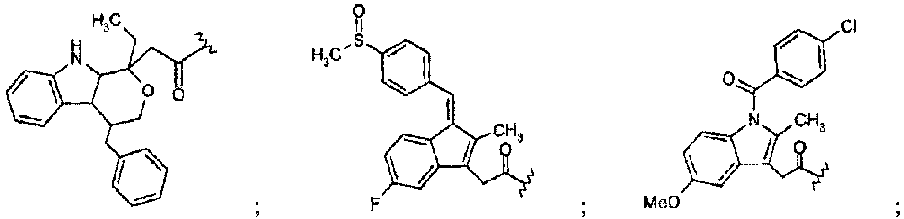 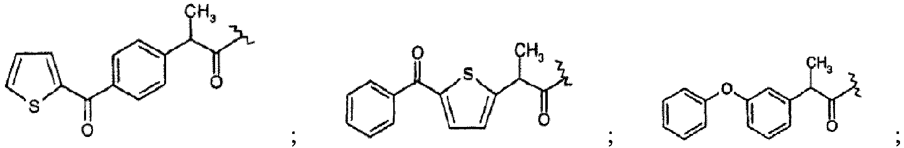 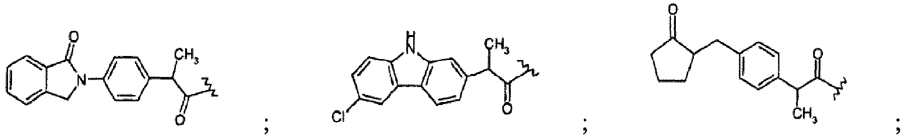 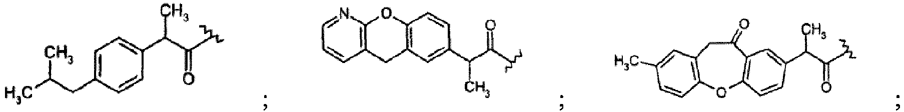 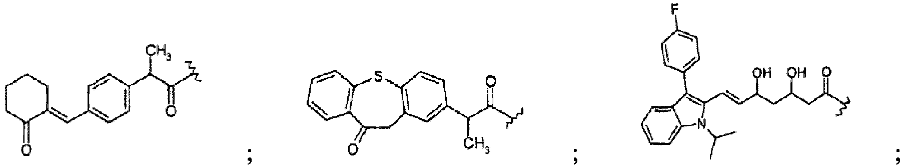 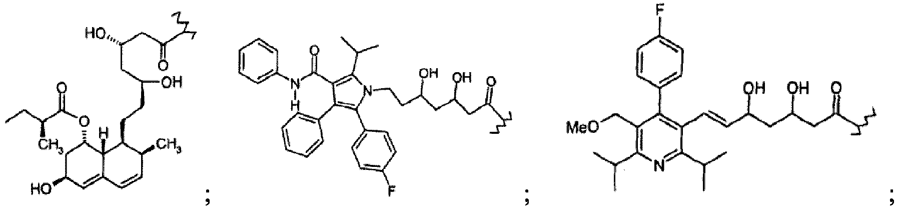 and 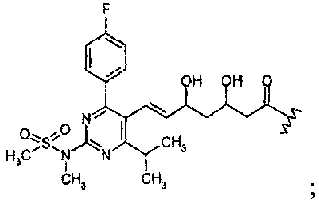
X is -O-, -S- or -NR ¹ -, wherein R¹ is H or a linear or branched C₁-C₆ alkyl group;
Y is a divalent group with the following definition:
a)
- Linear or branched C₁-C₂₀ alkylene, optionally substituted by one or more substituents selected from the group consisting of: halogen, hydroxyl, -ONOz, -OC(O)(C₁ -C₁₀ alkyl)-ONOz and - O(C₁-C₁₀ alkyl)-ONO₂;
- C₅ -C₇ cycloalkylene, optionally substituted by linear or branched C ₁ -C ₁₀ alkyl;
b) 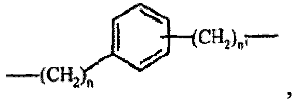 wherein n is an integer selected from 0-20, n' is an integer selected from 1-20;
c) 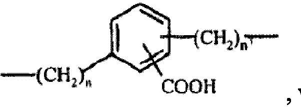 wherein n is an integer selected from 0-20, n' is an integer from 1-20;
d) 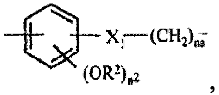 wherein X₁ is -OCO- or -COO-, and R² is H or CH₃, na is an integer from 1-20, and n² is an integer from 0-2;
e) 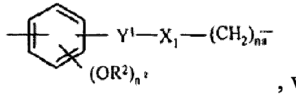 wherein Y ¹ is -CH₂-CH₂-(CH₂)n²-, or -CH=CH-(CH₂)n²-, X₁, na, n² and R² are as defined above;
f) 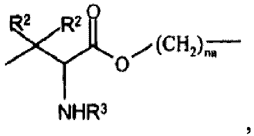 wherein na and R² are as defined above, and R³ is H or -COCH₃; or
g) 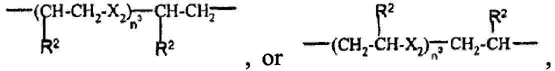 wherein X₂ is -O- or -S-, n³ is an integer from 1-6, and R ² is defined as above.

26. The use of any one of claims 1-24, wherein said NO-NSAID compound comprises a structure represented by formula (3):
M-X-Y-ONO₂ (3),
wherein M is selected from the following structures: 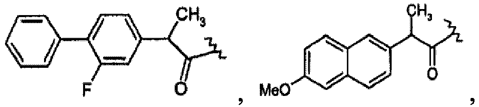 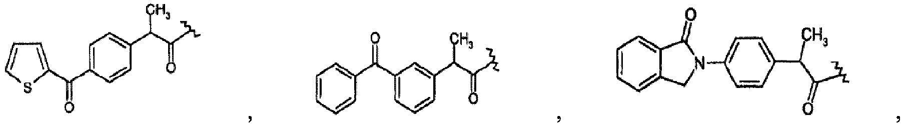 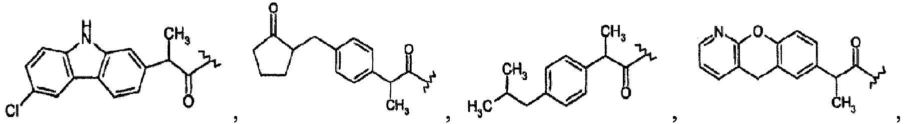 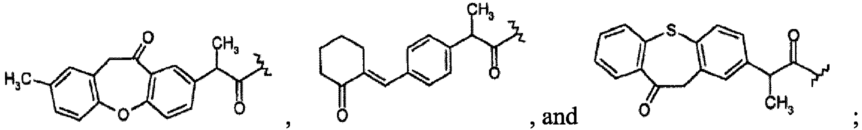
X is -O-; Y is a linear or branched C₁ -C₁₀ alkylene group.

27. The use of any one of claims 1-24, wherein said NO-NSAID compound comprises a structure represented by formula (21): 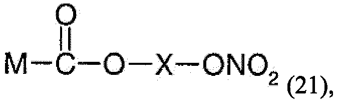 wherein
X is a linker and M is a structure selected from the following group: 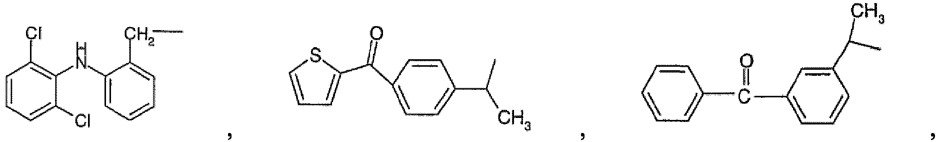 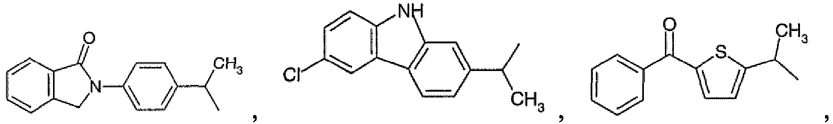 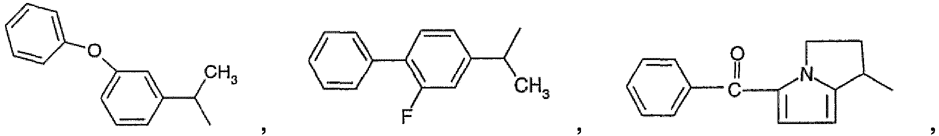 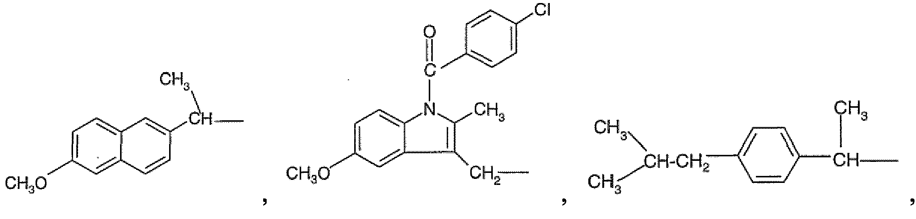 and

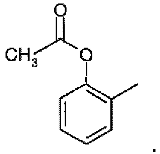

28. The use of any one of claims 1-24, wherein said NO-NSAID compound comprises a compound selected from the group consisting of: 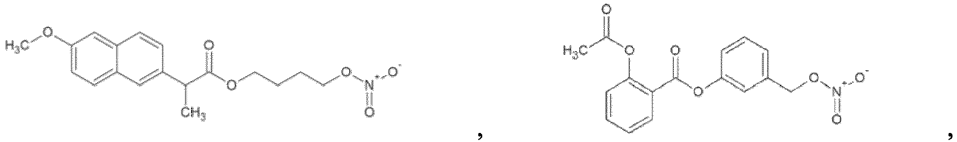 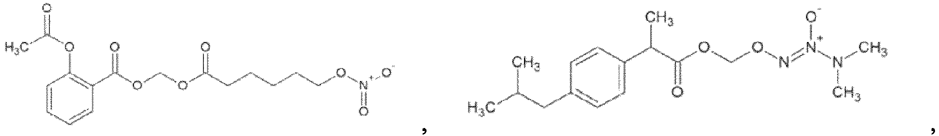 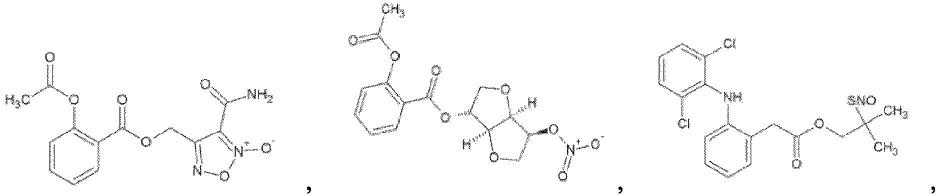 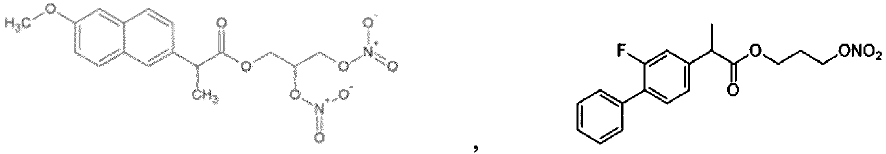 and

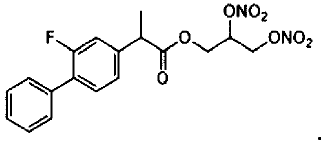

29. The use of any one of claims 1-28, wherein in the NO-NSAID compound, the molar ratio of the NO-releasing moiety to the NSAID moiety is about 2: 1 to about 1: 2.

30. The use of any one of claims 1-29, wherein said anti-tumor agent comprises a small molecule compound, a small molecule conjugate, a protein and/or a polynucleotide.

31. The use of any one of claims 1-30, wherein said anti-tumor agent comprises a chemotherapeutic agent, a targeted therapeutic agent and/or an immunotherapeutic agent.

32. The use of claim 31, wherein said anti-tumor agent is a targeted therapeutic agent.

33. The use of any one of claims 31-32, wherein the targeted therapeutic agent comprises a small molecule compound and/or an antibody or antigen-binding fragment thereof.

34. The use of claim 33, wherein said antibody comprises a monoclonal antibody, a multispecific antibody, a chimeric antibody, a humanized antibody, a fully human antibody and/or an antibody drug conjugate.

35. The use of claim 34, wherein said antigen-binding fragment comprises Fab, Fab', F(ab)₂, Fv fragment, F(ab')₂, scFv, di-scFv and/or dAb.

36. The use of any one of claims 31-35, wherein said targeted therapeutic agent targets molecules inside tumor cells, on the surface of tumor cells and/or in the tumor microenvironment.

37. The use of any one of claims 31-36, wherein said targeted therapeutic agent targets proteins and/or nucleic acid molecules.

38. The use of any one of claims 31-37, wherein said targeted therapeutic agent targets a tumor associated antigen.

39. The use of any one of claims 31-38, wherein said targeted therapeutic agent targets one or more targets selected from the group consisting of: VEGF, EGFR, EGFRl, EGFR2, EGFR3, EGFR4, HER2, HER3 , HER4 , VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c -Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI , HDAC, FAK, PYK2 , and mutants thereof.

40. The use of any one of claims 31-39, wherein said targeted therapeutic inhibits the activity of one or more targets selected from the group consisting of: VEGF, EGFR, EGFR1, EGFR2, EGFR3, EGFR4, HER2, HER3, HER4 , VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK , PI3K, NTRK, MSI, HDAC, FAK, PYK2, and mutants thereof.

41. The use of any one of claims 31-40, wherein said targeted therapeutic agent reduces the expression of one or more targets selected from the group consisting of: VEGF, EGFR, EGFR1, EGFR2, EGFR3, EGFR4, HER2, HER3, HER4 , VEGFR, VEGFR1, VEGFR2, VEGFR3, VEGFR4, PDGFR, PDGFRα, PDGFRβ, KIT, c-Kit, Ret, Raf, Raf-1, Abl, FGFR, FGFR1, MET, c-MET, Tie2, Src, c-Src, AXL, Ret, BCR-ABL, CSF-1R, FGFR, FGFR1, FGFR2, FGFR3, FGFR4, mTOR, TORC, BRaf, MEK, MEK1, MEK2, ALK, ABL, CDK, JAK, PI3K, NTRK, MSI, HDAC, FAK, PYK2, and mutants thereof.

42. The use of any one of claims 31-41, wherein said targeted therapeutic agent comprises a hormone, a signal transduction inhibitor, a gene expression modulator, an apoptosis inducer, an angiogenesis inhibitor and/or a toxin delivery molecule.

43. The use of any one of claims 31-42, wherein said targeted therapeutic agent is a tyrosinase inhibitor.

44. The use of any one of claims 31-42, wherein said targeted therapeutic agent is a VEGFR inhibitor and/or a VEGF inhibitor.

45. The use of claim 44, wherein said VEGFR inhibitor inhibits VEGFR1, VEGFR2 and/or VEGFR3.

46. The use of any one of claims 31-42, wherein said targeted therapeutic agent is an EGFR inhibitor.

47. The use of any one of claims 31-42, wherein said targeted therapeutic agent is a BRAF inhibitor.

48. The use of any one of claims 31-42, wherein said targeted therapeutic agent is a PDGFR inhibitor.

49. The use of any one of claims 31-42, wherein said targeted therapeutic agent is an FGFR inhibitor.

50. The use of any one of claims 31-42, wherein said targeted therapeutic agent is an mTOR inhibitor.

51. The use of any one of claims 31-42, wherein said targeted therapeutic agent is a HER2 inhibitor.

52. The use of any one of claims 31-42, wherein said targeted therapeutic agent is selected from one or more of the following group: afatinib, dacomitinib, osimertinib, EAI045 , gefitinib, almonertinib, pyrotinib, brigatinib, neratinib, olmutinib, bosutinib, icotinib, vandetanib, lapatinib , alflutinib, BPI-7711, mobocertinib, dovitinib, zorifertinib, varlitinib, orelabrutinib, acalabrutinib, brutinib, ibrutinib, dasatinib, pirtobrutinib, tolebrutinib, rilzabrutinib, fenebrutinib, evobrutinib, selumetinib, tivozanib, dovitinib, surufatinib, binimetinib, cobimetinib, trametinib, regorafenib, GSK-1120212, alpelisib, duvelisib, copanlisib, idelalisib, nortriptyline, inavolisib, dactolisib, apitolisib, parsaclisib, buparlisib, rigosertib, enzastaurin, paxalisib, leniolisib, ipatasertib, zotarolimus, sirolimus, everolimus, temsirolimus, sorafenib, apatinib, lenvatinib, sunitinib, cabozantinib, axitinib, nintedanib, brivanib, vatalanib, fruquintinib, dabrafenib, vemurafenib, encorafenib, pazopanib, crizotinib, panobinostat , erlotinib, rituximab, panitumumab, cetuximab, erfonrilimab, efactinib, cadonilimab, ramucirumab, bevacizumab, anlotinib, ponatinib, famitinib, erdatinib, AZD4547, infigratinib, BCD-217, amivantamab, MCLA-129, EMB-01, LY3164530, JNJ-61186372, anti-EGFR and cMet bispecific antibodies, GB263, their pharmaceutically acceptable salts and any combination thereof.

53. The use of any one of claims 31-52, wherein said targeted therapeutic agent is administered in combination with one or more other therapies.

54. The use of claim 31, wherein said anti-tumor agent is a chemotherapeutic agent.

55. The use of claim 54, wherein said chemotherapeutic agent comprises a pyrimidine nucleoside analog and/or a prodrug thereof.

56. The use of any one of claims 54-55, wherein said chemotherapeutic agent comprises one or more selected from the group consisting of: capecitabine, cytarabine, docetaxel, adriamycin, fluorouracil (5-FU), floxuridine, tegafur, idarubicin, paclitaxel, epirubicin, acelarin (NUC-1031), doxorubicin, leucovorin, cisplatin, paclitaxel, cyclophosphamide, vincristine, and 5-FU prodrug.

57. The use of claim 56, wherein said 5-FU prodrug comprises tegafur, 5'-deoxy-fluorouridine, floxuridine, 2'-deoxy-fluorouridine, prodrug derivative of floxuridine, prodrug derivative of 2'-deoxy-fluorouridine, trifluoro-methyl-2'-deoxyuridine, 6-azauridine and/or 3-deazauridine.

58. The use of any one of claims 56-57, wherein said chemotherapeutic agent is administered in combination with one or more other therapies.

59. The use of claim 58, wherein said one or more other therapies comprise one or more other anti-tumor therapies.

60. The use of any one of claims 1-59, wherein said disease or disorder is caused by administration of said anti-tumor agent.

61. The use of any one of claims 1-60, wherein said disease or disorder onset or progression after administration of said anti-tumor agent.

62. The use of any one of claims 1-61, wherein said subject did not suffer from the disease or disorder prior to administration of the anti-tumor agent.

63. The use of any one of claims 1-62, wherein said disease or disorder comprises an epithelial tissue disease or disorder.

64. The use of any one of claims 1-63, wherein said epithelial tissue disease or disorder comprises a disease or disorder associated with endothelial cell lesions and/or a disease or disorder associated with epithelial cell lesions.

65. The use of claim 64, wherein said endothelial cells comprise vascular endothelial cells.

66. The use of any one of claims 64-65, wherein said epithelial cells comprise skin epithelial cells, oral epithelial cells, nasal epithelial cells, gastric epithelial cells and/or intestinal epithelial cells.

67. The use of any one of claims 1-66, wherein said disease or disorder comprises a skin disease or disorder, a sensory disease or disorder and/or a gastrointestinal disease or disorder.

68. The use of claim 67, wherein the skin disease or disorder comprises alopecia, body odor, bullous dermatitis, dry skin, eczema, erythema multiforme, erythroderma, lipoatrophy, hair color changes, abnormal hair texture, hirsutism, hyperhidrosis, hyperkeratosis, hypertrichosis, hypohidrosis, lipohypertrophy, nail changes, nail discoloration, nail loss, nail ridges, skin pain, hand-foot syndrome, photosensitivity, pruritus, purpura, acneiform rash, maculopapular, scalp pain, skin atrophy, skin hyperpigmentation, skin hypopigmentation, skin induration, skin ulcers, Stevens-Johnson syndrome, subcutaneous emphysema, telangiectasia, toxic epidermal necrosis, rash and/or urticaria.

69. The use of any one of claims 67-68, wherein said skin disease or disorder is hand-foot syndrome.

70. The use of any one of claims 67-69, wherein the severity of said skin disease or disorder is NCI-CTCAE grade 1 or above, grade 2 or above, grade 3 or above, grade 4 or above, or grade 5.

71. The use of any one of claims 1-70, wherein said subject comprises a cancer patient.

72. The use of claim 71, wherein said disease or disorder affects a different site than the cancer.

73. The use of any one of claims 1-72, wherein said medicament does not substantially affect the therapeutic effect of the anti-tumor agent.

74. The use of any one of claims 1-73, wherein said medicament is prepared for topical administration.

75. The use of any one of claims 1-74, wherein said medicament is prepared for transdermal administration.

76. The use of any one of claims 1-75, wherein said medicament is prepared as a cream, lotion, gel, ointment, salve, spray, liposome formulation, liniment and/or aerosol.

77. The use of any one of claims 1-76, wherein the administration site of said medicament is different from the administration site of said anti-tumor agent.

78. The use of claim 77, wherein the administration site of said medicament is not the site of cancer occurrence or potential metastasis.

79. The use of any one of claims 1-78, wherein the administration method of said medicament is different from the administration method of said anti-tumor agent.

80. The use of any one of claims 1-79, wherein the concentration of said NO-NSAID compound in said medicament is from about 0.005% w/w to about 40% w/w.

81. The use of any one of claims 1-80, wherein the concentration of said NO-NSAID compound in said medicament is from about 0.5% w/w to about 10% w/w.

82. A method of preventing, ameliorating and/or treating a disease or disorder associated with administration of an anti-tumor agent in a subject, the method comprising administering to the subject in need thereof an effective amount of a NO-NSAID compound or a pharmaceutically acceptable salt thereof.

83. A NO-NSAID compound or pharmaceutically acceptable salt thereof, for use in preventing, ameliorating, and/or treating a disease or disorder associated with the administration of an anti-tumor agent in a subject.

84. A pharmaceutical combination comprising: 1) an anti-tumor agent; and 2) a NO-NSAID compound.

85. The pharmaceutical combination of claim 84, wherein said anti-tumor agent and said NO-NSAID compound are not mixed with each other.

86. The use of any one of claims 84-85, wherein said anti-tumor agent and said NO-NSAID compound are each independently present in a separate container.

87. The pharmaceutical combination of any one of claims 84-86, wherein said NO-NSAID compound is prepared for transdermal administration.

88. The pharmaceutical combination of any one of claims 84-87, wherein said NO-NSAID compound is prepared as an ointment.

89. The use of any one of claims 84-88, wherein the concentration of said NO-NSAID compound is from about 0.005% w/w to about 40% w/w.

90. The use of any one of claims 84-89, wherein the concentration of said NO-NSAID compound is from about 0.5 % w/w to about 10 % w/w.

91. The pharmaceutical combination of any one of claims 84-90, wherein said NO-NSAID compound in 2) is capable of preventing, ameliorating and/or treating a disease or disorder associated with said anti-tumor agent in 1).

92. The pharmaceutical combination of any one of claims 84-91, wherein said NO-NSAID compound in 2) does not substantially affect the therapeutic effect of said anti-tumor agent in 1).

93. The pharmaceutical combination of any one of claims 84-92, wherein said NO-NSAID compound of 2) is administered before, simultaneously with, or after said anti-tumor agent of 1).

94. A use of a NO-releasing agent and an NSAID in the preparation a medicament, said medicament is used for preventing, ameliorating and/or treating disease or disorder associated with the administration of an anti-tumor agent in a subject.

95. A method of preventing, ameliorating and/or treating a disease or disorder associated with administration of an anti-tumor agent in a subject, the method comprising administering to a subject in need thereof an effective amount of a NO-releasing agent and an NSAID.
A pharmaceutical combination comprising: 1) an anti-tumor agent; 2) a NO-releasing agent; and 3) an NSAID.
